(19) Europäisches Patentamt
European Patent Office
Office européen des brevets

(11) EP 3 669 652 A1

(12) **EUROPEAN PATENT APPLICATION**

(43) Date of publication:
**24.06.2020 Bulletin 2020/26**

(21) Application number: **18215136.5**

(22) Date of filing: **21.12.2018**

(51) Int Cl.:
***A01N 25/00*** *(2006.01)* ***A01N 43/90*** *(2006.01)*
***C07D 495/04*** *(2006.01)* ***A01P 3/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Bayer AG**
**51373 Leverkusen (DE)**

(72) Inventor: **The designation of the inventor has not yet been filed**

(74) Representative: **BIP Patents**
**c/o Bayer Intellectual Property GmbH**
**Alfred-Nobel-Straße 10**
**40789 Monheim am Rhein (DE)**

(54) **ACTIVE COMPOUND COMBINATION**

(57) The present invention relates to active compound combinations comprising at least one compound of formula (I)

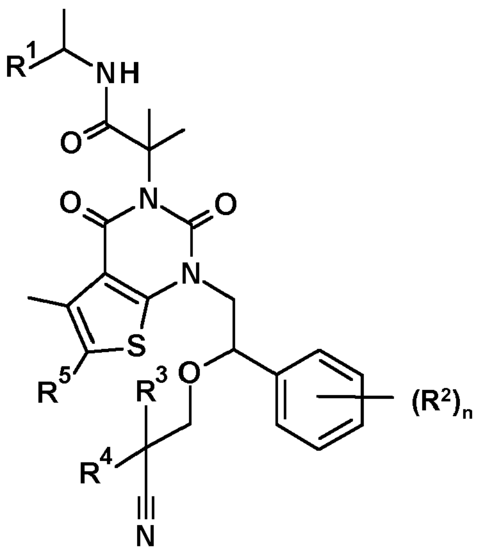

(I)

as defined in claim 1 as well as at least one further fungicide, to formulations comprising such compound combination, and to the use thereof as biologically active agents, especially for control of harmful microorganisms in crop protection and in the protection of materials and as plant growth regulators.

EP 3 669 652 A1

## Description

The present invention relates to an active compound combination, a formulation comprising such compound combination and to the use thereof as biologically active combination, especially for control of harmful microorganisms in crop protection and in the protection of materials.

It is already known that compounds or formula (I) can be used in crop protection as fungicides. Such compounds, processes to make them and their use as fungicides are for example disclosed in WO 2013/071169 A1, WO2017/091602, WO2017/091617 and WO2017/091627.

Since the environmental and economic requirements imposed on modern-day crop protection agents and compositions are continually increasing, with regard, for example, to the spectrum of action, toxicity, selectivity, application rate, formation of residues, and favorable preparation ability, and since, furthermore, there may be problems, for example, with resistances, a constant task is to develop new compositions, in particular fungicidal agents, which in some areas at least help to fulfil the abovementioned requirements. The present invention provides an active compound combination and a formulation comprising said combinations which in some asppects at least achieve the stated objective.

Accordingly, the present invention provides an active compound combination comprising

(A) at least one compound of formula (I)

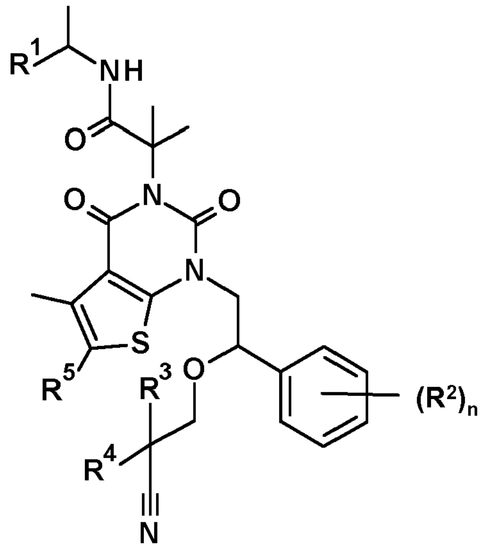

(I),

wherein

| | |
|---|---|
| $R^1$ | represents hydrogen or $-CH_3$; |
| $R^2$ | represents independently of one another $-OCH_3$ or F, |
| $R^3$, $R^4$ | represents independently of one another hydrogen or $-CH_3$; |
| $R^5$ | represents either |

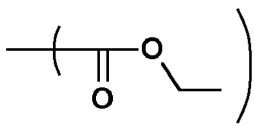

or

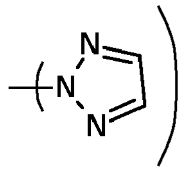

n is an integer and is 0, 1 or 2;

or a salt or N-oxide thereof,
and

(B) at least one further active compound selected from the following groups

(1) inhibitors of the ergosterol synthesis,
(2) inhibitors of the respiratory chain at complex I or II,
(3) inhibitors of the respiratory chain at complex III,
(4) inhibitors of the mitosis and cell division,
(5) compounds capable of having a multisite action,
(6) compounds capable of inducing a host defense,
(7) inhibitors of the amino acid and/or protein biosynthesis,
(8) inhibitors of the ATP production,
(9) inhibitors of the cell wall synthesis,
(10) inhibitors of the lipid and membrane synthesis,
(11) inhibitors of the melanine biosynthesis,
(12) inhibitors of the nucleic acid synthesis,
(13) inhibitors of the signal transduction,
(14) compounds capable of acting as uncoupler,
(15) other fungicides.

The active compound combination according to the invention comprises (A) at least one compound of formula (I) or a salt or N-oxide thereof. The salts or N-oxides of the compound of formula (I) also have fungicidal properties.
Preferably the compound combination of the invention comprises at least one compound of formula (1.01)

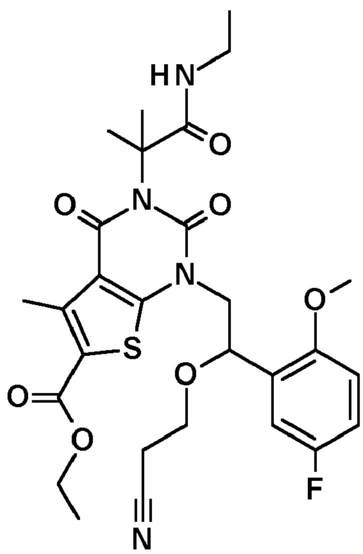

(I.01)

or a salt or N-oxide thereof.
Preferably the compound combination of the invention comprises at least one compound of formula (1.02)

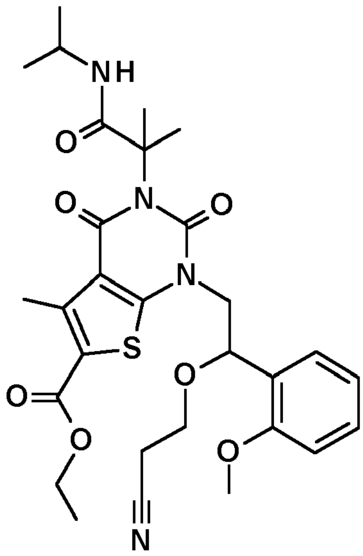

(I.02)

or a salt or N-oxide thereof.

Preferably the compound combination of the invention comprises at least one compound of formula (1.03)

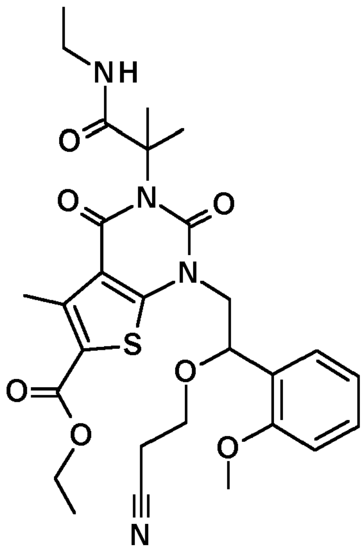

(I.03)

or a salt or N-oxide thereof.

Preferably the compound combination of the invention comprises at least one compound of formula (1.04)

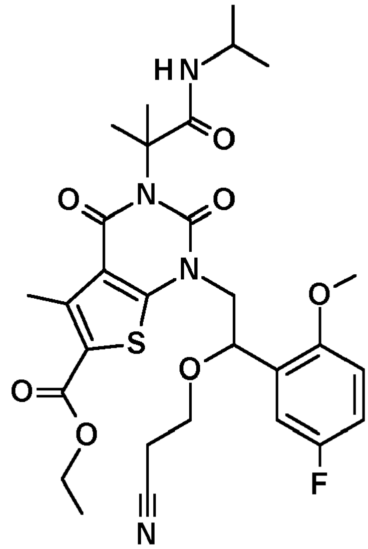

(I.04)

or a salt or N-oxide thereof.

Preferably the compound combination of the invention comprises at least one compound of formula (I-05)

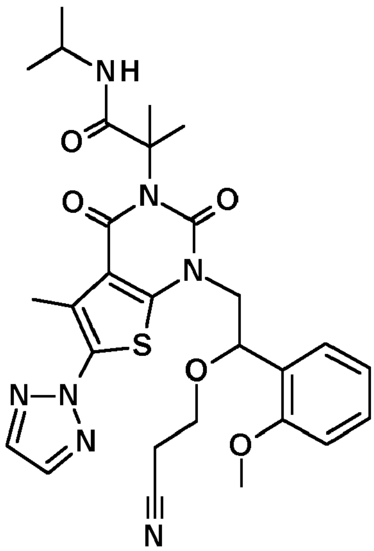

(I.05)

or a salt or N-oxide thereof.

Preferably the compound combination of the invention comprises at least one compound of formula (1.06)

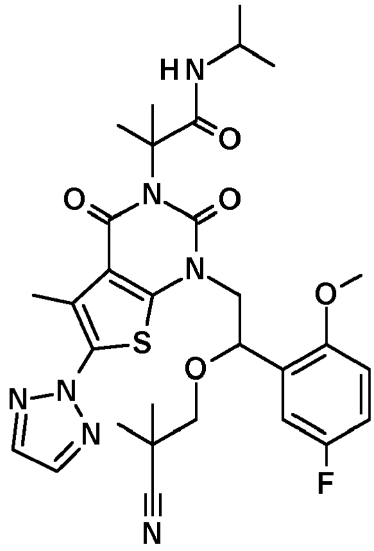

(I.06)

or a salt or N-oxide thereof.

Depending on the nature of the substituents, the compounds of formula (I) can be present as mixtures of different possible isomeric forms, in particular of stereoisomers, such as, for example, E and Z, threo and erythro, and also optical isomers, and, if appropriate, also of tautomers. If applicable, compounds of formula (I) comprise both the E and the Z isomers, and also the threo and erythro, and the optical isomers, any mixtures of these isomers, and the possible tautomeric forms.

Depending on the nature of the substituents, the compounds of formula (I) can exist in one or more optical or chiral isomer forms depending on the number of asymmetric centres in the compound. The invention thus relates equally to combinations comprising any of the optical isomers and their racemic or scalemic mixtures (the term "scalemic" denotes a mixture of enantiomers in different proportions) and mixtures of all the possible stereoisomers, in all proportions. The diastereoisomers and/or the optical isomers can be separated according to the methods which are known per se by the man ordinary skilled in the art.

Depending on the nature of the substituents, the compounds of formula (I) can also exist in one or more geometric isomer forms depending on the number of double bonds in the compound. The invention thus relates equally to all geometric isomers and to all possible mixtures, in all proportions. The geometric isomers can be separated according to general methods, which are known per se by the man ordinary skilled in the art.

*Further active compounds*

The active compound combination according to the invention comprise (B) at least one further active compound selected from the following groups

(1) inhibitors of the ergosterol synthesis,

(2) inhibitors of the respiratory chain at complex I or II,

(3) inhibitors of the respiratory chain at complex III,

(4) inhibitors of the mitosis and cell division,

(5) compounds capable of having a multisite action,

(6) compounds capable of inducing a host defense,

(7) inhibitors of the amino acid and/or protein biosynthesis,

(8) inhibitors of the ATP production,

(9) inhibitors of the cell wall synthesis,

(10) inhibitors of the lipid and membrane synthesis,

(11) inhibitors of the melanine biosynthesis,

(12) inhibitors of the nucleic acid synthesis,

(13) inhibitors of the signal transduction,

(14) compounds capable of acting as uncoupler,

(15) other fungicides.

Preferred compound combinations according to the invention comprise (B) at least one further active compound selected from:

inhibitors of the ergosterol synthesis selected from the group consisting of (1.001) cyproconazole, (1.002) difenoconazole, (1.003) epoxiconazole, (1.004) fenhexamid, (1.005) fenpropidin, (1.006) fenpropimorph, (1.007) fenpyrazamine, (1.008) fluquinconazole, (1.009) flutriafol, (1.010) imazalil, (1.011) imazalil sulfate, (1.012) ipconazole, (1.013) metconazole, (1.014) myclobutanil, (1.015) paclobutrazol, (1.016) prochloraz, (1.017) propiconazole, (1.018) prothioconazole, (1.019) pyrisoxazole, (1.020) spiroxamine, (1.021) tebuconazole, (1.022) tetraconazole, (1.023) triadimenol, (1.024) tridemorph, (1.025) triticonazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-l-ylmethyl)-cyclopentanol, (1.028) (2R)-2-(1-chlorocyclopropyl)-4-[(1R)-2,2-dichlorocyclopropyl]-1-(1H-1,2,4-triazol-1-yl)butan-2-ol, (1.029) (2R)-2-(1-chlorocyclopropyl)-4-[(1S)-2,2-dichlorocyclopropyl]-1-(1H-1,2,4-triazol-1-yl)butan-2-ol, (1.030) (2R)-2-[4-(4-chlorophenoxy)-2-(trifluoromethyl)phenyl]-1-(1H-1,2,4-triazol-1-yl)propan-2-ol, (1.031) (2S)-2-(1-chlorocyclopropyl)-4-[(1R)-2,2-dichlorocyclopropyl]-1-(1H-1,2,4-triazol-1-yl)butan-2-ol, (1.032) (2S)-2-(1-chlorocyclopropyl)-4-[(1S)-2,2-dichlorocyclopropyl]-1-(1H-1,2,4-triazol-1-yl)butan-2-ol, (1.033) (2S)-2-[4-(4-chlorophenoxy)-2-(trifluoromethyl)phenyl]-1-(1H-1,2,4-triazol-1-yl)propan-2-ol, (1.034) (R)- [3 -(4-chloro-2-fluorophenyl)-5-(2,4-difluorophenyl)-1,2-oxazol-4-yl](pyridin-3-yl)methanol, (1.035) (S)-[3-(4-chloro-2-fluorophenyl)-5-(2,4-difluorophenyl)-1,2-oxazol-4-yl](pyridin-3-yl)methanol, (1.036) [3-(4-chloro-2-fluorophenyl)-5-(2,4-difluorophenyl)-1,2-oxazol-4-yl](pyridin-3-yl)methanol, (1.037) 1 -({(2R,4S)-2-[2-chloro-4-(4-chlorophenoxy)phenyl]-4-methyl-1,3-dioxolan-2-yl}methyl)-1H-1,2,4-triazole, (1.038) 1-({(2S,4S)-2-[2-chloro-4-(4-chlorophenoxy)phenyl]-4-methyl-1,3-dioxolan-2-yl}methyl)-1H-1,2,4-triazole, (1.039) 1-{[3-(2-chlorophenyl)-2-(2,4-difluorophenyl)oxiran-2-yl]methyl}-1H-1,2,4-triazol-5-yl thiocyanate, (1.040) 1-{[rel(2R,3R)-3-(2-chlorophenyl)-2-(2,4-difluorophenyl)oxiran-2-yl]methyl}-1H-1,2,4-triazol-5-yl thiocyanate, (1.041) 1-{[rel(2R,3S)-3-(2-chlorophenyl)-2-(2,4-difluorophenyl)oxiran-2-yl]methyl}-1H-1,2,4-triazol-5-yl thiocyanate, (1.042) 2-[(2R,4R,5R)-1-(2,4-dichlorophenyl)-5-hydroxy-2,6,6-trimethylheptan-4-yl]-2,4-dihydro-3H-1,2,4-triazole-3-thione, (1.043) 2-[(2R,4R,5S)-1-(2,4-dichlorophenyl)-5-hydroxy-2,6,6-trimethylheptan-4-yl]-2,4-dihydro-3H-1,2,4-triazole-3-thione, (1.044) 2-[(2R,4S,5R)-1-(2,4-dichlorophenyl)-5-hydroxy-2,6,6-trimethylheptan-4-yl]-2,4-dihydro-3H-1,2,4-triazole-3-thione, (1.045) 2-[(2R,4S,5S)-1-(2,4-dichlorophenyl)-5-hydroxy-2,6,6-trimethylheptan-4-yl]-2,4-dihydro-3H-1,2,4-triazole-3-thione, (1.046) 2-[(2S,4R,5R)-1-(2,4-dichlorophenyl)-5-hydroxy-2,6,6-trimethylheptan-4-yl]-2,4-dihydro-3H-1,2,4-triazole-3-thione, (1.047) 2-[(2S,4R,5S)-1-(2,4-dichlorophenyl)-5-hydroxy-2,6,6-trimethylheptan-4-yl]-2,4-dihydro-3H-1,2,4-triazole-3-thione, (1.048) 2-[(2S,4S,5R)-1-(2,4-dichlorophenyl)-5-hydroxy-2,6,6-trimethylheptan-4-yl]-2,4-dihydro-3H-1,2,4-triazole-3-thione, (1.049) 2-[(2S,4S,5S)-1-(2,4-dichlorophenyl)-5-hydroxy-2,6,6-trimethylheptan-4-yl]-2,4-dihydro-3H-1,2,4-triazole-3-thione, (1.050) 2-[1-(2,4-dichlorophenyl)-5-hydroxy-2,6,6-trimethylheptan-4-yl]-2,4-dihydro-3H-1,2,4-triazole-3-thione, (1.051) 2-[2-chloro-4-(2,4-dichlorophenoxy)phenyl]-1-(1H-1,2,4-triazol-1-yl)propan-2-ol, (1.052) 2-[2-chloro-4-(4-chlorophenoxy)phenyl]-1-(1H-1,2,4-triazol-1-yl)butan-2-ol, (1.053) 2-[4-(4-chlorophenoxy)-2-(trifluoromethyl)phenyl]-1-(1H-1,2,4-triazol-1-yl)butan-2-ol, (1.054) 2-[4-(4-chlorophenoxy)-2-(trifluoromethyl)phenyl]-1-(1H-1,2,4-triazol-1-yl)pentan-2-ol, (1.055) 2-[4-(4-chlorophenoxy)-2-(trifluoromethyl)phenyl]-1-(1H-1,2,4-triazol-1-yl)propan-2-ol, (1.056) 2-{[3-(2-chlorophenyl)-2-(2,4-difluorophenyl)oxiran-2-yl]methyl}-2,4-dihydro-3H-1,2,4-triazole-3-thione, (1.057) 2-{[rel(2R,3R)-3-(2-chlorophenyl)-2-(2,4-difluorophenyl)oxiran-2-yl]methyl}-2,4-dihydro-3H-1,2,4-triazole-3-thione, (1.058) 2-{[rel(2R,3S)-3-(2-chlorophenyl)-2-(2,4-difluorophenyl)oxiran-2-yl]methyl}-2,4-dihydro-3H-1,2,4-triazole-3-thione,(1.059)5-(4-chlorobenzyl)-2-(chloromethyl)-2-methyl-1-(1H-1,2,4-triazol-1-ylmethyl)cyclopentanol, (1.060) 5-(allylsulfanyl)-1-{[3-(2-chlorophenyl)-2-(2,4-difluorophenyl)oxiran-2-yl]methyl}-1H-1,2,4-triazole, (1.061) 5-(allylsulfanyl)-1-{[rel(2R,3R)-3-(2-chlorophenyl)-2-(2,4-difluorophenyl)oxiran-2-yl]methyl}-1H-1,2,4-triazole, (1.062) 5-(allylsulfanyl)-1-{[rel(2R,3S)-3-(2-chlorophenyl)-2-(2,4-difluorophenyl)oxiran-2-yl]methyl}-1H-1,2,4-triazole,(1.063) N'-(2,5-dimethyl-4-{[3-(1,1,2,2-tetrafluoroethoxy)phenyl]sulfanyl}phenyl)-N-ethyl-N-methylimidoformamide, (1.064) N'-(2,5-dimethyl-4-{[3-(2,2,2-trifluoroethoxy)phenyl]sulfanyl}phenyl)-N-ethyl-N-methylimidoformamide, (1.065) N'-(2,5-dimethyl-4- {[3-(2,2,3,3-tetrafluoropropoxy)phenyl]sulfanyl}phenyl)-N-ethyl-N-methylimidoformamide, (1.066) N'-(2,5-dimethyl-4- {[3-(pentafluoroethoxy)phenyl]sulfanyl}phenyl)-N-ethyl-N-methylimidoformamide, (1.067) N'-(2,5-dimethyl-4-{3-[(1,1,2,2-tetrafluoroethyl)sulfanyl]phenoxy}phenyl)-N-ethyl-N-methylimidoformamide, (1.068) N'-(2,5-dimethyl-4-{3-[(2,2,2-trifluoroethyl)sulfanyl]phenoxy}phenyl)-N-ethyl-N-methylimidoformamide, (1.069) N'-(2,5-dimethyl-4- {3-[(2,2,3,3-tetrafluoropropyl)sulfanyl]phenoxy}phenyl)-N-ethyl-N-methylimidoformamide, (1.070) N'-(2,5-dimethyl-4-{3-[(pentafluoroethyl)sulfanyl]phenoxy}phenyl)-N-ethyl-N-methylimidoformamide, (1.071) N'-(2,5-dimethyl-4-phenoxyphenyl)-N-ethyl-N-methylimidoformamide, (1.072) N'-(4-{[3-(difluoromethoxy)phenyl]sulfanyl}-2,5-dimethylphenyl)-N-ethyl-N-methylimidoformamide, (1.073) N'-(4-{3-[(difluoromethyl)sulfanyl]phenoxy}-2,5-dimethylphenyl)-N-ethyl-N-methylimidoformamide, (1.074) N'- [5-bromo-6-(2,3-dihydro-1H-inden-2-yloxy)-2-methylpyridin-3 -yl] -N-ethyl-N-methylimidoformamide, (1.075) N'-{4-[(4,5-dichloro-1,3-thiazol-2-yl)oxy]-2,5-dimethylphenyl}-N-ethyl-N-methylimidoformamide, (1.076) N'-{5-bromo-6-[(1R)-1-(3,5-difluorophenyl)ethoxy]-2-methylpyridin-3-yl}-N-ethyl-N-methylimidoformamide, (1.077) N'-{5-bromo-6-[(1S)-1-(3,5-difluorophenyl)ethoxy]-2-methylpyridin-3-yl}-N-ethyl-N-methylimidoformamide, (1.078) N'- {5-bromo-6-[(cis-4-isopropylcyclohexyl)oxy]-2-methylpyridin-3-yl} -N-ethyl-N-methylimidoformamide, (1.079) N'- {5-bromo-6-[(trans-4-isopropylcyclohexyl)oxy]-2-methylpyridin-3-yl}-N-ethyl-N-methylimidoformamide, (1.080) N'-{5-bromo-6-[1-(3,5-difluorophenyl)ethoxy]-2-methylpyridin-3-yl}-N-ethyl-N-methylimidoformamide, (1.081) Mefentrifluconazole, (1.082) Ipfentrifluconazole, and (1.083) 2-[6-(4-bromophenoxy)-2-(trifluoromethyl)-3 -pyridyl] -1 -(1,2,4-triazol-1 -yl)propan-2-ol,

inhibitors of the respiratory chain at complex I or II selected from the group consisting of (2.001) benzovindiflupyr, (2.002) bixafen, (2.003) boscalid, (2.004) carboxin, (2.005) fluopyram, (2.006) flutolanil, (2.007) fluxapyroxad, (2.008) furametpyr, (2.009) Isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.020) Pyraziflumid, (2.021) sedaxane, (2.022) 1,3-dimethyl-N-(1,1,3-trimethyl-2,3-dihydro-1H-inden-4-yl)-1H-pyrazole-4-carboxamide, (2.023) 1,3-dimethyl-N-[(3R)-1,1,3-trimethyl-2,3-dihydro-1H-inden-4-yl]-1H-pyrazole-4-carboxamide, (2.024) 1,3 -dimethyl-N- [(3 S)-1,1,3 -trimethyl-2,3 -dihydro-1H-inden-4-yl] -1H-pyrazole-4-carboxamide, (2.025) 1-methyl-3-(trifluoromethyl)-N-[2'-(trifluoromethyl)biphenyl-2-yl]-1H-pyrazole-4-carboxamide, (2.026) 2-fluoro-6-(trifluoromethyl)-N-(1,1,3-trimethyl-2,3-dihydro-1H-inden-4-yl)benzamide, (2.027) 3-(difluoromethyl)-1-methyl-N-(1,1,3-trimethyl-2,3-dihydro-1H-inden-4-yl)-1H-pyrazole-4-carboxamide, (2.028) 3-(difluoromethyl)-1-methyl-N-[(3R)-1,1,3-trimethyl-2,3-dihydro-1H-inden-4-yl]-1H-pyrazole-4-carboxamide, (2.029) 3-(difluoromethyl)-1-methyl-N-[(3 S)-1,1,3-trimethyl-2,3-dihydro-1H-inden-4-yl]-1H-pyrazole-4-carboxamide, (2.030) 3-(difluoromethyl)-N-(7-fluoro-1,1,3-trimethyl-2,3-dihydro-1H-inden-4-yl)-1-methyl-1H-pyrazole-4-carboxamide, (2.031) 3 -(difluoromethyl)-N-[(3R)-7-fluoro-1,1,3-trimethyl-2,3-dihydro-1H-inden-4-yl]-1-methyl-1H-pyrazole-4-carboxamide, (2.032) 3 -(difluoromethyl)-N- [(3 S)-7-fluoro-1,1,3 -trimethyl-2,3 -dihydro-1H-inden-4-yl] -1 -methyl-1H-pyrazole-4-carboxamide, (2.033) 5,8-difluoro-N-[2-(2-fluoro-4-{[4-(trifluoromethyl)pyridin-2-yl]oxy}phenyl)ethyl]quinazolin-4-amine, (2.034) N-(2-cyclopentyl-5-fluorobenzyl)-N-cyclopropyl-3-(difluoromethyl)-5-fluoro-1-methyl-1H-pyrazole-4-carboxamide, (2.035) N-(2-tert-butyl-5-methylbenzyl)-N-cyclopropyl-3-(difluoromethyl)-5-fluoro-1-methyl-1H-pyrazole-4-carboxamide, (2.036) N-(2-tert-butylbenzyl)-N-cyclopropyl-3-(difluoromethyl)-5-fluoro-1-methyl-1H-pyrazole-4-carboxamide, (2.037) N-(5-chloro-2-ethylbenzyl)-N-cyclopropyl-3-(difluoromethyl)-5-fluoro-1-methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide, (2.039) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide, (2.040) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide, (2.041) N-[1-(2,4-dichlorophenyl)-1-methoxypropan-2-yl]-3-(difluoromethyl)-1-methyl-1H-pyrazole-4-carboxamide, (2.042) N-[2-chloro-6-(trifluoromethyl)benzyl]-N-cyclopropyl-3-(difluoromethyl)-5-fluoro-1-methyl-1H-pyrazole-4-carboxamide, (2.043) N-[3-chloro-2-fluoro-6-(trifluoromethyl)benzyl]-N-cyclopropyl-3-(difluoromethyl)-5-fluoro-1-methyl-1H-pyrazole-4-carboxamide, (2.044) N-[5-chloro-2-(trifluoromethyl)benzyl]-N-cyclopropyl-3-(difluoromethyl)-5-fluoro-1-methyl-1H-pyrazole-4-carboxamide, (2.045) N-cyclopropyl-3-(difluoromethyl)-5-fluoro-1-methyl-N-[5-methyl-2-(trifluoromethyl)benzyl]-1H-pyrazole-4-carboxamide, (2.046) N-cyclopropyl-3-(difluoromethyl)-5-fluoro-N-(2-fluoro-6-isopropylbenzyl)-1-methyl-1H-pyrazole-4-carboxamide, (2.047) N-cyclopropyl-3-(difluoromethyl)-5-fluoro-N-(2-isopropyl-5-methylbenzyl)-1-methyl-1H-pyrazole-4-carboxamide, (2.048) N-cyclopropyl-3-(difluoromethyl)-5-fluoro-N-(2-isopropylbenzyl)-1-methyl-1H-pyrazole-4-carbothioamide, (2.049) N-cyclopropyl-3-(difluoromethyl)-5-fluoro-N-(2-isopropylbenzyl)-1-methyl-1H-pyrazole-4-carboxamide, (2.050) N-cyclopropyl-3-(difluoromethyl)-5-fluoro-N-(5-fluoro-2-isopropylbenzyl)-1-methyl-1H-pyrazole-4-carboxamide, (2.051) N-cyclopropyl-3-(difluoromethyl)-N-(2-ethyl-4,5-dimethylbenzyl)-5-fluoro-1-methyl-1H-pyrazole-4-carboxamide, (2.052) N-cyclopropyl-3-(difluoromethyl)-N-(2-ethyl-5-fluorobenzyl)-5-fluoro-1-methyl-1H-pyrazole-4-carboxamide, (2.053) N-cyclopropyl-3-(difluoromethyl)-N-(2-ethyl-5-methylbenzyl)-5-fluoro-1-methyl-1H-pyrazole-4-carboxamide, (2.054) N-cyclopropyl-N-(2-cyclopropyl-5-fluorobenzyl)-3-(difluoromethyl)-5-fluoro-1-methyl-1H-pyrazole-4-carboxamide, (2.055) N-cyclopropyl-N-(2-cyclopropyl-5-methylbenzyl)-3-(difluoromethyl)-5-fluoro-1-methyl-1H-pyrazole-4-carboxamide, and (2.056) N-cyclopropyl-N-(2-cyclopropylbenzyl)-3-(difluoromethyl)-5-fluoro-1-methyl-1H-pyrazole-4-carboxamide,

inhibitors of the respiratory chain at complex III selected from the group consisting of (3.001) ametoctradin, (3.002) amisulbrom, (3.003) azoxystrobin, (3.004) coumethoxystrobin, (3.005) coumoxystrobin, (3.006) cyazofamid, (3.007) dimoxystrobin, (3.008) enoxastrobin, (3.009) famoxadone, (3.010) fenamidone, (3.011) flufenoxystrobin, (3.012) fluoxastrobin, (3.013) kresoxim-methyl, (3.014) metominostrobin, (3.015) orysastrobin, (3.016) picoxystrobin, (3.017) pyraclostrobin, (3.018) pyrametostrobin, (3.019) pyraoxystrobin, (3.020) trifloxystrobin, (3.021) (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, (3.022) (2E,3Z)-5-{[1-(4-chlorophenyl)-1H-pyrazol-3-yl]oxy}-2-(methoxyimino)-N,3-dimethylpent-3-enamide, (3.023) (2R)-2-{2-[(2,5-dimethylphenoxy)methyl]phenyl}-2-methoxy-N-methylacetamide, (3.024) (2S)-2-{2-[(2,5-dimethylphenoxy)methyl]phenyl}-2-methoxy-N-methylacetamide, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) 2-{2-[(2,5-dimethylphenoxy)methyl]phenyl}-2-methoxy-N-methylacetamide, (3.027) N-(3-ethyl-3,5,5-trimethylcyclohexyl)-3 -formamido-2-hydroxybenzamide, (3.028) (2E,3Z)-5-{[1-(4-chloro-2-fluorophenyl)-1H-pyrazol-3-yl]oxy}-2-(methoxyimino)-N,3 -dimethylpent-3 -enamide, and (3.029) methyl {5-[3-(2,4-dimethylphenyl)-1H-pyrazol-1-yl]-2-methylbenzyl}carbamate, (3.030) (1*S*)-2,2-bis(4-fluorophenyl)-1-methylethyl *N*-{[3-(acetyloxy)-4-methoxy-2-pyridyl]carbonyl}-L-alaninate,

inhibitors of the mitosis and cell division selected from the group consisting of (4.001) carbendazim, (4.002) diethofencarb, (4.003) ethaboxam, (4.004) fluopicolide, (4.005) pencycuron, (4.006) thiabendazole, (4.007) thiophanate-methyl, (4.008) zoxamide, (4.009) 3-chloro-4-(2,6-difluorophenyl)-6-methyl-5-phenylpyridazine, (4.010) 3-chloro-5-(4-chlorophenyl)-4-(2,6-difluorophenyl)-6-methylpyridazine, (4.011) 3-chloro-5-(6-chloropyridin-3-yl)-6-methyl-4-(2,4,6-trifluorophenyl)pyridazine, (4.012) 4-(2-bromo-4-fluorophenyl)-N-(2,6-difluorophenyl)-1,3-dimethyl-1H-pyrazol-5-amine, (4.013) 4-(2-bromo-4-fluorophenyl)-N-(2-bromo-6-fluorophenyl)-1,3-dimethyl-1H-pyrazol-5-amine, (4.014) 4-(2-bromo-4-fluorophenyl)-N-(2-bromophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.016) 4-(2-bromo-4-fluorophenyl)-N-(2-chlorophenyl)-1,3-dimethyl-1H-pyrazol-5-amine, (4.017) 4-(2-bromo-4-fluorophenyl)-N-(2-fluorophenyl)-1,3-dimethyl-1H-pyrazol-5-amine, (4.018) 4-(2-chloro-4-fluorophenyl)-N-(2,6-difluorophenyl)-1,3-dimethyl-1H-pyrazol-5-amine, (4.019) 4-(2-chloro-4-fluorophenyl)-N-(2-chloro-6-fluorophenyl)-1,3-dimethyl-1H-pyrazol-5-amine, (4.020) 4-(2-chloro-4-fluorophenyl)-N-(2-chlorophenyl)-1,3-dimethyl-1H-pyrazol-5-amine, (4.021) 4-(2-chloro-4-fluorophenyl)-N-(2-fluorophenyl)-1,3-dimethyl-1H-pyrazol-5-amine, (4.022) 4-(4-chlorophenyl)-5-(2,6-difluorophenyl)-3,6-dimethylpyridazine, (4.023) N-(2-bromo-6-fluorophenyl)-4-(2-chloro-4-fluorophenyl)-1,3-dimethyl-1H-pyrazol-5-amine, (4.024) N-(2-bromophenyl)-4-(2-chloro-4-fluorophenyl)-1,3-dimethyl-1H-pyrazol-5-amine, and (4.025) N-(4-chloro-2,6-difluorophenyl)-4-(2-chloro-4-fluorophenyl)-1,3-dimethyl-1H-pyrazol-5-amine,

compounds capable of having a multisite action selected from the group consisting of (5.001) bordeaux mixture, (5.002) captafol, (5.003) captan, (5.004) chlorothalonil, (5.005) copper hydroxide, (5.006) copper naphthenate, (5.007) copper oxide, (5.008) copper oxychloride, (5.009) copper(2+) sulfate, (5.010) dithianon, (5.011) dodine, (5.012) folpet, (5.013) mancozeb, (5.014) maneb, (5.015) metiram, (5.016) metiram zinc, (5.017) oxine-copper, (5.018) propineb, (5.019) sulfur and sulfur preparations including calcium polysulfide, (5.020) thiram, (5.021) zineb, (5.022) ziram, and (5.023) 6-ethyl-5,7-dioxo-6,7-dihydro-5H-pyrrolo[3',4':5,6][1,4]dithiino[2,3-c][1,2]thiazole-3-carbonitrile,

compounds capable of inducing a host defense selected from the group consisting of (6.001) acibenzolar-S-methyl, (6.002) isotianil, (6.003) probenazole, and (6.004) tiadinil,

inhibitors of the amino acid and/or protein biosynthesis selected from the group consisting of (7.001) cyprodinil, (7.002) kasugamycin, (7.003) kasugamycin hydrochloride hydrate, (7.004) oxytetracycline, (7.005) pyrimethanil, and (7.006) 3-(5-fluoro-3,3,4,4-tetramethyl-3,4-dihydroisoquinolin-1-yl)quinolone,

inhibitors of the ATP production selected from the group consisting of (8.001) silthiofam,

inhibitors of the cell wall synthesis selected from the group consisting of (9.001) benthiavalicarb, (9.002) dimethomorph, (9.003) flumorph, (9.004) iprovalicarb, (9.005) mandipropamid, (9.006) pyrimorph, (9.007) valifenalate, (9.008) (2E)-3-(4-tert-butylphenyl)-3-(2-chloropyridin-4-yl)-1-(morpholin-4-yl)prop-2-en-1-one, and (9.009) (2Z)-3-(4-tert-butylphenyl)-3-(2-chloropyridin-4-yl)-1-(morpholin-4-yl)prop-2-en-1-one,

inhibitors of the lipid and membrane synthesis selected from the group consisting of (10.001) propamocarb, (10.002) propamocarb hydrochloride, and (10.003) tolclofos-methyl,

inhibitors of the melanine biosynthesis selected from the group consisting of (11.001) tricyclazole, and (11.002) 2,2,2-trifluoroethyl {3-methyl-1-[(4-methylbenzoyl)amino]butan-2-yl}carbamate,

inhibitors of the nucleic acid synthesis selected from the group consisting of (12.001) benalaxyl, (12.002) benalaxyl-M (kiralaxyl), (12.003) metalaxyl, and (12.004) metalaxyl-M (mefenoxam),

inhibitors of the signal transduction selected from the group consisting of (13.001) fludioxonil, (13.002) iprodione, (13.003) procymidone, (13.004) proquinazid, (13.005) quinoxyfen, and (13.006) vinclozolin,

compounds capable of acting as uncoupler selected from the group consisting of (14.001) fluazinam, and (14.002) meptyldinocap,

other fungicides selected from the group consisting of (15.001) abscisic acid, (15.002) benthiazole, (15.003) bethoxazin, (15.004) capsimycin, (15.005) carvone, (15.006) chinomethionat, (15.007) cufraneb, (15.008) cyflufenamid, (15.009) cymoxanil, (15.010) cyprosulfamide, (15.011) flutianil, (15.012) fosetyl-aluminium, (15.013) fosetyl-calcium, (15.014) fosetyl-sodium, (15.015) methyl isothiocyanate, (15.016) metrafenone, (15.017) mildiomycin, (15.018) natamycin, (15.019) nickel dimethyldithiocarbamate, (15.020) nitrothal-isopropyl, (15.021) oxamocarb, (15.022) oxathiapiprolin, (15.023) oxyfenthiin, (15.024) pentachlorophenol and salts, (15.025) phosphorous acid and its salts, (15.026) propamocarb-fosetylate, (15.027) pyriofenone (chlazafenone), (15.028) tebufloquin, (15.029) tecloftalam, (15.030) tolnifanide, (15.031) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.032) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.033) 2-(6-benzylpyridin-2-yl)quinazoline, (15.034) 2,6-dimethyl-1H,5H-[1,4]dithiino[2,3-c:5,6-c']dipyrrole-1,3,5,7(2H,6H)-tetrone, (15.035) 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl} -1,3-thiazol-2-yl)piperidin-1-yl]ethanone, (15.036) 2-[3,5-bis(difluoromethyl)-1H-pyrazol-1 -yl]-1 -[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone, (15.037) 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl] ethanone, (15.038) 2-[6-(3-fluoro-4-methoxyphenyl)-5-methylpyridin-2-yl]quinazoline, (15.039) 2-{(5R)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl} -3-chlorophenyl methanesulfonate, (15.040) 2-{(5S)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.041) 2-{2-[(7,8-difluoro-2-methylquinolin-3-yl)oxy]-6-fluorophenyl}propan-2-ol, (15.042) 2-{2-fluoro-6-[(8-fluoro-2-methylquinolin-3-yl)oxy]phenyl}propan-2-ol, (15.043) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl} -3 -chlorophenyl methanesulfonate, (15.044) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}phenyl methanesulfonate, (15.045) 2-phenylphenol and salts, (15.046) 3-(4,4,5-trifluoro-3,3-dimethyl-3,4-dihydroisoquinolin-1-yl)quinoline, (15.047) 3-(4,4-difluoro-3,3-dimethyl-3,4-dihydroisoquinolin-1-yl)quinoline, (15.048) 4-amino-5-fluoropyrimidin-2-ol (tautomeric form: 4-amino-5-fluoropyrimidin-2(1H)-one), (15.049) 4-oxo-4-[(2-phenylethyl)amino]butanoic acid, (15.050) 5-amino-1,3,4-thiadiazole-2-thiol, (15.051) 5-chloro-N'-phenyl-N'-(prop-2-yn-1-yl)thiophene-2-sulfonohydrazide, (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.054) 9-fluoro-2,2-dimethyl-5-(quinolin-3-yl)-2,3-dihydro-1,4-benzoxazepine, (15.055) but-3-yn-1-yl {6-[({[(Z)-(1-methyl-1H-tetrazol-5-yl)(phenyl)methylene]amino}oxy)methyl]pyridin-2-yl}carbamate, (15.056) ethyl (2Z)-3-amino-2-cyano-3-phenylacrylate, (15.057) phenazine-l-carboxylic acid, (15.058) propyl 3,4,5-trihydroxybenzoate, (15.059) quinolin-8-ol, (15.060) quinolin-8-ol sulfate (2:1), (15.061) tert-butyl {6-[({[(1-methyl-1H-tetrazol-5-yl)(phenyl)methylene]amino}oxy)methyl]pyridin-2-yl}carbamate, (15.062) 5-fluoro-4-imino-3-methyl-1-[(4-methylphenyl)sulfonyl]-3,4-dihydropyrimidin-2(1H)-one, (15.063) Metyltetraprole, (15.064) Aminopyrifen, (15. 065) Pyrapropoyne, (15.066) (N'-[2-chloro-4-(2-fluorophenoxy)-5-methylphenyl]-N-ethyl-N-methylimidoformamide), (15.067) (N'-(2-chloro-5-methyl-4-phenoxyphenyl)-N-ethyl-N-methylimidoformamide), (15.068) (2-{2-[(7,8-difluoro-2-methylquinolin-3-yl)oxy]-6-fluorophenyl}propan-2-ol), (15.069) (5-bromo-1-(5,6-dimethylpyridin-3-yl)-3,3-dimethyl-3,4-dihydroisoquinoline), (15.070) (3-(4,4-difluoro-5,5-dimethyl-4,5-dihydrothieno[2,3-c]pyridin-7-yl)quinoline), (15.071) (1-(4,5-dimethyl-1H-benzimidazol-1-yl)-4,4-difluoro-3,3-dimethyl-3,4-dihydroisoquinoline), (15.072) 8-fluoro-3-(5-fluoro-3,3-dimethyl-3,4-dihydroisoquinolin-1-yl)quinolone, (15.073) 8-fluoro-3-(5-fluoro-3,3,4,4-tetramethyl-3,4-dihydroisoquinolin-1-yl)quinolone, (15.074) 3-(4,4-difluoro-3,3-dimethyl-3,4-dihydroisoquinolin-1-yl)-8-fluoroquinoline, (15.075) (N-methyl-N-phenyl-4-[5-(trifluoromethyl)-1,2,4-oxadiazol-3-yl]benzamide), (15.076) (methyl {4-[5-(trifluoromethyl)-1,2,4-oxadiazol-3-yl]phenyl}carbamate), (15.077) (N-{4-[5-(trifluoromethyl)-1,2,4-oxadiazol-3-yl]benzyl}cyclopropanecarboxamide), (15.078) N-methyl-4-(5-(trifluoromethyl)-1,2,4-oxadiazol-3-yl]benzamide, (15.079) N-[(E)-methoxyiminomethyl]-4-[5-(trifluoromethyl)-1,2,4-oxadiazol-3-yl]benzamide, (15.080) N- [(Z)-methoxyiminomethyl] -4- [5-(trifluoromethyl)-1,2,4-oxadiazol-3-yl]benzamide, (15. 081) N-[4-[5-(trifluoromethyl)-l,2,4-oxadiazol-3-yl]phenyl]cyclopropanecarboxamide, (15.082) N-(2-fluorophenyl)-4-[5-(trifluoromethyl)-1,2,4-oxadiazol-3-yl]benzamide, (15.083) 2,2-difluoro-N-methyl-2-[4-[5-(trifluoromethyl)-1,2,4-oxadiazol-3-yl]phenyl]acetamide, (15.084) N-allyl-N-[[4-[5-(trifluoromethyl)-1,2,4-oxadiazol-3-yl)phenyl]methyl]acetamide, (15.085) N-[(E)-N-methoxy-C-methyl-carbonimidoyl]-4-(5-(trifluoromethyl)-1,2,4-oxadiazol-3-yl]benzamide, (15.086) N-[(Z)-N-methoxy-C-methyl-carbonimidoyl]-4-[5-(trifluoromethyl)-1,2,4-oxadiazol-3-yl]benzamide, (15.087) N-allyl-N-[[4-[5-(trifluoromethyl)-1,2,4-oxadiazol-3-yl]phenyl]methyl]propanamide, (15.088) 4,4-dimethyl-1-[[4-[5-(trifluoromethyl)-1,2,4-oxadiazol-3-yl]phenyl]methyl]pyrro lidin-2-one, (15.089) N-methyl-4-[5-(trifluoromethyl)-1,2,4-oxadiazol-3-yl]benzenecarbothioamide, (15.090) 5-methyl-1-[[4-[5-(trifluoromethyl)-1,2,4-oxadiazol-3-yl]phenyl]methyl]pyrrolidin-2-one, (15.091) N-((2,3-difluoro-4-[5-(trifluoromethyl)-1,2,4-oxadiazol-3-yl]phenyl]methyl]-3,3,3-trifluoro-propanamide, (15.092) 1-methoxy-1-methyl-3-[[4-[5-(trifluorometyhl}-1,2,4-oxadiazol-3-yl]phenyl]methyl]urea, (15.093) 1,1-diethyl-3-[[4-[5-(trifluoromethyl}-1,2,4-oxadiazol-3-yl]phenyl]methyl]urea, (15.094) N-[[4-[5- (trifluoromethyl)-1,2,4-oxadiazol-3-yl]phenyl)methyl)propanamide, (15.095) N-methoxy-N-[[4-[5-(trifluoromethyl)-1,2,4-oxadiazol-3-yl]phenyl]methyl]cyclopropanecarboxamide, (15.096) 1-methoxy-3-methyl-1-[[4-[5-(trifluoromethyl)-1,2,4-oxadiazol-3-yl]phenyl]methyl]urea, (15.097) N-methoxy-N-[[4-[5-(trifluoromethyl)-1,2,4-oxadiazol-3-yl]phenyl]methly)cyclopropanecarboxamide; (15.098) N,2-dimethoxy-N-[[4-[5-(trifluoromethyl}-1,2,4-oxadiazol-3-yl]phenyl]methyl]propanamide, (15.099) N-ethyl-2-methyl-N-[[4-[5-(trifluoromethyl)-1,2,4-oxadiazol-3-yl)phenyl]melhyl]propanamide, (15.100) 1-methoxy-3-methyl-1-[[4-[5-(trifluoromethyl)-1,2,4-oxadiazol-3-yl]phenyl]mehtyl]urea, (15.101) 1,3-dimethoxy-1-[[4-[5-(trifluoromehtyl)-1,2,4-oxadiazol-3-yl]phenyl]methyl]urea, (15.102) 3-ethyl-1-metho xy-1-[[4-[5-(trifluoromethyl)-1,2,4-oxadiazol-3-yl]phenyl]methyl]ur ea, (15.103) 1-[[4-[5-(t rifluoromethyl) -1,2, 4-oxad iazol-3-yl]phenyl]methyl]piperidin-2-one, (15.104) 4,4-dimethyl-2-[[4-(5-(trifluoromethyl)-1,2,4-oxadiazol-3-yl]phenyl]methyl]isooxazolidin-3-one, (15.105) 5,5-dimelhyl-2-[[4-[5-(trifluoromethyl)-1,2,4-oxadiazol-3-yl]phenyl]methyl]isoxazolidin-3-one, (15.106), 3,3-dimethyl-1-[[4-[5-(trifluoromethyl)-1,2,4-oxadiazol-3-yl]phenyl]methyl[piperidin-2-one, (15.107) 1-[[3-fluoro-4-(5-(trifluoromelhyl)-1,2,4-oxadiazol-3-yl]phenyl]methyl] azepan-2-one, (15.108) 4,4-dimethyl-2- [[4-(5-(trifluoromethyl)-1,2,4-oxadiazol-3-yl]phenyl]methyl]isoxazolidin-3-one and (15.109) 5,5-dimethyl-2-[[4-[5-(trifluoromethyl) -1,2,4- oxadiazol-3-yl]phenyl]methyl]isoxazolidin-3-one.

More preferred compound combinations according to the invention comprise (B) at least one further active compound selected from:

(1.001) cyproconazole, (1.002) difenoconazole, (1.003) epoxiconazole, (1.004) fenhexamid, (1.010) imazalil, (1.012) ipconazole, (1.013) metconazole, (1.017) propiconazole, (1.018) prothioconazole, (1.020) spiroxamine, (1.021) tebuconazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.059) 5-(4-chlorobenzyl)-2-(chloromethyl)-2-methyl-1-(1H-1,2,4-triazol-1-ylmethyl)cyclopentanol, (1.081) Mefentrifluconazole, (1.082) Ipfentrifluconazole, and (1.083) 2-[6-(4-bromophenoxy)-2-(trifluoromethyl)-3-pyridyl]-1-(1,2,4-triazol-1-yl)propan-2-ol,

(2.001) benzovindiflupyr, (2.002) bixafen, (2.003) boscalid, (2.005) fluopyram, (2.007) fluxapyroxad, (2.009) Isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.021) sedaxane, (2.027) 3-(difluoromethyl)-1-methyl-N-(1,1,3-trimethyl-2,3-dihydro-1H-inden-4-yl)-1H-pyrazole-4-carboxamide, (2.030) 3-(difluoromethyl)-N-(7-fluoro-1,1,3-trimethyl-2,3-dihydro-1H-inden-4-yl)-1 -methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide,

(3.003) azoxystrobin, (3.007) dimoxystrobin, (3.012) fluoxastrobin, (3.013) kresoxim-methyl, (3.016) picoxystrobin, (3.017) pyraclostrobin, (3.020) trifloxystrobin, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) 2-{2-[(2,5-dimethylphenoxy)methyl]phenyl}-2-methoxy-N-methylacetamide, (3.030) (1S)-2,2-bis(4-fluorophenyl)-1-methylethyl N-{[3-(acetyloxy)-4-methoxy-2-pyridyl]carbonyl}-L-alaninate,

(4.005) pencycuron, (4.007) thiophanate-methyl, (4.012) 4-(2-bromo-4-fluorophenyl)-N-(2,6-difluorophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine,

(5.003) captan, (5.004) chlorothalonil, (5.011) dodine, (5.012) folpet, (5.013) mancozeb, (5.015) metiram, (5.018) propineb,

(6.002) isotianil,

(7.001) cyprodinil, (7.005) pyrimethanil,

(12.003) metalaxyl, (12.004) metalaxyl-M (mefenoxam),

(13.001) fludioxonil, (13.002) iprodione, (13.004) proquinazid, (13.005) quinoxyfen,

(14.001) fluazinam, (14.002) meptyldinocap,

(15.008) cyflufenamid, (15.010) cyprosulfamide, (15.011) flutianil, (15.012) fosetyl-aluminium, (15.016) metrafenone, (15.027) pyriofenone (chlazafenone), and (15.047) 3-(4,4-difluoro-3,3-dimethyl-3,4-dihydroisoquinolin-1-yl)quinolone, (15.048) 4-amino-5-fluoropyrimidin-2-ol (tautomeric form: 4-amino-5-fluoropyrimidin-2(1H)-one), (15.052) 5-fluoro-2-[(4-fluorobenzyl)oxy]pyrimidin-4-amine, (15.053) 5-fluoro-2- [(4-methylbenzyl)oxy]pyrimidin-4-amine, (15.062) 5-fluoro-4-imino-3-methyl-1-[(4-methylphenyl)sulfonyl]-3,4-dihydropyrimidin-2(1H)-one.

Even more preferred compound combinations according to the invention comprise (B) at least one further active compound selected from:

(1.002) difenoconazole, (1.010) imazalil, (1.012) ipconazole, (1.018) prothioconazole, (1.020) spiroxamine, (1.021) tebuconazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.059) 5-(4-chlorobenzyl)-2-(chloromethyl)-2-methyl-1-(1H-1,2,4-triazol-1-ylmethyl)cyclopentanol, (1.081) Mefentrifluconazole, (1.082) Ipfentrifluconazole, and and (1.083) 2-[6-(4-bromophenoxy)-2-(trifluoromethyl)-3-pyridyl]-1-(1,2,4-triazol-1-yl)propan-2-ol,

(2.001) benzovindiflupyr, (2.002) bixafen, (2.005) fluopyram, (2.007) fluxapyroxad, (2.017) penflufen, (2.018) penthiopyrad, (2.019) pydiflumetofen, (2.021) sedaxane, (2.027) 3-(difluoromethyl)-1-methyl-N-(1,1,3-trimethyl-2,3-dihydro-1H-inden-4-yl)-1H-pyrazole-4-carboxamide, (2.030) 3-(difluoromethyl)-N-(7-fluoro-1,1,3-trimethyl-2,3-dihydro-1H-inden-4-yl)-1 -methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide,

(3.003) azoxystrobin, (3.012) fluoxastrobin, (3.016) picoxystrobin, (3.017) pyraclostrobin, (3.020) trifloxystrobin, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) 2-{2-[(2,5-dimethylphenoxy)methyl]phenyl}-2-methoxy-N-methylacetamide, (3.030) (1S)-2,2-bis(4-fluorophenyl)-1-methylethyl N-{[3-(acetyloxy)-4-methoxy-2-pyridyl]carbonyl}-L-alaninate,

(4.005) pencycuron, (4.007) thiophanate-methyl, (4.012) 4-(2-bromo-4-fluorophenyl)-N-(2,6-difluorophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine,

(5.004) chlorothalonil, (5.011) dodine, (5.012) folpet, (5.013) mancozeb, (5.018) propineb,

(6.002) isotianil,

(7.005) pyrimethanil,

(12.003) metalaxyl, (12.004) metalaxyl-M (mefenoxam),

(13.001) fludioxonil, (13.004) proquinazid,

(14.001) fluazinam, (14.002) meptyldinocap,

(15.008) cyflufenamid, (15.012) fosetyl-aluminium, (15.027) pyriofenone (chlazafenone), (15.047) 3-(4,4-difluoro-3,3-dimethyl-3,4-dihydroisoquinolin-1-yl)quinolone, (15.048) 4-amino-5-fluoropyrimidin-2-ol (tautomeric form: 4-amino-5-fluoropyrimidin-2(1H)-one), (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.062) 5-fluoro-4-imino-3-methyl-1-[(4-methylphenyl)sulfonyl]-3,4-dihydropyrimidin-2(1H)-one.

Most preferred compound combinations according to the invention comprise (B) at least one further active compound selected from:

(1.012) ipconazole, (1.018) prothioconazole, (1.020) spiroxamine, (1.021) tebuconazole, (1.083) 2-[6-(4-bromophenoxy)-2-(trifluoromethyl)-3 -pyridyl] -1 -(1,2,4-triazol-1 -yl)propan-2-ol

(2.002) bixafen, (2.005) fluopyram, (2.017) penflufen, (2.027) 3-(difluoromethyl)-1-methyl-N-(1,1,3-trimethyl-2,3-dihydro-1H-inden-4-yl)-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide,

(3.012) fluoxastrobin, (3.020) trifloxystrobin, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.030) (1S)-2,2-bis(4-fluorophenyl)-1-methylethyl N-{[3-(acetyloxy)-4-methoxy-2-pyridyl]carbonyl}-L-alaninate,

(4.005) pencycuron,

(5.004) chlorothalonil, (5.012) folpet, (5.013) mancozeb, (5.018) propineb,

(12.003) metalaxyl, (12.004) metalaxyl-M (mefenoxam),

(13.001) fludioxonil, (13.004) proquinazid,

(15.008) cyflufenamid and (15.012) fosetyl-aluminium.

Preferred compound combinations are selected from the group (G1) consisting of the following mixtures: (I.01) + (1.001), (I.01) + (1.002), (I.01) + (1.003), (I.01) + (1.004), (I.01) + (1.005), (I.01) + (1.006), (I.01) + (1.007), (I.01) + (1.008), (I.01) + (1.009), (I.01) + (1.010), (I.01) + (1.011), (I.01) + (1.012), (I.01) + (1.013), (I.01) + (1.014), (I.01) + (1.015), (I.01) + (1.016), (I.01) + (1.017), (I.01) + (1.018), (I.01) + (1.019), (I.01) + (1.020), (I.01) + (1.021), (I.01) + (1.022), (I.01) + (1.023), (I.01) + (1.024), (I.01) + (1.025), (I.01) + (1.026), (I.01) + (1.027), (I.01) + (1.028), (I.01) + (1.029), (I.01) + (1.030), (I.01) + (1.031), (I.01) + (1.032), (I.01) + (1.033), (I.01) + (1.034), (I.01) + (1.035), (I.01) + (1.036), (I.01) + (1.037), (I.01) + (1.038), (I.01) + (1.039), (I.01) + (1.040), (I.01) + (1.041), (I.01) + (1.042), (I.01) + (1.043), (I.01) + (1.044), (I.01) + (1.045), (I.01) + (1.046), (I.01) + (1.047), (I.01) + (1.048), (I.01) + (1.049), (I.01) + (1.050), (I.01) + (1.051), (I.01) + (1.052), (I.01) + (1.053), (I.01) + (1.054), (I.01) + (1.055), (I.01) + (1.056), (I.01) + (1.057), (I.01) + (1.058), (I.01) + (1.059), (I.01) + (1.060), (I.01) + (1.061), (I.01) + (1.062), (I.01) + (1.063), (I.01) + (1.064), (I.01) + (1.065), (I.01) + (1.066), (I.01) + (1.067), (I.01) + (1.068), (I.01) + (1.069), (I.01) + (1.070), (I.01) + (1.071), (I.01) + (1.072), (I.01) + (1.073), (I.01) + (1.074), (I.01) + (1.075), (I.01) + (1.076), (I.01) + (1.077), (I.01) + (1.078), (I.01) + (1.079), (I.01) + (1.080), (I.01) + (1.081), (I.01) + (1.082), (I.01) + (1.083), (I.01) + (2.001), (I.01) + (2.002), (I.01) + (2.003), (I.01) + (2.004), (I.01) + (2.005), (I.01) + (2.006), (I.01) + (2.007), (I.01) + (2.008), (I.01) + (2.009), (I.01) + (2.010), (I.01) + (2.011), (I.01) + (2.012), (I.01) + (2.013), (I.01) + (2.014), (I.01) + (2.015), (I.01) + (2.016), (I.01) + (2.017), (I.01) + (2.018), (I.01) + (2.019), (I.01) + (2.020), (I.01) + (2.021), (I.01) + (2.022), (I.01) + (2.023), (I.01) + (2.024), (I.01) + (2.025), (I.01) + (2.026), (I.01) + (2.027), (I.01) + (2.028), (I.01) + (2.029), (I.01) + (2.030), (I.01) + (2.031), (I.01) + (2.032), (I.01) + (2.033), (I.01) + (2.034), (I.01) + (2.035), (I.01) + (2.036), (I.01) + (2.037), (I.01) + (2.038), (I.01) + (2.039), (I.01) + (2.040), (I.01) + (2.041), (I.01) +

(2.042), (I.01) + (2.043), (I.01) + (2.044), (I.01) + (2.045), (I.01) + (2.046), (I.01) + (2.047), (I.01) + (2.048), (I.01) + (2.049), (I.01) + (2.050), (I.01) + (2.051), (I.01) + (2.052), (I.01) + (2.053), (I.01) + (2.054), (I.01) + (2.055), (I.01) + (2.056), (I.01) + (3.001), (I.01) + (3.002), (I.01) + (3.003), (I.01) + (3.004), (I.01) + (3.005), (I.01) + (3.006), (I.01) + (3.007), (I.01) + (3.008), (I.01) + (3.009), (I.01) + (3.010), (I.01) + (3.011), (I.01) + (3.012), (I.01) + (3.013), (I.01) + (3.014), (I.01) + (3.015), (I.01) + (3.016), (I.01) + (3.017), (I.01) + (3.018), (I.01) + (3.019), (I.01) + (3.020), (I.01) + (3.021), (I.01) + (3.022), (I.01) + (3.023), (I.01) + (3.024), (I.01) + (3.025), (I.01) + (3.026), (I.01) + (3.027), (I.01) + (3.028), (I.01) + (3.029), (I.01) + (3.030), (1.01) + (4.001), (1.01) + (4.002), (1.01) + (4.003), (1.01) + (4.004), (1.01) + (4.005), (1.01) + (4.006), (1.01) + (4.007), (1.01) + (4.008), (1.01) + (4.009), (1.01) + (4.010), (1.01) + (4.011), (1.01) + (4.012), (1.01) + (4.013), (1.01) + (4.014), (1.01) + (4.015), (1.01) + (4.016), (1.01) + (4.017), (1.01) + (4.018), (1.01) + (4.019), (1.01) + (4.020), (1.01) + (4.021), (1.01) + (4.022), (1.01) + (4.023), (1.01) + (4.024), (1.01) + (4.025), (1.01) + (5.001), (1.01) + (5.002), (1.01) + (5.003), (1.01) + (5.004), (1.01) + (5.005), (I.01) + (5.006), (I.01) + (5.007), (I.01) + (5.008), (I.01) + (5.009), (I.01) + (5.010), (I.01) + (5.011), (I.01) + (5.012), (I.01) + (5.013), (I.01) + (5.014), (I.01) + (5.015), (I.01) + (5.016), (I.01) + (5.017), (I.01) + (5.018), (I.01) + (5.019), (I.01) + (5.020), (I.01) + (5.021), (I.01) + (5.022), (I.01) + (5.023), (I.01) + (6.001), (I.01) + (6.002), (I.01) + (6.003), (I.01) + (6.004), (I.01) + (7.001), (I.01) + (7.002), (I.01) + (7.003), (I.01) + (7.004), (I.01) + (7.005), (I.01) + (7.006), (I.01) + (8.001), (I.01) + (9.001), (I.01) + (9.002), (I.01) + (9.003), (I.01) + (9.004), (I.01) + (9.005), (I.01) + (9.006), (I.01) + (9.007), (I.01) + (9.008), (I.01) + (9.009), (I.01) + (10.001), (I.01) + (10.002), (I.01) + (10.003), (I.01) + (11.001), (I.01) + (11.002), (I.01) + (12.001), (I.01) + (12.002), (I.01) + (12.003), (I.01) + (12.004), (I.01) + (13.001), (I.01) + (13.002), (I.01) + (13.003), (I.01) + (13.004), (I.01) + (13.005), (I.01) + (13.006), (I.01) + (14.001), (I.01) + (14.002), (I.01) + (15.001), (I.01) + (15.002), (I.01) + (15.003), (I.01) + (15.004), (I.01) + (15.005), (I.01) + (15.006), (I.01) + (15.007), (I.01) + (15.008), (I.01) + (15.009), (I.01) + (15.010), (I.01) + (15.011), (I.01) + (15.012), (I.01) + (15.013), (I.01) + (15.014), (I.01) + (15.015), (I.01) + (15.016), (I.01) + (15.017), (I.01) + (15.018), (I.01) + (15.019), (I.01) + (15.020), (I.01) + (15.021), (I.01) + (15.022), (I.01) + (15.023), (I.01) + (15.024), (I.01) + (15.025), (I.01) + (15.026), (I.01) + (15.027), (I.01) + (15.028), (I.01) + (15.029), (I.01) + (15.030), (I.01) + (15.031), (I.01) + (15.032), (I.01) + (15.033), (I.01) + (15.034), (I.01) + (15.035), (I.01) + (15.036), (I.01) + (15.037), (I.01) + (15.038), (I.01) + (15.039), (I.01) + (15.040), (I.01) + (15.041), (I.01) + (15.042), (I.01) + (15.043), (I.01) + (15.044), (I.01) + (15.045), (I.01) + (15.046), (I.01) + (15.047), (I.01) + (15.048), (I.01) + (15.049), (I.01) + (15.050), (I.01) + (15.051), (I.01) + (15.052), (I.01) + (15.053), (I.01) + (15.054), (I.01) + (15.055), (I.01) + (15.056), (I.01) + (15.057), (I.01) + (15.058), (I.01) + (15.059), (I.01) + (15.060), (I.01) + (15.061), (I.01) + (15.062). (I.01) + (15.063). (I.01) + (15.064). (I.01) + (15.065), (I.01) + 15.066), (I.01) + (15.067), (I.01) + (15.068), (I.01) + (15.069) , (I.01) + (15.070), (I.01) + (15.071), (I.01) + (15.072), (I.01) + (15.073), (I.01) + (15.074), (I.01) + (15.075), (I.01) + (15.076), (1.01), (I.01) + (15.077), (I.01) + (15.078), (I.01) + (15.079), (I.01) + (15.080), (I.01) + (15.081), (I.01) + (15.082), (I.01) + (15.083), (I.01) + (15.084), (I.01) + (15.085), (I.01) + (15.086), (I.01) + (15.087), (I.01) + (15.088), (I.01) + (15.089), (I.01) + (15.090), (I.01) + (15.091) , (I.01) + (15.092), (I.01) + (15.093), (I.01) + (15.094), (I.01) + (15.095), (I.01) + (15.096), (I.01) + (15.097), (I.01) + (15.098), (I.01) + (15.099), (I.01) + (15.100), (I.01) + (15.101), (I.01) + (15.102), (I.01) + (15.103), (I.01) + (15.104), (I.01) + (15.105), (I.01) + (15.106), (I.01) + (15.107), (I.01) + (15.108), (I.01) + (15.109).

Also preferred compound combinations are selected from the group (G2) consisting of the following mixtures: (I.02) + (1.001), (I.02) + (1.002), (I.02) + (1.003), (I.02) + (1.004), (I.02) + (1.005), (I.02) + (1.006), (I.02) + (1.007), (I.02) + (1.008), (I.02) + (1.009), (I.02) + (1.010), (I.02) + (1.011), (I.02) + (1.012), (I.02) + (1.013), (I.02) + (1.014), (I.02) + (1.015), (I.02) + (1.016), (I.02) + (1.017), (I.02) + (1.018), (I.02) + (1.019), (I.02) + (1.020), (I.02) + (1.021), (I.02) + (1.022), (I.02) + (1.023), (I.02) + (1.024), (I.02) + (1.025), (I.02) + (1.026), (I.02) + (1.027), (I.02) + (1.028), (I.02) + (1.029), (I.02) + (1.030), (I.02) + (1.031), (I.02) + (1.032), (I.02) + (1.033), (I.02) + (1.034), (I.02) + (1.035), (I.02) + (1.036), (I.02) + (1.037), (I.02) + (1.038), (I.02) + (1.039), (I.02) + (1.040), (I.02) + (1.041), (I.02) + (1.042), (I.02) + (1.043), (I.02) + (1.044), (I.02) + (1.045), (I.02) + (1.046), (I.02) + (1.047), (I.02) + (1.048), (I.02) + (1.049), (I.02) + (1.050), (I.02) + (1.051), (I.02) + (1.052), (I.02) + (1.053), (I.02) + (1.054), (I.02) + (1.055), (I.02) + (1.056), (I.02) + (1.057), (I.02) + (1.058), (I.02) + (1.059), (I.02) + (1.060), (I.02) + (1.061), (I.02) + (1.062), (I.02) + (1.063), (I.02) + (1.064), (I.02) + (1.065), (I.02) + (1.066), (I.02) + (1.067), (I.02) + (1.068), (I.02) + (1.069), (I.02) + (1.070), (I.02) + (1.071), (I.02) + (1.072), (I.02) + (1.073), (I.02) + (1.074), (I.02) + (1.075), (I.02) + (1.076), (I.02) + (1.077), (I.02) + (1.078), (I.02) + (1.079), (I.02) + (1.080), (I.02) + (1.081), (I.02) + (1.082), (I.02) + (1.083), (I.02) + (2.001), (I.02) + (2.002), (I.02) + (2.003), (I.02) + (2.004), (I.02) + (2.005), (I.02) + (2.006), (I.02) + (2.007), (I.02) + (2.008), (I.02) + (2.009), (I.02) + (2.010), (I.02) + (2.011), (I.02) + (2.012), (I.02) + (2.013), (I.02) + (2.014), (I.02) + (2.015), (I.02) + (2.016), (I.02) + (2.017), (I.02) + (2.018), (I.02) + (2.019), (I.02) + (2.020), (I.02) + (2.021), (I.02) + (2.022), (I.02) + (2.023), (I.02) + (2.024), (I.02) + (2.025), (I.02) + (2.026), (I.02) + (2.027), (I.02) + (2.028), (I.02) + (2.029), (I.02) + (2.030), (I.02) + (2.031), (I.02) + (2.032), (I.02) + (2.033), (I.02) + (2.034), (I.02) + (2.035), (I.02) + (2.036), (I.02) + (2.037), (I.02) + (2.038), (I.02) + (2.039), (I.02) + (2.040), (I.02) + (2.041), (I.02) + (2.042), (I.02) + (2.043), (I.02) + (2.044), (I.02) + (2.045), (I.02) + (2.046), (I.02) + (2.047), (I.02) + (2.048), (I.02) + (2.049), (I.02) + (2.050), (I.02) + (2.051), (I.02) + (2.052), (I.02) + (2.053), (I.02) + (2.054), (I.02) + (2.055), (I.02) + (2.056), (I.02) + (3.001), (I.02) + (3.002), (I.02) + (3.003), (I.02) + (3.004), (I.02) + (3.005), (I.02) + (3.006), (I.02) + (3.007), (I.02) + (3.008), (I.02) + (3.009), (I.02) + (3.010), (I.02) + (3.011), (I.02) + (3.012), (I.02) + (3.013), (I.02) + (3.014), (I.02) + (3.015), (I.02) + (3.016), (I.02) + (3.017), (I.02) + (3.018), (I.02) + (3.019), (I.02) + (3.020), (I.02) + (3.021), (I.02) + (3.022), (I.02) + (3.023), (I.02) + (3.024), (I.02) + (3.025), (I.02) + (3.026), (I.02) + (3.027), (I.02) + (3.028), (I.02) + (3.029), (I.02) +

(3.030), (I.02) + (4.001), (I.02) + (4.002), (I.02) + (4.003), (I.02) + (4.004), (I.02) + (4.005), (I.02) + (4.006), (I.02) + (4.007), (I.02) + (4.008), (I.02) + (4.009), (I.02) + (4.010), (I.02) + (4.011), (I.02) + (4.012), (I.02) + (4.013), (I.02) + (4.014), (I.02) + (4.015), (I.02) + (4.016), (I.02) + (4.017), (I.02) + (4.018), (I.02) + (4.019), (I.02) + (4.020), (I.02) + (4.021), (I.02) + (4.022), (I.02) + (4.023), (I.02) + (4.024), (I.02) + (4.025), (I.02) + (5.001), (I.02) + (5.002), (I.02) + (5.003), (I.02) + (5.004), (I.02) + (5.005), (I.02) + (5.006), (I.02) + (5.007), (I.02) + (5.008), (I.02) + (5.009), (I.02) + (5.010), (I.02) + (5.011), (I.02) + (5.012), (I.02) + (5.013), (I.02) + (5.014), (I.02) + (5.015), (I.02) + (5.016), (I.02) + (5.017), (I.02) + (5.018), (I.02) + (5.019), (I.02) + (5.020), (I.02) + (5.021), (I.02) + (5.022), (I.02) + (5.023), (I.02) + (6.001), (I.02) + (6.002), (I.02) + (6.003), (I.02) + (6.004), (I.02) + (7.001), (I.02) + (7.002), (I.02) + (7.003), (I.02) + (7.004), (I.02) + (7.005), (I.02) + (7.006), (I.02) + (8.001), (I.02) + (9.001), (I.02) + (9.002), (I.02) + (9.003), (I.02) + (9.004), (I.02) + (9.005), (I.02) + (9.006), (I.02) + (9.007), (I.02) + (9.008), (I.02) + (9.009), (I.02) + (10.001), (I.02) + (10.002), (I.02) + (10.003), (I.02) + (11.001), (I.02) + (11.002), (I.02) + (12.001), (I.02) + (12.002), (I.02) + (12.003), (I.02) + (12.004), (I.02) + (13.001), (I.02) + (13.002), (I.02) + (13.003), (I.02) + (13.004), (I.02) + (13.005), (I.02) + (13.006), (I.02) + (14.001), (I.02) + (14.002), (I.02) + (15.001), (I.02) + (15.002), (I.02) + (15.003), (I.02) + (15.004), (I.02) + (15.005), (I.02) + (15.006), (I.02) + (15.007), (I.02) + (15.008), (I.02) + (15.009), (I.02) + (15.010), (I.02) + (15.011), (I.02) + (15.012), (I.02) + (15.013), (I.02) + (15.014), (I.02) + (15.015), (I.02) + (15.016), (I.02) + (15.017), (I.02) + (15.018), (I.02) + (15.019), (I.02) + (15.020), (I.02) + (15.021), (I.02) + (15.022), (I.02) + (15.023), (I.02) + (15.024), (I.02) + (15.025), (I.02) + (15.026), (I.02) + (15.027), (I.02) + (15.028), (I.02) + (15.029), (I.02) + (15.030), (I.02) + (15.031), (I.02) + (15.032), (I.02) + (15.033), (I.02) + (15.034), (I.02) + (15.035), (I.02) + (15.036), (I.02) + (15.037), (I.02) + (15.038), (I.02) + (15.039), (I.02) + (15.040), (I.02) + (15.041), (I.02) + (15.042), (I.02) + (15.043), (I.02) + (15.044), (I.02) + (15.045), (I.02) + (15.046), (I.02) + (15.047), (I.02) + (15.048), (I.02) + (15.049), (I.02) + (15.050), (I.02) + (15.051), (I.02) + (15.052), (I.02) + (15.053), (I.02) + (15.054), (I.02) + (15.055), (I.02) + (15.056), (I.02) + (15.057), (I.02) + (15.058), (I.02) + (15.059), (I.02) + (15.060), (I.02) + (15.061), (I.02) + (15.062). (I.02) + (15.063). (1.02) + (15.064,) and (I.02) + (15.065), (I.02) + 15.066), (I.02) + (15.067), (I.02) + (15.068), (I.02) + (15.069) , (I.02) + (15.070), (I.02) + (15.071), (I.02) + (15.072), (I.02) + (15.073), (I.02) + (15.074), (I.02) + (15.075), (I.02) + (15.076), (I.02), (I.02) + (15.077), (I.02) + (15.078), (I.02) + (15.079), (I.02) + (15. 080), (I.02) + (15.081), (I.02) + (15.082), (I.02) + (15.083), (I.02) + (15.084), (I.02) + (15.085), (I.02) + (15.086), (I.02) + (15.087), (I.02) + (15.088), (I.02) + (15.089), (I.02) + (15.090), (I.02) + (15.091), (I.02) + (15.092), (I.02) + (15.093), (I.02) + (15.094), (I.02) + (15.095), (I.02) + (15.096), (I.02) + (15.097), (I.02) + (15.098), (I.02) + (15.099), (I.02) + (15.100), (I.02) + (15.101), (I.02) + (15.102), (I.02) + (15.103), (I.02) + (15.104), (I.02) + (15.105), (I.02) + (15.106), (I.02) + (15.107), (I.02) + (15.108), (I.02) + (15.109).

Also preferred compound combinations are selected from the group (G3) consisting of the following mixtures: (I.03) + (1.001), (I.03) + (1.002), (I.03) + (1.003), (I.03) + (1.004), (I.03) + (1.005), (I.03) + (1.006), (I.03) + (1.007), (I.03) + (1.008), (I.03) + (1.009), (I.03) + (1.010), (I.03) + (1.011), (I.03) + (1.012), (I.03) + (1.013), (I.03) + (1.014), (I.03) + (1.015), (I.03) + (1.016), (I.03) + (1.017), (I.03) + (1.018), (I.03) + (1.019), (I.03) + (1.020), (I.03) + (1.021), (I.03) + (1.022), (I.03) + (1.023), (I.03) + (1.024), (I.03) + (1.025), (I.03) + (1.026), (I.03) + (1.027), (I.03) + (1.028), (I.03) + (1.029), (I.03) + (1.030), (I.03) + (1.031), (I.03) + (1.032), (I.03) + (1.033), (I.03) + (1.034), (I.03) + (1.035), (I.03) + (1.036), (I.03) + (1.037), (I.03) + (1.038), (I.03) + (1.039), (I.03) + (1.040), (I.03) + (1.041), (I.03) + (1.042), (I.03) + (1.043), (I.03) + (1.044), (I.03) + (1.045), (I.03) + (1.046), (I.03) + (1.047), (I.03) + (1.048), (I.03) + (1.049), (I.03) + (1.050), (I.03) + (1.051), (I.03) + (1.052), (I.03) + (1.053), (I.03) + (1.054), (I.03) + (1.055), (I.03) + (1.056), (I.03) + (1.057), (I.03) + (1.058), (I.03) + (1.059), (I.03) + (1.060), (I.03) + (1.061), (I.03) + (1.062), (I.03) + (1.063), (I.03) + (1.064), (I.03) + (1.065), (I.03) + (1.066), (I.03) + (1.067), (I.03) + (1.068), (I.03) + (1.069), (I.03) + (1.070), (I.03) + (1.071), (I.03) + (1.072), (I.03) + (1.073), (I.03) + (1.074), (I.03) + (1.075), (I.03) + (1.076), (I.03) + (1.077), (I.03) + (1.078), (I.03) + (1.079), (I.03) + (1.080), (I.03) + (1.081), (I.03) + (1.082), (I.03) + (1.083), (I.03) + (2.001), (I.03) + (2.002), (I.03) + (2.003), (I.03) + (2.004), (I.03) + (2.005), (I.03) + (2.006), (I.03) + (2.007), (I.03) + (2.008), (I.03) + (2.009), (I.03) + (2.010), (I.03) + (2.011), (I.03) + (2.012), (I.03) + (2.013), (I.03) + (2.014), (I.03) + (2.015), (I.03) + (2.016), (I.03) + (2.017), (I.03) + (2.018), (I.03) + (2.019), (I.03) + (2.020), (I.03) + (2.021), (I.03) + (2.022), (I.03) + (2.023), (I.03) + (2.024), (I.03) + (2.025), (I.03) + (2.026), (I.03) + (2.027), (I.03) + (2.028), (I.03) + (2.029), (I.03) + (2.030), (I.03) + (2.031), (I.03) + (2.032), (I.03) + (2.033), (I.03) + (2.034), (I.03) + (2.035), (I.03) + (2.036), (I.03) + (2.037), (I.03) + (2.038), (I.03) + (2.039), (I.03) + (2.040), (I.03) + (2.041), (I.03) + (2.042), (I.03) + (2.043), (I.03) + (2.044), (I.03) + (2.045), (I.03) + (2.046), (I.03) + (2.047), (I.03) + (2.048), (I.03) + (2.049), (I.03) + (2.050), (I.03) + (2.051), (I.03) + (2.052), (I.03) + (2.053), (I.03) + (2.054), (I.03) + (2.055), (I.03) + (2.056), (I.03) + (3.001), (I.03) + (3.002), (I.03) + (3.003), (I.03) + (3.004), (I.03) + (3.005), (I.03) + (3.006), (I.03) + (3.007), (I.03) + (3.008), (I.03) + (3.009), (I.03) + (3.010), (I.03) + (3.011), (I.03) + (3.012), (I.03) + (3.013), (I.03) + (3.014), (I.03) + (3.015), (I.03) + (3.016), (I.03) + (3.017), (I.03) + (3.018), (I.03) + (3.019), (I.03) + (3.020), (I.03) + (3.021), (I.03) + (3.022), (I.03) + (3.023), (I.03) + (3.024), (I.03) + (3.025), (I.03) + (3.026), (I.03) + (3.027), (I.03) + (3.028), (I.03) + (3.029), (I.03) + (3.030), (I.03) + (4.001), (I.03) + (4.002), (I.03) + (4.003), (I.03) + (4.004), (I.03) + (4.005), (I.03) + (4.006), (I.03) + (4.007), (I.03) + (4.008), (I.03) + (4.009), (I.03) + (4.010), (I.03) + (4.011), (I.03) + (4.012), (I.03) + (4.013), (I.03) + (4.014), (I.03) + (4.015), (I.03) + (4.016), (I.03) + (4.017), (I.03) + (4.018), (I.03) + (4.019), (I.03) + (4.020), (I.03) + (4.021), (I.03) + (4.022), (I.03) + (4.023), (I.03) + (4.024), (I.03) + (4.025), (I.03) + (5.001), (I.03) + (5.002), (I.03) + (5.003), (I.03) + (5.004), (I.03) + (5.005), (I.03) + (5.006), (I.03) + (5.007), (I.03) + (5.008), (I.03) + (5.009), (I.03) + (5.010), (I.03) + (5.011), (I.03) + (5.012), (I.03) + (5.013), (I.03) + (5.014), (I.03) + (5.015), (I.03) + (5.016), (I.03) + (5.017), (I.03) + (5.018), (I.03) +

(5.019), (I.03) + (5.020), (I.03) + (5.021), (I.03) + (5.022), (I.03) + (5.023), (I.03) + (6.001), (I.03) + (6.002), (I.03) + (6.003), (I.03) + (6.004), (1.03) + (7.001), (I.03) + (7.002), (I.03) + (7.003), (I.03) + (7.004), (I.03) + (7.005), (I.03) + (7.006), (I.03) + (8.001), (I.03) + (9.001), (I.03) + (9.002), (I.03) + (9.003), (I.03) + (9.004), (I.03) + (9.005), (I.03) + (9.006), (I.03) + (9.007), (I.03) + (9.008), (I.03) + (9.009), (I.03) + (10.001), (I.03) + (10.002), (I.03) + (10.003), (I.03) + (11.001), (I.03) + (11.002), (I.03) + (12.001), (I.03) + (12.002), (I.03) + (12.003), (I.03) + (12.004), (I.03) + (13.001), (I.03) + (13.002), (I.03) + (13.003), (I.03) + (13.004), (I.03) + (13.005), (I.03) + (13.006), (I.03) + (14.001), (I.03) + (14.002), (I.03) + (15.001), (I.03) + (15.002), (I.03) + (15.003), (I.03) + (15.004), (I.03) + (15.005), (I.03) + (15.006), (I.03) + (15.007), (I.03) + (15.008), (I.03) + (15.009), (I.03) + (15.010), (I.03) + (15.011), (I.03) + (15.012), (I.03) + (15.013), (I.03) + (15.014), (I.03) + (15.015), (I.03) + (15.016), (I.03) + (15.017), (I.03) + (15.018), (I.03) + (15.019), (I.03) + (15.020), (I.03) + (15.021), (I.03) + (15.022), (I.03) + (15.023), (I.03) + (15.024), (I.03) + (15.025), (I.03) + (15.026), (I.03) + (15.027), (I.03) + (15.028), (I.03) + (15.029), (I.03) + (15.030), (I.03) + (15.031), (I.03) + (15.032), (I.03) + (15.033), (I.03) + (15.034), (I.03) + (15.035), (I.03) + (15.036), (I.03) + (15.037), (I.03) + (15.038), (I.03) + (15.039), (I.03) + (15.040), (I.03) + (15.041), (I.03) + (15.042), (I.03) + (15.043), (I.03) + (15.044), (I.03) + (15.045), (I.03) + (15.046), (I.03) + (15.047), (I.03) + (15.048), (I.03) + (15.049), (I.03) + (15.050), (I.03) + (15.051), (I.03) + (15.052), (I.03) + (15.053), (I.03) + (15.054), (I.03) + (15.055), (I.03) + (15.056), (I.03) + (15.057), (I.03) + (15.058), (I.03) + (15.059), (I.03) + (15.060), (I.03) + (15.061), (I.03) + (15.062). (I.03) + (15.063). (I.03) + (15.064). and (I.03) + (15.062065), (I.03) + 15.066), (I.03) + (15.067), (I.03) + (15.068), (I.03) + (15.069), (I.03) + (15.070), (I.03) + (15.071), (I.03) + (15.072), (I.03) + (15.073), (I.03) + (15.074), (I.03) + (15.075), (I.03) + (15.076), (I.03), (I.03) + (15.077), (I.03) + (15.078), (I.03) + (15.079), (I.03) + (15. 080), (I.03) + (15.081), (I.03) + (15.082), (I.03) + (15.083), (I.03) + (15.084), (I.03) + (15.085), (I.03) + (15.086), (I.03) + (15.087), (I.03) + (15.088), (I.03) + (15.089), (I.03) + (15.090), (I.03) + (15.091), (I.03) + (15.092), (I.03) + (15.093), (I.03) + (15.094), (I.03) + (15.095), (I.03) + (15.096), (I.03) + (15.097), (I.03) + (15.098), (I.03) + (15.099), (I.03) + (15.100), (I.03) + (15.101), (I.03) + (15.102), (I.03) + (15.103), (I.03) + (15.104), (I.03) + (15.105), (I.03) + (15.106), (I.03) + (15.107), (I.03) + (15.108), (I.03) + (15.109).

Also preferred compound combinations are selected from the group (G4) consisting of the following mixtures: (I.04) + (1.001), (I.04) + (1.002), (I.04) + (1.003), (I.04) + (1.004), (I.04) + (1.005), (I.04) + (1.006), (I.04) + (1.007), (I.04) + (1.008), (I.04) + (1.009), (I.04) + (1.010), (I.04) + (1.011), (I.04) + (1.012), (I.04) + (1.013), (I.04) + (1.014), (I.04) + (1.015), (I.04) + (1.016), (I.04) + (1.017), (I.04) + (1.018), (I.04) + (1.019), (I.04) + (1.020), (I.04) + (1.021), (I.04) + (1.022), (I.04) + (1.023), (I.04) + (1.024), (I.04) + (1.025), (I.04) + (1.026), (I.04) + (1.027), (I.04) + (1.028), (I.04) + (1.029), (I.04) + (1.030), (I.04) + (1.031), (I.04) + (1.032), (I.04) + (1.033), (I.04) + (1.034), (I.04) + (1.035), (I.04) + (1.036), (I.04) + (1.037), (I.04) + (1.038), (I.04) + (1.039), (I.04) + (1.040), (I.04) + (1.041), (I.04) + (1.042), (I.04) + (1.043), (I.04) + (1.044), (I.04) + (1.045), (I.04) + (1.046), (I.04) + (1.047), (I.04) + (1.048), (I.04) + (1.049), (I.04) + (1.050), (I.04) + (1.051), (I.04) + (1.052), (I.04) + (1.053), (I.04) + (1.054), (I.04) + (1.055), (I.04) + (1.056), (I.04) + (1.057), (I.04) + (1.058), (I.04) + (1.059), (I.04) + (1.060), (I.04) + (1.061), (I.04) + (1.062), (I.04) + (1.063), (I.04) + (1.064), (I.04) + (1.065), (I.04) + (1.066), (I.04) + (1.067), (I.04) + (1.068), (I.04) + (1.069), (I.04) + (1.070), (I.04) + (1.071), (I.04) + (1.072), (I.04) + (1.073), (I.04) + (1.074), (I.04) + (1.075), (I.04) + (1.076), (I.04) + (1.077), (I.04) + (1.078), (I.04) + (1.079), (I.04) + (1.080), (I.04) + (1.081), (I.04) + (1.082), (I.04) + (1.083), (I.04) + (2.001), (I.04) + (2.002), (I.04) + (2.003), (I.04) + (2.004), (I.04) + (2.005), (I.04) + (2.006), (I.04) + (2.007), (I.04) + (2.008), (I.04) + (2.009), (I.04) + (2.010), (I.04) + (2.011), (I.04) + (2.012), (I.04) + (2.013), (I.04) + (2.014), (I.04) + (2.015), (I.04) + (2.016), (I.04) + (2.017), (I.04) + (2.018), (I.04) + (2.019), (I.04) + (2.020), (I.04) + (2.021), (I.04) + (2.022), (I.04) + (2.023), (I.04) + (2.024), (I.04) + (2.025), (I.04) + (2.026), (I.04) + (2.027), (I.04) + (2.028), (I.04) + (2.029), (I.04) + (2.030), (I.04) + (2.031), (I.04) + (2.032), (I.04) + (2.033), (I.04) + (2.034), (I.04) + (2.035), (I.04) + (2.036), (I.04) + (2.037), (I.04) + (2.038), (I.04) + (2.039), (I.04) + (2.040), (I.04) + (2.041), (I.04) + (2.042), (I.04) + (2.043), (I.04) + (2.044), (I.04) + (2.045), (I.04) + (2.046), (I.04) + (2.047), (I.04) + (2.048), (I.04) + (2.049), (I.04) + (2.050), (I.04) + (2.051), (I.04) + (2.052), (I.04) + (2.053), (I.04) + (2.054), (I.04) + (2.055), (I.04) + (2.056), (I.04) + (3.001), (I.04) + (3.002), (I.04) + (3.003), (I.04) + (3.004), (I.04) + (3.005), (I.04) + (3.006), (I.04) + (3.007), (I.04) + (3.008), (I.04) + (3.009), (I.04) + (3.010), (I.04) + (3.011), (I.04) + (3.012), (I.04) + (3.013), (I.04) + (3.014), (I.04) + (3.015), (I.04) + (3.016), (I.04) + (3.017), (I.04) + (3.018), (I.04) + (3.019), (I.04) + (3.020), (I.04) + (3.021), (I.04) + (3.022), (I.04) + (3.023), (I.04) + (3.024), (I.04) + (3.025), (I.04) + (3.026), (I.04) + (3.027), (I.04) + (3.028), (I.04) + (3.029), (I.04) + (3.030), (I.04) + (4.001), (I.04) + (4.002), (I.04) + (4.003), (I.04) + (4.004), (I.04) + (4.005), (I.04) + (4.006), (I.04) + (4.007), (I.04) + (4.008), (I.04) + (4.009), (I.04) + (4.010), (I.04) + (4.011), (I.04) + (4.012), (I.04) + (4.013), (I.04) + (4.014), (I.04) + (4.015), (I.04) + (4.016), (I.04) + (4.017), (I.04) + (4.018), (I.04) + (4.019), (I.04) + (4.020), (I.04) + (4.021), (I.04) + (4.022), (I.04) + (4.023), (I.04) + (4.024), (I.04) + (4.025), (I.04) + (5.001), (I.04) + (5.002), (I.04) + (5.003), (I.04) + (5.004), (I.04) + (5.005), (I.04) + (5.006), (I.04) + (5.007), (I.04) + (5.008), (I.04) + (5.009), (I.04) + (5.010), (I.04) + (5.011), (I.04) + (5.012), (I.04) + (5.013), (I.04) + (5.014), (I.04) + (5.015), (I.04) + (5.016), (I.04) + (5.017), (I.04) + (5.018), (I.04) + (5.019), (I.04) + (5.020), (I.04) + (5.021), (I.04) + (5.022), (I.04) + (5.023), (I.04) + (6.001), (I.04) + (6.002), (I.04) + (6.003), (I.04) + (6.004), (I.04) + (7.001), (I.04) + (7.002), (I.04) + (7.003), (I.04) + (7.004), (I.04) + (7.005), (I.04) + (7.006), (I.04) + (8.001), (I.04) + (9.001), (I.04) + (9.002), (I.04) + (9.003), (I.04) + (9.004), (I.04) + (9.005), (I.04) + (9.006), (I.04) + (9.007), (I.04) + (9.008), (I.04) + (9.009), (I.04) + (10.001), (I.04) + (10.002), (I.04) + (10.003), (I.04) + (11.001), (I.04) + (11.002), (I.04) + (12.001), (I.04) + (12.002), (I.04) + (12.003), (I.04) + (12.004), (I.04) + (13.001), (I.04) + (13.002), (I.04) + (13.003), (I.04) + (13.004), (I.04) + (13.005), (I.04) + (13.006), (I.04) + (14.001), (I.04) + (14.002), (I.04) + (15.001),

(I.04) + (15.002), (I.04) + (15.003), (I.04) + (15.004), (I.04) + (15.005), (I.04) + (15.006), (I.04) + (15.007), (I.04) + (15.008), (I.04) + (15.009), (I.04) + (15.010), (I.04) + (15.011), (I.04) + (15.012), (I.04) + (15.013), (I.04) + (15.014), (I.04) + (15.015), (I.04) + (15.016), (I.04) + (15.017), (I.04) + (15.018), (I.04) + (15.019), (I.04) + (15.020), (I.04) + (15.021), (I.04) + (15.022), (I.04) + (15.023), (I.04) + (15.024), (I.04) + (15.025), (I.04) + (15.026), (I.04) + (15.027), (I.04) + (15.028), (I.04) + (15.029), (I.04) + (15.030), (I.04) + (15.031), (I.04) + (15.032), (I.04) + (15.033), (I.04) + (15.034), (I.04) + (15.035), (I.04) + (15.036), (I.04) + (15.037), (I.04) + (15.038), (I.04) + (15.039), (I.04) + (15.040), (I.04) + (15.041), (I.04) + (15.042), (I.04) + (15.043), (I.04) + (15.044), (I.04) + (15.045), (I.04) + (15.046), (I.04) + (15.047), (I.04) + (15.048), (I.04) + (15.049), (I.04) + (15.050), (I.04) + (15.051), (I.04) + (15.052), (I.04) + (15.053), (I.04) + (15.054), (I.04) + (15.055), (I.04) + (15.056), (I.04) + (15.057), (I.04) + (15.058), (I.04) + (15.059), (I.04) + (15.060), (I.04) + (15.061), (I.04) + (15.062). (I.04) + (15.063). (I.04) + (15.064). and (I.04) + (15.062065), (I.04) + 15.066), (I.04) + (15.067), (I.04) + (15.068), (I.04) + (15.069), (I.04) + (15.070), (I.04) + (15.071), (I.04) + (15.072), (I.04) + (15.073), (I.04) + (15.074), (I.04) + (15.075), (I.04) + (15.076), (I.04), (I.04) + (15.077), (I.04) + (15.078), (I.04) + (15.079), (I.04) + (15.080), (I.04) + (15.081), (I.04) + (15.082), (I.04) + (15.083), (I.04) + (15.084), (I.04) + (15.085), (I.04) + (15.086), (I.04) + (15.087), (I.04) + (15.088), (I.04) + (15.089), (I.04) + (15.090), (I.04) + (15.091), (I.04) + (15.092), (I.04) + (15.093), (I.04) + (15.094), (I.04) + (15.095), (I.04) + (15.096), (I.04) + (15.097), (I.04) + (15.098), (I.04) + (15.099), (I.04) + (15.100), (I.04) + (15.101), (I.04) + (15.102), (I.04) + (15.103), (I.04) + (15.104), (I.04) + (15.105), (I.04) + (15.106), (I.04) + (15.107), (I.04) + (15.108), (I.04) + (15.109).

Also preferred compound combinations are selected from the group (G5) consisting of the following mixtures: (I.05) + (1.001), (I.05) + (1.002), (I.05) + (1.003), (I.05) + (1.004), (I.05) + (1.005), (I.05) + (1.006), (I.05) + (1.007), (I.05) + (1.008), (I.05) + (1.009), (I.05) + (1.010), (I.05) + (1.011), (I.05) + (1.012), (I.05) + (1.013), (I.05) + (1.014), (I.05) + (1.015), (I.05) + (1.016), (I.05) + (1.017), (I.05) + (1.018), (I.05) + (1.019), (I.05) + (1.020), (I.05) + (1.021), (I.05) + (1.022), (I.05) + (1.023), (I.05) + (1.024), (I.05) + (1.025), (I.05) + (1.026), (I.05) + (1.027), (I.05) + (1.028), (I.05) + (1.029), (I.05) + (1.030), (I.05) + (1.031), (I.05) + (1.032), (I.05) + (1.033), (I.05) + (1.034), (I.05) + (1.035), (I.05) + (1.036), (I.05) + (1.037), (I.05) + (1.038), (I.05) + (1.039), (I.05) + (1.040), (I.05) + (1.041), (I.05) + (1.042), (I.05) + (1.043), (I.05) + (1.044), (I.05) + (1.045), (I.05) + (1.046), (I.05) + (1.047), (I.05) + (1.048), (I.05) + (1.049), (I.05) + (1.050), (I.05) + (1.051), (I.05) + (1.052), (I.05) + (1.053), (I.05) + (1.054), (I.05) + (1.055), (I.05) + (1.056), (I.05) + (1.057), (I.05) + (1.058), (I.05) + (1.059), (I.05) + (1.060), (I.05) + (1.061), (I.05) + (1.062), (I.05) + (1.063), (I.05) + (1.064), (I.05) + (1.065), (I.05) + (1.066), (I.05) + (1.067), (I.05) + (1.068), (I.05) + (1.069), (I.05) + (1.070), (I.05) + (1.071), (I.05) + (1.072), (I.05) + (1.073), (I.05) + (1.074), (I.05) + (1.075), (I.05) + (1.076), (I.05) + (1.077), (I.05) + (1.078), (I.05) + (1.079), (I.05) + (1.080), (I.05) + (1.081), (I.05) + (1.082), (I.05) + (1.083), (I.05) + (2.001), (I.05) + (2.002), (I.05) + (2.003), (I.05) + (2.004), (I.05) + (2.005), (I.05) + (2.006), (I.05) + (2.007), (I.05) + (2.008), (I.05) + (2.009), (I.05) + (2.010), (I.05) + (2.011), (I.05) + (2.012), (I.05) + (2.013), (I.05) + (2.014), (I.05) + (2.015), (I.05) + (2.016), (I.05) + (2.017), (I.05) + (2.018), (I.05) + (2.019), (I.05) + (2.020), (I.05) + (2.021), (I.05) + (2.022), (I.05) + (2.023), (I.05) + (2.024), (I.05) + (2.025), (I.05) + (2.026), (I.05) + (2.027), (I.05) + (2.028), (I.05) + (2.029), (I.05) + (2.030), (I.05) + (2.031), (I.05) + (2.032), (I.05) + (2.033), (I.05) + (2.034), (I.05) + (2.035), (I.05) + (2.036), (I.05) + (2.037), (I.05) + (2.038), (I.05) + (2.039), (I.05) + (2.040), (I.05) + (2.041), (I.05) + (2.042), (I.05) + (2.043), (I.05) + (2.044), (I.05) + (2.045), (I.05) + (2.046), (I.05) + (2.047), (I.05) + (2.048), (I.05) + (2.049), (I.05) + (2.050), (I.05) + (2.051), (I.05) + (2.052), (I.05) + (2.053), (I.05) + (2.054), (I.05) + (2.055), (I.05) + (2.056), (I.05) + (3.001), (I.05) + (3.002), (I.05) + (3.003), (I.05) + (3.004), (I.05) + (3.005), (I.05) + (3.006), (I.05) + (3.007), (I.05) + (3.008), (I.05) + (3.009), (I.05) + (3.010), (I.05) + (3.011), (I.05) + (3.012), (I.05) + (3.013), (I.05) + (3.014), (I.05) + (3.015), (I.05) + (3.016), (I.05) + (3.017), (I.05) + (3.018), (I.05) + (3.019), (I.05) + (3.020), (I.05) + (3.021), (I.05) + (3.022), (I.05) + (3.023), (I.05) + (3.024), (I.05) + (3.025), (I.05) + (3.026), (I.05) + (3.027), (I.05) + (3.028), (I.05) + (3.029), (I.05) + (3.030), (I.05) + (4.001), (I.05) + (4.002), (I.05) + (4.003), (I.05) + (4.004), (I.05) + (4.005), (I.05) + (4.006), (I.05) + (4.007), (I.05) + (4.008), (I.05) + (4.009), (I.05) + (4.010), (I.05) + (4.011), (I.05) + (4.012), (I.05) + (4.013), (I.05) + (4.014), (I.05) + (4.015), (I.05) + (4.016), (I.05) + (4.017), (I.05) + (4.018), (I.05) + (4.019), (I.05) + (4.020), (I.05) + (4.021), (I.05) + (4.022), (I.05) + (4.023), (I.05) + (4.024), (I.05) + (4.025), (I.05) + (5.001), (I.05) + (5.002), (I.05) + (5.003), (I.05) + (5.004), (I.05) + (5.005), (I.05) + (5.006), (I.05) + (5.007), (I.05) + (5.008), (I.05) + (5.009), (I.05) + (5.010), (I.05) + (5.011), (I.05) + (5.012), (I.05) + (5.013), (I.05) + (5.014), (I.05) + (5.015), (I.05) + (5.016), (I.05) + (5.017), (I.05) + (5.018), (I.05) + (5.019), (I.05) + (5.020), (I.05) + (5.021), (I.05) + (5.022), (I.05) + (5.023), (I.05) + (6.001), (I.05) + (6.002), (I.05) + (6.003), (I.05) + (6.004), (I.05) + (7.001), (I.05) + (7.002), (I.05) + (7.003), (I.05) + (7.004), (I.05) + (7.005), (I.05) + (7.006), (I.05) + (8.001), (I.05) + (9.001), (I.05) + (9.002), (I.05) + (9.003), (I.05) + (9.004), (I.05) + (9.005), (I.05) + (9.006), (I.05) + (9.007), (I.05) + (9.008), (I.05) + (9.009), (I.05) + (10.001), (I.05) + (10.002), (I.05) + (10.003), (I.05) + (11.001), (I.05) + (11.002), (I.05) + (12.001), (I.05) + (12.002), (I.05) + (12.003), (I.05) + (12.004), (I.05) + (13.001), (I.05) + (13.002), (I.05) + (13.003), (I.05) + (13.004), (I.05) + (13.005), (I.05) + (13.006), (I.05) + (14.001), (I.05) + (14.002), (I.05) + (15.001), (I.05) + (15.002), (I.05) + (15.003), (I.05) + (15.004), (I.05) + (15.005), (I.05) + (15.006), (I.05) + (15.007), (I.05) + (15.008), (I.05) + (15.009), (I.05) + (15.010), (I.05) + (15.011), (I.05) + (15.012), (I.05) + (15.013), (I.05) + (15.014), (I.05) + (15.015), (I.05) + (15.016), (I.05) + (15.017), (I.05) + (15.018), (I.05) + (15.019), (I.05) + (15.020), (I.05) + (15.021), (I.05) + (15.022), (I.05) + (15.023), (I.05) + (15.024), (I.05) + (15.025), (I.05) + (15.026), (I.05) + (15.027), (I.05) + (15.028), (I.05) + (15.029), (I.05) + (15.030), (I.05) + (15.031), (I.05) + (15.032), (I.05) + (15.033), (I.05) + (15.034), (I.05) + (15.035), (I.05) + (15.036), (I.05) + (15.037), (I.05) + (15.038), (I.05) + (15.039), (I.05) + (15.040), (I.05) + (15.041),

(I.05) + (15.042), (I.05) + (15.043), (I.05) + (15.044), (I.05) + (15.045), (I.05) + (15.046), (I.05) + (15.047), (I.05) + (15.048), (I.05) + (15.049), (I.05) + (15.050), (I.05) + (15.051), (I.05) + (15.052), (I.05) + (15.053), (I.05) + (15.054), (I.05) + (15.055), (I.05) + (15.056), (I.05) + (15.057), (I.05) + (15.058), (I.05) + (15.059), (I.05) + (15.060), (I.05) + (15.061), (I.05) + (15.062). (I.05) + (15.063). (I.05) + (15.064). and (I.05) + (15.062065), (I.05) + 15.066), (I.05) + (15.067), (I.05) + (15.068), (I.05) + (15.069), (I.05) + (15.070), (I.05) + (15.071), (I.05) + (15.072), (I.05) + (15.073), (I.05) + (15.074), (I.05) + (15.075), (I.05) + (15.076), (1.05), (I.05) + (15.077), (I.05) + (15.078), (I.05) + (15.079), (I.05) + (15.080), (I.05) + (15.081), (I.05) + (15.082), (I.05) + (15.083), (I.05) + (15.084), (I.05) + (15.085), (I.05) + (15.086), (I.05) + (15.087), (I.05) + (15.088), (I.05) + (15.089), (I.05) + (15.090), (I.05) + (15.091), (I.05) + (15.092), (I.05) + (15.093), (I.05) + (15.094), (I.05) + (15.095), (I.05) + (15.096), (I.05) + (15.097), (I.05) + (15.098), (I.05) + (15.099), (I.05) + (15.100), (I.05) + (15.101), (I.05) + (15.102), (I.05) + (15.103), (I.05) + (15.104), (I.05) + (15.105), (I.05) + (15.106), (I.05) + (15.107), (I.05) + (15.108), (I.05) + (15.109).

Also preferred compound combinations are selected from the group (G6) consisting of the following mixtures: (I.06) + (1.001), (I.06) + (1.002), (I.06) + (1.003), (I.06) + (1.004), (I.06) + (1.005), (I.06) + (1.006), (I.06) + (1.007), (I.06) + (1.008), (I.06) + (1.009), (I.06) + (1.010), (I.06) + (1.011), (I.06) + (1.012), (I.06) + (1.013), (I.06) + (1.014), (I.06) + (1.015), (I.06) + (1.016), (I.06) + (1.017), (I.06) + (1.018), (I.06) + (1.019), (I.06) + (1.020), (I.06) + (1.021), (I.06) + (1.022), (I.06) + (1.023), (I.06) + (1.024), (I.06) + (1.025), (I.06) + (1.026), (I.06) + (1.027), (I.06) + (1.028), (I.06) + (1.029), (I.06) + (1.030), (I.06) + (1.031), (I.06) + (1.032), (I.06) + (1.033), (I.06) + (1.034), (I.06) + (1.035), (I.06) + (1.036), (I.06) + (1.037), (I.06) + (1.038), (I.06) + (1.039), (I.06) + (1.040), (I.06) + (1.041), (I.06) + (1.042), (I.06) + (1.043), (I.06) + (1.044), (I.06) + (1.045), (I.06) + (1.046), (I.06) + (1.047), (I.06) + (1.048), (I.06) + (1.049), (I.06) + (1.050), (I.06) + (1.051), (I.06) + (1.052), (I.06) + (1.053), (I.06) + (1.054), (I.06) + (1.055), (I.06) + (1.056), (I.06) + (1.057), (I.06) + (1.058), (I.06) + (1.059), (I.06) + (1.060), (I.06) + (1.061), (I.06) + (1.062), (I.06) + (1.063), (I.06) + (1.064), (I.06) + (1.065), (I.06) + (1.066), (I.06) + (1.067), (I.06) + (1.068), (I.06) + (1.069), (I.06) + (1.070), (I.06) + (1.071), (I.06) + (1.072), (I.06) + (1.073), (I.06) + (1.074), (I.06) + (1.075), (I.06) + (1.076), (I.06) + (1.077), (I.06) + (1.078), (I.06) + (1.079), (I.06) + (1.080), (I.06) + (1.081), (I.06) + (1.082), (I.06) + (1.083), (I.06) + (2.001), (I.06) + (2.002), (I.06) + (2.003), (I.06) + (2.004), (I.06) + (2.005), (I.06) + (2.006), (I.06) + (2.007), (I.06) + (2.008), (I.06) + (2.009), (I.06) + (2.010), (I.06) + (2.011), (I.06) + (2.012), (I.06) + (2.013), (I.06) + (2.014), (I.06) + (2.015), (I.06) + (2.016), (I.06) + (2.017), (I.06) + (2.018), (I.06) + (2.019), (I.06) + (2.020), (I.06) + (2.021), (I.06) + (2.022), (I.06) + (2.023), (I.06) + (2.024), (I.06) + (2.025), (I.06) + (2.026), (I.06) + (2.027), (I.06) + (2.028), (I.06) + (2.029), (I.06) + (2.030), (I.06) + (2.031), (I.06) + (2.032), (I.06) + (2.033), (I.06) + (2.034), (I.06) + (2.035), (I.06) + (2.036), (I.06) + (2.037), (I.06) + (2.038), (I.06) + (2.039), (I.06) + (2.040), (I.06) + (2.041), (I.06) + (2.042), (I.06) + (2.043), (I.06) + (2.044), (I.06) + (2.045), (I.06) + (2.046), (I.06) + (2.047), (I.06) + (2.048), (I.06) + (2.049), (I.06) + (2.050), (I.06) + (2.051), (I.06) + (2.052), (I.06) + (2.053), (I.06) + (2.054), (I.06) + (2.055), (I.06) + (2.056), (I.06) + (3.001), (I.06) + (3.002), (I.06) + (3.003), (I.06) + (3.004), (I.06) + (3.005), (I.06) + (3.006), (I.06) + (3.007), (I.06) + (3.008), (I.06) + (3.009), (I.06) + (3.010), (I.06) + (3.011), (I.06) + (3.012), (I.06) + (3.013), (I.06) + (3.014), (I.06) + (3.015), (I.06) + (3.016), (I.06) + (3.017), (I.06) + (3.018), (I.06) + (3.019), (I.06) + (3.020), (I.06) + (3.021), (I.06) + (3.022), (I.06) + (3.023), (I.06) + (3.024), (I.06) + (3.025), (I.06) + (3.026), (I.06) + (3.027), (I.06) + (3.028), (I.06) + (3.029), (I.06) + (3.030), (I.06) + (4.001), (I.06) + (4.002), (I.06) + (4.003), (I.06) + (4.004), (I.06) + (4.005), (I.06) + (4.006), (I.06) + (4.007), (I.06) + (4.008), (I.06) + (4.009), (I.06) + (4.010), (I.06) + (4.011), (I.06) + (4.012), (I.06) + (4.013), (I.06) + (4.014), (I.06) + (4.015), (I.06) + (4.016), (I.06) + (4.017), (I.06) + (4.018), (I.06) + (4.019), (I.06) + (4.020), (I.06) + (4.021), (I.06) + (4.022), (I.06) + (4.023), (I.06) + (4.024), (I.06) + (4.025), (I.06) + (5.001), (I.06) + (5.002), (I.06) + (5.003), (I.06) + (5.004), (I.06) + (5.005), (I.06) + (5.006), (I.06) + (5.007), (I.06) + (5.008), (I.06) + (5.009), (I.06) + (5.010), (I.06) + (5.011), (I.06) + (5.012), (I.06) + (5.013), (I.06) + (5.014), (I.06) + (5.015), (I.06) + (5.016), (I.06) + (5.017), (I.06) + (5.018), (I.06) + (5.019), (I.06) + (5.020), (I.06) + (5.021), (I.06) + (5.022), (I.06) + (5.023), (I.06) + (6.001), (I.06) + (6.002), (I.06) + (6.003), (I.06) + (6.004), (I.06) + (7.001), (I.06) + (7.002), (I.06) + (7.003), (I.06) + (7.004), (I.06) + (7.005), (I.06) + (7.006), (I.06) + (8.001), (I.06) + (9.001), (I.06) + (9.002), (I.06) + (9.003), (I.06) + (9.004), (I.06) + (9.005), (I.06) + (9.006), (I.06) + (9.007), (I.06) + (9.008), (I.06) + (9.009), (I.06) + (10.001), (I.06) + (10.002), (I.06) + (10.003), (I.06) + (11.001), (I.06) + (11.002), (I.06) + (12.001), (I.06) + (12.002), (I.06) + (12.003), (I.06) + (12.004), (I.06) + (13.001), (I.06) + (13.002), (I.06) + (13.003), (I.06) + (13.004), (I.06) + (13.005), (I.06) + (13.006), (I.06) + (14.001), (I.06) + (14.002), (I.06) + (15.001), (I.06) + (15.002), (I.06) + (15.003), (I.06) + (15.004), (I.06) + (15.005), (I.06) + (15.006), (I.06) + (15.007), (I.06) + (15.008), (I.06) + (15.009), (I.06) + (15.010), (I.06) + (15.011), (I.06) + (15.012), (I.06) + (15.013), (I.06) + (15.014), (I.06) + (15.015), (I.06) + (15.016), (I.06) + (15.017), (I.06) + (15.018), (I.06) + (15.019), (I.06) + (15.020), (I.06) + (15.021), (I.06) + (15.022), (I.06) + (15.023), (I.06) + (15.024), (I.06) + (15.025), (I.06) + (15.026), (I.06) + (15.027), (I.06) + (15.028), (I.06) + (15.029), (I.06) + (15.030), (I.06) + (15.031), (I.06) + (15.032), (I.06) + (15.033), (I.06) + (15.034), (I.06) + (15.035), (I.06) + (15.036), (I.06) + (15.037), (I.06) + (15.038), (I.06) + (15.039), (I.06) + (15.040), (I.06) + (15.041), (I.06) + (15.042), (I.06) + (15.043), (I.06) + (15.044), (I.06) + (15.045), (I.06) + (15.046), (I.06) + (15.047), (I.06) + (15.048), (I.06) + (15.049), (I.06) + (15.050), (I.06) + (15.051), (I.06) + (15.052), (I.06) + (15.053), (I.06) + (15.054), (I.06) + (15.055), (I.06) + (15.056), (I.06) + (15.057), (I.06) + (15.058), (I.06) + (15.059), (I.06) + (15.060), (I.06) + (15.061), (I.06) + (15.062). (I.06) + (15.063). (I.06) + (15.064). and (I.06) + (15.062065), (I.06) + 15.066), (I.06) + (15.067), (I.06) + (15.068), (I.06) + (15.069), (I.06) + (15.070), (I.06) + (15.071), (I.06) + (15.072), (I.06) + (15.073), (I.06) + (15.074), (I.06) + (15.075), (I.06) + (15.076), (1.06), (I.06) + (15.077), (I.06) + (15.078), (I.06) + (15.079), (I.06) + (15.080), (I.06) + (15.081), (I.06) + (15.082), (I.06) + (15.083), (I.06)

+ (15.084), (I.06) + (15.085), (I.06) + (15.086), (I.06) + (15.087), (I.06) + (15.088), (I.06) + (15.089), (I.06) + (15.090), (I.06) + (15.091), (I.06) + (15.092), (I.06) + (15.093), (I.06) + (15.094), (I.06) + (15.095), (I.06) + (15.096), (I.06) + (15.097), (I.06) + (15.098), (I.06) + (15.099), (I.06) + (15.100), (I.06) + (15.101), (I.06) + (15.102), (I.06) + (15.103), (I.06) + (15.104), (I.06) + (15.105), (I.06) + (15.106), (I.06) + (15.107), (I.06) + (15.108), (I.06) + (15.109).

Even more preferred compound combinations are selected from the group (G1-A) consisting of the following mixtures:
(I.01) + (1.012), (I.01) + (1.018), (I.01) + (1.020), (I.01) + (1.021), (I.01) + (1.083), (I.01) + (2.002), (I.01) + (2.005), (I.01) + (2.017), (I.01) + (2.027), (I.01) + (2.038), (I.01) + (3.020), (I.01) + (3.025), (I.01) + (3.030), (I.01) + (4.005), (I.01) + (5.004), (I.01) + (5.013), (I.01) + (5.018), (I.01) + (12.003), (I.01) + (12.004), (I.01) + (13.001), (I.01) + (13.004), (I.01) + (15.008).

Even more preferred compound combinations are also selected from the group (G2-A) consisting of the following mixtures:
(I.02) + (1.012), (I.02) + (1.018), (I.02) + (1.020), (I.02) + (1.021), (I.02) + (1.083), (I.02) + (2.002), (I.02) + (2.005), (I.02) + (2.017), (I.02) + (2.027), (I.02) + (2.038), (I.02) + (3.020), (I.02) + (3.025), (I.02) + (3.030), (I.02) + (4.005), (I.02) + (5.004), (I.02) + (5.013), (I.02) + (5.018), (I.02) + (12.003), (I.02) + (12.004), (I.02) + (13.001), (I.02) + (13.004), (I.02) + (15.008).

Even more preferred compound combinations are also selected from the group (G3-A) consisting of the following mixtures:
(I.03) + (1.012), (I.03) + (1.018), (I.03) + (1.020), (I.03) + (1.021), (I.03) + (1.083), (I.03) + (2.002), (I.03) + (2.005), (I.03) + (2.017), (I.03) + (2.027), (I.03) + (2.038), (I.03) + (3.020), (I.03) + (3.025), (I.03) + (3.030), (I.03) + (4.005), (I.03) + (5.004), (I.03) + (5.013), (I.03) + (5.018), (I.03) + (12.003), (I.03) + (12.004), (I.03) + (13.001), (I.03) + (13.004), (I.03) + (15.008).

Even more preferred compound combinations are also selected from the group (G4-A) consisting of the following mixtures:
(I.04) + (1.012), (I.04) + (1.018), (I.04) + (1.020), (I.04) + (1.021), (I.04) + (1.083), (I.04) + (2.002), (I.04) + (2.005), (I.04) + (2.017), (I.04) + (2.027), (I.04) + (2.038), (I.04) + (3.020), (I.04) + (3.025), (I.04) + (3.030), (I.04) + (4.005), (I.04) + (5.004), (I.04) + (5.013), (I.04) + (5.018), (I.04) + (12.003), (I.04) + (12.004), (I.04) + (13.001), (I.04) + (13.004), (I.04) + (15.008).

Even more preferred compound combinations are also selected from the group (G5-A) consisting of the following mixtures:
(I.05) + (1.012), (I.05) + (1.018), (I.05) + (1.020), (I.05) + (1.021), (I.05) + (1.083), (I.05) + (2.002), (I.05) + (2.005), (I.05) + (2.017), (I.05) + (2.027), (I.05) + (2.038), (I.05) + (3.020), (I.05) + (3.025), (I.05) + (3.030), (I.05) + (4.005), (I.05) + (5.004), (I.05) + (5.013), (I.05) + (5.018), (I.05) + (12.003), (I.05) + (12.004), (I.05) + (13.001), (I.05) + (13.004), (I.05) + (15.008).

Even more preferred compound combinations are also selected from the group (G6-A) consisting of the following mixtures:
(I.06) + (1.012), (I.06) + (1.018), (I.06) + (1.020), (I.06) + (1.021), (I.06) + (1.083), (I.06) + (2.002), (I.06) + (2.005), (I.06) + (2.017), (I.06) + (2.027), (I.06) + (2.038), (I.06) + (3.020), (I.06) + (3.025), (I.06) + (3.030), (I.06) + (4.005), (I.06) + (5.004), (I.06) + (5.013), (I.06) + (5.018), (I.06) + (12.003), (I.06) + (12.004), (I.06) + (13.001), (I.06) + (13.004), (I.06) + (15.008).

In the combinations according to the invention the compounds (A), i.e. the compounds of formula (I), and (B), i.e. further active compounds selected from groups (1) to (15), can be present in a broad range of effective weight ratio of A:B, for example in a range of 100:1 to 1:100, preferably in a weight ratio of 50:1 to 1:50, most preferably in a weight ratio of 20:1 to 1:20. Further ratios of A:B which can be used according to the present invention with increasing preference in the order given are: 95:1 to 1:95, 90:1 to 1:90, 85:1 to 1:85, 80:1 to 1:80, 75:1 to 1:75, 70:1 to 1:70, 65:1 to 1:65, 60:1 to 1:60, 55:1 to 1:55, 45:1 to 1:45, 40:1 to 1:40, 35:1 to 1:35, 30:1 to 1:30, 25:1 to 1:25, 15:1 to 1:15, 10:1 to 1:10, 5:1 to 1:5, 4:1 to 1:4, 3:1 to 1:3, 2:1 to 1:2.

It is further preferred if the active compound combination according to the invention comprises at least two further active compounds. Even more preferred is if it comprises at least two further active compounds which are each selected from different groups (1) to (15) as described on page 6. Still further preferred is an active compound combination which comprises at least at least two further active compounds which are selected from the group consisting of (1.012), (1.018), (1.020), (1.021), (1.083), (2.002), (2.005), (2.017), (2.027), (2.038), (3.012), (3.020), (3.025, (3.030), (4.005), (5.004), (5.012), (5.013), (5.018), (12.003), (12.004), (13.001), (13.004), (15.008), and (15.012). Most preferred is an active compound combination which comprises at least at least two further active compounds which are each selected from different groups (1) to (15) as described on page 6 and which are selected from the group consisting of (1.012), (1.018), (1.020), (1.021), (1.083), (2.002), (2.005), (2.017), (2.027), (2.038), (3.012), (3.020), (3.025, (3.030), (4.005), (5.004), (5.012), (5.013), (5.018), (12.003), (12.004), (13.001), (13.004), (15.008), and (15.012).

Where a compound (A) or a compound (B) can be present in isomeric forms and/or tautomeric forms, such a

compound is understood hereinabove and hereinbelow also to include, where applicable, corresponding isomeric and/or tautomeric forms or mixtures thereof, even when these are not specifically mentioned in each case.

The compound combination of the invention or the compounds of formula (I) may also be combined with one or more biological control agents.

Examples of biological control agents which may be combined with the compound combination of the invention are:

(A) Antibacterial agents selected from the group of:

(A1) bacteria, such as (A1.1) *Bacillus subtilis,* in particular strain QST713/AQ713 (available as SERENADE OPTI or SERENADE ASO from Bayer CropScience LP, US, having NRRL Accession No. B21661and described in U.S. Patent No. 6,060,051); (A1.2) *Bacillus amyloliquefaciens,* in particular strain D747 (available as Double Nickel™ from Certis, US, having accession number FERM BP-8234 and disclosed in US Patent No. 7,094,592); (A1.3) *Bacillus pumilus,* in particular strain BU F-33 (having NRRL Accession No. 50185); (A1.4) *Bacillus subtilis var. amyloliquefaciens* strain FZB24 (available as Taegro® from Novozymes, US); (A1.5) a *Paenibacillus* sp. strain having Accession No. NRRL B-50972 or Accession No. NRRL B-67129 and described in International Patent Publication No. WO 2016/154297; and

(A2) fungi, such as (A2.1) *Aureobasidium pullulans,* in particular blastospores of strain DSM14940; (A2.2) *Aureobasidium pullulans* blastospores of strain DSM 14941; (A2.3) *Aureobasidium pullulans,* in particular mixtures of blastospores of strains DSM14940 and DSM14941;

(B) Fungicides selected from the group of:

(B1) bacteria, for example (B1.1) *Bacillus subtilis,* in particular strain QST713/AQ713 (available as SERENADE OPTI or SERENADE ASO from Bayer CropScience LP, US, having NRRL Accession No. B21661and described in U.S. Patent No. 6,060,051); (B1.2) *Bacillus pumilus,* in particular strain QST2808 (available as SONATA® from Bayer CropScience LP, US, having Accession No. NRRL B-30087 and described in U.S. Patent No. 6,245,551); (B1.3) *Bacillus pumilus,* in particular strain GB34 (available as Yield Shield® from Bayer AG, DE); (B1.4) *Bacillus pumilus,* in particular strain BU F-33 (having NRRL Accession No. 50185); (B1.5) *Bacillus amyloliquefaciens,* in particular strain D747 (available as Double Nickel™ from Certis, US, having accession number FERM BP-8234 and disclosed in US Patent No. 7,094,592); (B1.6) *Bacillus subtilis* Y1336 (available as BIOBAC® WP from Bion-Tech, Taiwan, registered as a biological fungicide in Taiwan under Registration Nos. 4764, 5454, 5096 and 5277); (B1.7) *Bacillus amyloliquefaciens* strain MBI 600 (available as SUBTILEX from BASF SE); (B1.8) *Bacillus subtilis* strain GB03 (available as Kodiak® from Bayer AG, DE); (B1.9) *Bacillus subtilis var. amyloliquefaciens* strain FZB24 (available from Novozymes Biologicals Inc., Salem, Virginia or Syngenta Crop Protection, LLC, Greensboro, North Carolina as the fungicide TAEGRO® or TAEGRO® ECO (EPA Registration No. 70127-5); (B1.10) *Bacillus mycoides,* isolate J (available as BmJ TGAI or WG from Certis USA); (B1.11) *Bacillus licheniformis,* in particular strain SB3086 (available as EcoGuard TM Biofungicide and Green Releaf from Novozymes); (B1.12) a *Paenibacillus* sp. strain having Accession No. NRRL B-50972 or Accession No. NRRL B-67129 and described in International Patent Publication No. WO 2016/154297.

In some embodiments, the biological control agent is a *Bacillus subtilis* or *Bacillus amyloliquefaciens* strain that produces a fengycin or plipastatin-type compound, an iturin-type compound, and/or a surfactin-type compound. For background, see the following review article: Ongena, M., et al., "Bacillus Lipopeptides: Versatile Weapons for Plant Disease Biocontrol," Trends in Microbiology, Vol 16, No. 3, March 2008, pp. 115-125. *Bacillus* strains capable of producing lipopeptides include *Bacillus subtilis* QST713 (available as SERENADE OPTI or SERENADE ASO from Bayer CropScience LP, US, having NRRL Accession No. B21661and described in U.S. Patent No. 6,060,051), *Bacillus amyloliquefaciens* strain D747 (available as Double Nickel™ from Certis, US, having accession number FERM BP-8234 and disclosed in US Patent No. 7,094,592); *Bacillus subtilis* MBI600 (available as SUBTILEX® from Becker Underwood, US EPA Reg. No. 71840-8**);** *Bacillus subtilis* Y1336 (available as BIOBAC® WP from Bion-Tech, Taiwan, registered as a biological fungicide in Taiwan under Registration Nos. 4764, 5454, 5096 and 5277); *Bacillus amyloliquefaciens,* in particular strain FZB42 (available as RHIZOVITAL® from ABiTEP, DE); and *Bacillus subtilis* var. *amyloliquefaciens* FZB24 (available from Novozymes Biologicals Inc., Salem, Virginia or Syngenta Crop Protection, LLC, Greensboro, North Carolina as the fungicide TAEGRO® or TAEGRO® ECO (EPA Registration

No. 70127-5); and

(B2) fungi, for example: (B2.1) *Coniothyrium minitans,* in particular strain CON/M/91-8 (Accession No. DSM-9660; e.g. Contans® from Bayer); (B2.2) *Metschnikowia fructicola,* in particular strain NRRL Y-30752 (e.g. Shemer®); (B2.3) *Microsphaeropsis ochracea* (e.g. Microx® from Prophyta); (B2.5) *Trichoderma spp.,* including *Trichoderma atroviride,* strain SC1 described in International Application No. PCT/IT2008/000196); (B2.6) *Trichoderma harzianum rifai* strain KRL-AG2 (also known as strain T-22, /ATCC 208479, e.g. PLANTSHIELD T-22G, Rootshield®, and TurfShield from BioWorks, US); (B2.14) *Gliocladium roseum,* strain 321U from W.F. Stoneman Company LLC; (B2.35) *Talaromyces flavus,* strain V117b; (B2.36) *Trichoderma asperellum,* strain ICC 012 from Isagro; (B2.37) *Trichoderma asperellum,* strain SKT-1 (e.g. ECO-HOPE® from Kumiai Chemical Industry); (B2.38) *Trichoderma atroviride,* strain CNCM 1-1237 (e.g. Esquive® WP from Agrauxine, FR); (B2.39) *Trichoderma atroviride,* strain no. V08/002387; (B2.40) *Trichoderma atroviride,* strain NMI no. V08/002388; (B2.41) *Trichoderma atroviride,* strain NMI no. V08/002389; (B2.42) *Trichoderma atroviride,* strain NMI no. V08/002390; (B2.43) *Trichoderma atroviride,* strain LC52 (e.g. Tenet by Agrimm Technologies Limited); (B2.44) *Trichoderma atroviride,* strain ATCC 20476 (IMI 206040); (B2.45) *Trichoderma atroviride,* strain T11 (IMI352941/ CECT20498); (B2.46) *Trichoderma harmatum*; (B2.47) *Trichoderma harzianum;* (B2.48) *Trichoderma harzianum rifai T39* (e.g. Trichodex® from Makhteshim, US); (B2.49) *Trichoderma harzianum,* in particular, strain KD (e.g. Trichoplus from Biological Control Products, SA (acquired by Becker Underwood)); (B2.50) *Trichoderma harzianum,* strain ITEM 908 (e.g. Trianum-P from Koppert); (B2.51) *Trichoderma harzianum,* strain TH35 (e.g. Root-Pro by Mycontrol); (B2.52) *Trichoderma virens* (also known as *Gliocladium virens),* in particular strain GL-21 (e.g. SoilGard 12G by Certis, US); (B2.53) *Trichoderma viride,* strain TV1(e.g. Trianum-P by Koppert); (B2.54) *Ampelomyces quisqualis,* in particular strain AQ 10 (e.g. AQ 10® by IntrachemBio Italia); (B2.56) *Aureobasidium pullulans,* in particular blastospores of strain DSM14940; (B2.57) *Aureobasidium pullulans,* in particular blastospores of strain DSM 14941; (B2.58) *Aureobasidium pullulans,* in particular mixtures of blastospores of strains DSM14940 and DSM 14941 (e.g. Botector® by bio-ferm, CH); (B2.64) *Cladosporium cladosporioides,* strain H39 (by Stichting Dienst Landbouwkundig Onderzoek); (B2.69) *Gliocladium catenulatum* (Synonym: *Clonostachys rosea f. catenulate*) strain J1446 (e.g. Prestop ® by AgBio Inc. and also e.g. Primastop® by Kemira Agro Oy); (B2.70) *Lecanicillium lecanii* (formerly known as *Verticillium lecanii*) *conidia* of strain KV01 (e.g. Vertalec® by Koppert/Arysta); (B2.71) *Penicillium vermiculatum;* (B2.72) *Pichia anomala,* strain WRL-076 (NRRL Y-30842); (B2.75) *Trichoderma atroviride,* strain SKT-1 (FERM P-16510); (B2.76) *Trichoderma atroviride,* strain SKT-2 (FERM P-16511); (B2.77) *Trichoderma atroviride,* strain SKT-3 (FERM P-17021); (B2.78) *Trichoderma gamsii* (formerly *T. viride*), strain ICC080 (IMI CC 392151 CABI, e.g. BioDerma by AGROBIOSOL DE MEXICO, S.A. DE C.V.); (B2.79) *Trichoderma harzianum,* strain DB 103 (e.g., T-Gro 7456 by Dagutat Biolab); (B2.80) *Trichoderma polysporum,* strain IMI 206039 (e.g. Binab TF WP by BINAB Bio-Innovation AB, Sweden); (B2.81) *Trichoderma stromaticum* (e.g. Tricovab by Ceplac, Brazil); (B2.83) *Ulocladium oudemansii,* in particular strain HRU3 (e.g. Botry-Zen® by Botry-Zen Ltd, NZ); (B2.84) *Verticillium albo-atrum* (formerly *V. dahliae*), strain WCS850 (CBS 276.92; e.g. Dutch Trig by Tree Care Innovations); (B2.86) *Verticillium chlamydosporium*; (B2.87) mixtures of *Trichoderma asperellum* strain ICC 012 and *Trichoderma gamsii* strain ICC 080 (product known as e.g. BIO-TAM™ from Bayer CropScience LP, US).

Further examples of biological control agents which may be combined with the compound combination of the invention or the compounds of formula (I) are:

bacteria selected from the group consisting of *Bacillus cereus,* in particular *B. cereus* strain CNCM I-1562 and *Bacillus firmus,* strain I-1582 (Accession number CNCM 1-1582), *Bacillus subtilis strain* OST 30002 (Accession No. NRRL B-50421), *Bacillus thuringiensis,* in particular *B. thuringiensis* subspecies *israelensis* (serotype H-14), strain AM65-52 (Accession No. ATCC 1276), *B. thuringiensis subsp. aizawai,* in particular strain ABTS-1857 (SD-1372), *B. thuringiensis subsp. kurstaki* strain HD-1, *B. thuringiensis subsp. tenebrionis* strain NB 176 (SD-5428), *Pasteuria penetrans, Pasteuria spp.* (Rotylenchulus reniformis nematode)-PR3 (Accession Number ATCC SD-5834), *Streptomyces microflavus* strain AQ6121 (= QRD 31.013, NRRL B-50550), and *Streptomyces galbus* strain AQ 6047 (Acession Number NRRL 30232);

fungi and yeasts selected from the group consisting of *Beauveria bassiana,* in particular strain ATCC 74040, *Lecanicillium spp.,* in particular strain HRO LEC 12, *Metarhizium anisopliae,* in particular strain F52 (DSM3884 or ATCC 90448), *Paecilomyces fumosoroseus* (now: *Isaria fumosorosea),* in particular strain IFPC 200613, or strain Apopka 97 (Accesion No. ATCC 20874), and *Paecilomyces lilacinus,* in particular *P. lilacinus* strain 251 (AGAL 89/030550);

viruses selected from the group consisting of *Adoxophyes orana* (summer fruit tortrix) granulosis virus (GV), *Cydia pomonella* (codling moth) granulosis virus (GV), *Helicoverpa armigera* (cotton bollworm) nuclear polyhedrosis virus

(NPV), *Spodoptera exigua* (beet armyworm) mNPV, *Spodoptera frugiperda* (fall armyworm) mNPV, and *Spodoptera littoralis* (African cotton leafworm) NPV.

bacteria and fungi which can be added as 'inoculant' to plants or plant parts or plant organs and which, by virtue of their particular properties, promote plant growth and plant health. Examples are: *Agrobacterium spp., Azorhizobium caulinodans, Azospirillum spp., Azotobacter spp., Bradyrhizobium spp., Burkholderia spp.,* in particular *Burkholderia cepacia* (formerly known as *Pseudomonas cepacia*), *Gigaspora spp.,* or *Gigaspora monosporum, Glomus spp., Laccaria spp., Lactobacillus buchneri, Paraglomus spp., Pisolithus tinctorus, Pseudomonas spp., Rhizobium spp.,* in particular *Rhizobium trifolii, Rhizopogon spp., Scleroderma spp., Suillus spp.,* and *Streptomyces spp*.

plant extracts and products formed by microorganisms including proteins and secondary metabolites which can be used as biological control agents, such as *Allium sativum, Artemisia absinthium,* azadirachtin, Biokeeper WP, *Cassia nigricans, Celastrus angulatus, Chenopodium anthelminticum,* chitin, Armour-Zen, *Dryopteris filix-mas, Equisetum arvense,* Fortune Aza, Fungastop, Heads Up *(Chenopodium quinoa* saponin extract), *Pyrethrum*/*Pyrethrins, Quassia amara, Quercus, Quillaja,* Regalia, "Requiem ™ Insecticide", rotenone, *ryania*/ryanodine, *Symphytum officinale, Tanacetum vulgare,* thymol, Triact 70, TriCon, *Tropaeulum majus, Urtica dioica,* Veratrin, *Viscum album, Brassicaceae* extract, in particular oilseed rape powder or mustard powder.

Examples of insecticides, acaricides and nematicides, respectively, which could be mixed with the compound combination of the invention or the compounds of formula (I) are:

(1) Acetylcholinesterase (AChE) inhibitors, such as, for example, carbamates, for example alanycarb, aldicarb, bendiocarb, benfuracarb, butocarboxim, butoxycarboxim, carbaryl, carbofuran, carbosulfan, ethiofencarb, fenobucarb, formetanate, furathiocarb, isoprocarb, methiocarb, methomyl, metolcarb, oxamyl, pirimicarb, propoxur, thiodicarb, thiofanox, triazamate, trimethacarb, XMC and xylylcarb; or organophosphates, for example acephate, azamethiphos, azinphos-ethyl, azinphos-methyl, cadusafos, chlorethoxyfos, chlorfenvinphos, chlormephos, chlorpyrifos-methyl, coumaphos, cyanophos, demeton-S-methyl, diazinon, dichlorvos/DDVP, dicrotophos, dimethoate, dimethylvinphos, disulfoton, EPN, ethion, ethoprophos, famphur, fenamiphos, fenitrothion, fenthion, fosthiazate, heptenophos, imicyafos, isofenphos, isopropyl O-(methoxyaminothiophosphoryl) salicylate, isoxathion, malathion, mecarbam, methamidophos, methidathion, mevinphos, monocrotophos, naled, omethoate, oxydemeton-methyl, parathion-methyl, phenthoate, phorate, phosalone, phosmet, phosphamidon, phoxim, pirimiphos-methyl, profenofos, propetamphos, prothiofos, pyraclofos, pyridaphenthion, quinalphos, sulfotep, tebupirimfos, temephos, terbufos, tetrachlorvinphos, thiometon, triazophos, triclorfon and vamidothion.

(2) GABA-gated chloride channel blockers, such as, for example, cyclodiene-organochlorines, for example chlordane and endosulfan or phenylpyrazoles (fiproles), for example ethiprole and fipronil.

(3) Sodium channel modulators, such as, for example, pyrethroids, e.g. acrinathrin, allethrin, d-cis-trans allethrin, d-trans allethrin, bifenthrin, bioallethrin, bioallethrin s-cyclopentenyl isomer, bioresmethrin, cycloprothrin, cyfluthrin, beta-cyfluthrin, cyhalothrin, lambda-cyhalothrin, gamma-cyhalothrin, cypermethrin, alpha-cypermethrin, beta-cypermethrin, theta-cypermethrin, zeta-cypermethrin, cyphenothrin [(1R)-trans-isomer], deltamethrin, empenthrin [(EZ)-(1R)-isomer], esfenvalerate, etofenprox, fenpropathrin, fenvalerate, flucythrinate, flumethrin, tau-fluvalinate, halfenprox, imiprothrin, kadethrin, momfluorothrin, permethrin, phenothrin [(1R)-trans-isomer], prallethrin, pyrethrins (pyrethrum), resmethrin, silafluofen, tefluthrin, tetramethrin, tetramethrin [(1R)- isomer)], tralomethrin and transfluthrin or DDT or methoxychlor.

(4) Nicotinic acetylcholine receptor (nAChR) competitive modulators, such as, for example, neonicotinoids, e.g. acetamiprid, clothianidin, dinotefuran, imidacloprid, nitenpyram, thiacloprid and thiamethoxam or nicotine or sulfoxaflor or flupyradifurone.

(5) Nicotinic acetylcholine receptor (nAChR) allosteric modulators, such as, for example, spinosyns, e.g. spinetoram and spinosad.

(6) Glutamate-gated chloride channel (GluCl) allosteric modulators, such as, for example, avermectins/milbemycins, for example abamectin, emamectin benzoate, lepimectin and milbemectin.

(7) Juvenile hormone mimics, such as, for example, juvenile hormone analogues, e.g. hydroprene, kinoprene and methoprene or fenoxycarb or pyriproxyfen.

(8) Miscellaneous non-specific (multi-site) inhibitors, such as, for example, alkyl halides, e.g. methyl bromide and other alkyl halides; or chloropicrine or sulphuryl fluoride or borax or tartar emetic or methyl isocyanate generators, e.g. diazomet and metam.

(9) Modulators of Chordotonal Organs, such as, for example pymetrozine or flonicamid.

(10) Mite growth inhibitors, such as, for example clofentezine, hexythiazox and diflovidazin or etoxazole.

(11) Microbial disruptors of the insect gut membrane, such as, for example *Bacillus thuringiensis* subspecies *israelensis, Bacillus sphaericus, Bacillus thuringiensis* subspecies *aizawai, Bacillus thuringiensis* subspecies *kurstaki, Bacillus thuringiensis* subspecies *tenebrionis,* and *B.t.* plant proteins: Cry1Ab, CrylAc, Cry1Fa, Cry1A.105, Cry2Ab, Vip3A, mCry3A, Cry3Ab, Cry3Bb, Cry34Ab1/35Ab1.

(12) Inhibitors of mitochondrial ATP synthase, such as, ATP disruptors such as, for example, diafenthiuron or organotin compounds, for example azocyclotin, cyhexatin and fenbutatin oxide or propargite or tetradifon.

(13) Uncouplers of oxidative phosphorylation via disruption of the proton gradient, such as, for example, chlorfenapyr, DNOC and sulfluramid.

(14) Nicotinic acetylcholine receptor channel blockers, such as, for example, bensultap, cartap hydrochloride, thiocylam, and thiosultap-sodium.

(15) Inhibitors of chitin biosynthesis, type 0, such as, for example, bistrifluron, chlorfluazuron, diflubenzuron, flucycloxuron, flufenoxuron, hexaflumuron, lufenuron, novaluron, noviflumuron, teflubenzuron and triflumuron.

(16) Inhibitors of chitin biosynthesis, type 1, for example buprofezin.

(17) Moulting disruptor (in particular for Diptera, i.e. dipterans), such as, for example, cyromazine.

(18) Ecdysone receptor agonists, such as, for example, chromafenozide, halofenozide, methoxyfenozide and tebufenozide.

(19) Octopamine receptor agonists, such as, for example, amitraz.

(20) Mitochondrial complex III electron transport inhibitors, such as, for example, hydramethylnone or acequinocyl or fluacrypyrim.

(21) Mitochondrial complex I electron transport inhibitors, such as, for example from the group of the METI acaricides, e.g. fenazaquin, fenpyroximate, pyrimidifen, pyridaben, tebufenpyrad and tolfenpyrad or rotenone (Derris).

(22) Voltage-dependent sodium channel blockers, such as, for example indoxacarb or metaflumizone.

(23) Inhibitors of acetyl CoA carboxylase, such as, for example, tetronic and tetramic acid derivatives, e.g. spirodiclofen, spiromesifen and spirotetramat.

(24) Mitochondrial complex IV electron transport inhibitors, such as, for example, phosphines, e.g. aluminium phosphide, calcium phosphide, phosphine and zinc phosphide or cyanides, e.g. calcium cyanide, potassium cyanide and sodium cyanide.

(25) Mitochondrial complex II electron transport inhibitors, such as, for example, *beta*-ketonitrile derivatives, e.g. cyenopyrafen and cyflumetofen and carboxanilides, such as, for example, pyflubumide.

(28) Ryanodine receptor modulators, such as, for example, diamides, e.g. chlorantraniliprole, cyantraniliprole and flubendiamide,

further active compounds such as, for example, Afidopyropen, Afoxolaner, Azadirachtin, Benclothiaz, Benzoximate, Bifenazate, Broflanilide, Bromopropylate, Chinomethionat, Chloroprallethrin, Cryolite, Cyclaniliprole, Cycloxaprid, Cyhalodiamide, Dicloromezotiaz, Dicofol, epsilon-Metofluthrin, epsilon-Momfluthrin, Flometoquin, Fluazaindolizine, Fluensulfone, Flufenerim, Flufenoxystrobin, Flufiprole, Fluhexafon, Fluopyram, Fluralaner, Fluxametamide, Fufenozide, Guadipyr, Heptafluthrin, Imidaclothiz, Iprodione, kappa-Bifenthrin, kappa-Tefluthrin, Lotilaner, Meperfluthrin, Paichongding, Pyridalyl, Pyrifluquinazon, Pyriminostrobin, Spirobudiclofen, Tetramethylfluthrin, Tetraniliprole, Tetrachlorantraniliprole, Tigolaner, Tioxazafen, Thiofluoximate, Triflumezopyrim and iodomethane; furthermore preparations based on *Bacillus firmus* (1-1582, BioNeem, Votivo), and also the following compounds: 1-{2-fluoro-4-methyl-5-[(2,2,2-trifluoroethyl)sulphinyl]phenyl}-3-(trifluoromethyl)-1H-1,2,4-triazole-5-amine (known from WO2006/043635) (CAS 885026-50-6), {1'-[(2E)-3-(4-chlorophenyl)prop-2-en-1-yl]-5-fluorospiro[indol-3,4'-piperidin]-1(2H)-yl}(2-chloropyridin-4-yl)methanone (known from WO2003/106457) (CAS 637360-23-7), 2-chloro-N-[2-{1-[(2E)-3-(4-chlorophenyl)prop-2-en-1-yl]piperidin-4-yl}-4-(trifluoromethyl)phenyl]isonicotinamide (known from WO2006/003494) (CAS 872999-66-1), 3-(4-chloro-2,6-dimethylphenyl)-4-hydroxy-8-methoxy-1,8-diazaspiro[4.5]dec-3-en-2-one (known from WO 2010052161) (CAS 1225292-17-0), 3-(4-chloro-2,6-dimethylphenyl)-8-methoxy-2-oxo-1,8-diazaspiro[4.5]dec-3-en-4-yl ethyl carbonate (known from EP2647626) (CAS 1440516-42-6), 4-(but-2-yn-1-yloxy)-6-(3,5-dimethylpiperidin-1-yl)-5-fluoropyrimidine (known from WO2004/099160) (CAS 792914-58-0), PF1364 (known from JP2010/018586) (CAS 1204776-60-2), N-[(2E)-1-[(6-chloropyridin-3-yl)methyl]pyridin-2(1H)-ylidene]-2,2,2-trifluoroacetamide (known from WO2012/029672) (CAS 1363400-41-2), (3*E*)-3-[1-[(6-chloro-3-pyridyl)methyl]-2-pyridylidene]-1,1,1-trifluoro-propan-2-one (known from WO2013/144213) (CAS 1461743-15-6), , *N*-[3-(benzylcarbamoyl)-4-chlorophenyl]-1-methyl-3-(pentafluoroethyl)-4-(trifluoromethyl)-1*H*-pyrazole-5-carboxamide (known from WO2010/051926) (CAS 1226889-14-0), 5-bromo-4-chloro-*N*-[4-chloro-2-methyl-6-(methylcarbamoyl)phenyl]-2-(3-chloro-2-pyridyl)pyrazole-3-carboxamide (known from CN103232431) (CAS 1449220-44-3), 4-[5-(3,5-dichlorophenyl)-4,5-dihydro-5-(trifluoromethyl)-3-isoxazolyl]-2-methyl-*N*-(*cis*-1-oxido-3-thietanyl)-benzamide, 4-[5-(3,5-dichlorophenyl)-4,5-dihydro-5-(trifluoromethyl)-3-isoxazolyl]-2-methyl-*N*-(*trans*-1-oxido-3-thietanyl)-benzamide and 4-[(5*S*)-5-(3,5-dichlorophenyl)-4,5-dihydro-5-(trifluoromethyl)-3-isoxazolyl]-2-methyl-*N*-(*cis*-1-oxido-3-thietanyl)benzamide (known from WO 2013/050317 A1) (CAS 1332628-83-7), *N*-[3-chloro-1-(3-pyridinyl)-1*H*-pyrazol-4-yl]-*N*-ethyl-3-[(3,3,3-trifluoropropyl)sulfinyl]-propanamide, (+)-*N*-[3-chloro-1-(3-pyridinyl)-1*H*-pyrazol-4-yl]-*N*-ethyl-3-[(3,3,3-trifluoropropyl)sulfinyl]-propanamide and (-)-*N*-[3-chloro-1-(3-pyridinyl)-1*H*-pyrazol-4-yl]-*N*-ethyl-3-[(3,3,3-trifluoropropyl)sulfinyl]-propanamide (known from WO 2013/162715 A2, WO 2013/162716 A2, US 2014/0213448 A1) (CAS 1477923-37-7), 5-[[(2*E*)-3-chloro-2-propen-1-yl]amino]-1-[2,6-dichloro-4-(trifluoromethyl)phenyl]-4-[(trifluoromethyl)sulfinyl]-1*H*-pyrazole-3-carbonitrile (known from CN 101337937 A) (CAS 1105672-77-2), 3-bromo-*N*-[4-chloro-2-methyl-6-[(methylamino)thioxomethyl]phenyl]-1-(3-chloro-2-pyridinyl)-1*H*-pyrazole-5-carboxamide, (Liudaibenjiaxuanan, known from CN 103109816 A) (CAS 1232543-85-9); *N*-[4-chloro-2-[[(1,1-dimethylethyl)amino]carbonyl]-6-methylphenyl]-1-(3-chloro-2-pyridinyl)-3-(fluoromethoxy)-1*H*-Pyrazole-5-carboxamide (known from WO 2012/034403 A1) (CAS 1268277-22-0), *N*-[2-(5-amino-1,3,4-thiadiazol-2-yl)-4-chloro-6-methylphenyl]-3-bromo-1-(3-chloro-2-pyridinyl)-1*H*-pyrazole-5-carboxamide (known from WO 2011/085575 A1) (CAS 1233882-22-8), 4-[3-[2,6-dichloro-4-[(3,3-dichloro-2-propen-1-yl)oxy]phenoxy]propoxy]-2-methoxy-6-(trifluoromethyl)-pyrimidine (known from CN 101337940 A) (CAS 1108184-52-6); (2E)- and 2(*Z*)-2-[2-(4-cyanophenyl)-1-[3-(trifluoromethyl)phenyl]ethylidene]-*N*-[4-(difluoromethoxy)phenyl]-hydrazinecarboxamide (known from CN 101715774 A) (CAS 1232543-85-9); 3-(2,2-dichloroethenyl)-2,2-dimethyl-4-(1*H*-benzimidazol-2-yl)phenyl-cyclopropanecarboxylic acid ester (known from CN 103524422 A) (CAS 1542271-46-4); (4a*S*)-7-chloro-2,5-dihydro-2-[[(methoxycarbonyl)[4-[(trifluoromethyl)thio]phenyl] amino]carbonyl]-indeno[1,2-e][1,3,4]oxadiazine-4a(3*H*)-carboxylic acid methyl ester (known from CN 102391261 A) (CAS 1370358-69-2); 6-deoxy-3-*O*-ethyl-2,4-di-*O*-methyl-, 1-[*N*-[4-[1-[4-(1,1,2,2,2-pentafluoroethoxy)phenyl]-1*H*-1,2,4-triazol-3-yl]phenyl]carbamate]-α,-L-mannopyranose (known from US 2014/0275503 A1) (CAS 1181213-14-8); 8-(2-cyclopropylmethoxy-4-trifluoromethyl-phenoxy)-3-(6-trifluoromethyl-pyridazin-3-yl)-3-aza-bicyclo[3.2.1]octane (CAS 1253850-56-4), (8-*anti*)-8-(2-cyclopropylmethoxy-4-trifluoromethyl-phenoxy)-3-(6-trifluoromethyl-pyridazin-3-yl)-3-azabicyclo[3.2.1]octane (CAS 933798-27-7), (8-*syn*)-8-(2-cyclopropylmethoxy-4-trifluoromethyl-phenoxy)-3-(6-trifluoromethyl-pyridazin-3-yl)-3-aza-bicyclo[3.2.1]octane (known from WO 2007040280 A1, WO 2007040282 A1) (CAS 934001-66-8), N-[3-chloro-1-(3-pyridinyl)-1H-pyrazol-4-yl]-N-ethyl-3-[(3,3,3-trifluoropropyl)thio]-propanamide (known from WO 2015/058021 A1, WO 2015/058028 A1) (CAS 1477919-27-9) and N-[4-(aminothioxomethyl)-2-methyl-6-[(methylamino)carbonyl]phenyl]-3-bromo-1-(3-chloro-2-pyridinyl)-1*H*-pyrazole-5-carboxamide (known from CN 103265527 A) (CAS 1452877-50-7), 5-(1,3-dioxan-2-yl)-4-[[4-(trifluoromethyl)phenyl] methoxy]-pyrimidine (known from WO 2013/115391 A1) (CAS 1449021-97-9), 3-(4-chloro-2,6-dimethylphenyl)-4-hydroxy-8-methoxy-1-methyl-1,8-diazaspiro[4.5]dec-3-en-2-one (known from WO 2010/066780 A1, WO 2011/151146 A1) (CAS 1229023-34-0), 3-(4-chloro-2,6-dimethylphenyl)-8-methoxy-1-methyl-1,8-diazaspiro[4.5]decane-2,4-dione (known from WO 2014/187846 A1) (CAS 1638765-58-8), 3-(4-chloro-2,6-dimethylphenyl)-8-methoxy-1-methyl-2-oxo-1,8-diazaspiro[4.5]dec-3-en-4-yl-carbonic acid ethyl ester (known from WO 2010/066780 A1, WO 2011151146 A1) (CAS 1229023-00-0), N-[1-[(6-chloro-3-pyridinyl)methyl]-2(1*H*)-pyridinylidene]-2,2,2-trifluoro-acetamide (known from DE 3639877 A1, WO 2012029672 A1) (CAS 1363400-41-2), [N(*E*)]-N-[1-[(6-chloro-3-pyridinyl)methyl]-2(1H)-pyridinylidene]-2,2,2-trifluoro-acetamide, (known from WO 2016005276 A1) (CAS 1689566-03-7), [N(Z)]-N-[1-[(6-chloro-3-pyridinyl)methyl]-2(1H)-pyridinylidene]-2,2,2-trifluoro-acetamide, (CAS 1702305-40-5), 3-*endo*-3-[2-propoxy-4-(trifluoromethyl)phenoxy]-9-[[5-(trifluoromethyl)-2-pyridinyl]oxy]-9-azabicyclo[3.3.1]nonane (known from WO 2011/105506 A1, WO

2016/133011 A1) (CAS 1332838-17-1).

Examples of safeners which could be mixed with the compound combination of the invention or the compounds of formula (I) are, for example, benoxacor, cloquintocet (-mexyl), cyometrinil, cyprosulfamide, dichlormid, fenchlorazole (-ethyl), fenclorim, flurazole, fluxofenim, furilazole, isoxadifen (-ethyl), mefenpyr (-diethyl), naphthalic anhydride, oxabetrinil, 2-methoxy-N-({4-[(methylcarbamoyl)amino]phenyl}sulphonyl)benzamide (CAS 129531-12-0), 4-(dichloroacetyl)-1-oxa-4-azaspiro[4.5]decane (CAS 71526-07-3), 2,2,5-trimethyl-3-(dichloroacetyl)-1,3-oxazolidine (CAS 52836-31-4).

Examples of herbicides which could be mixed with the compound combination of the invention or the compounds of formula (I) are: Acetochlor, acifluorfen, acifluorfen-sodium, aclonifen, alachlor, allidochlor, alloxydim, alloxydim-sodium, ametryn, amicarbazone, amidochlor, amidosulfuron, 4-amino-3-chloro-6-(4-chloro-2-fluoro-3-methylphenyl)-5-fluoropyridine-2-carboxylic acid, aminocyclopyrachlor, aminocyclopyrachlor-potassium, aminocyclopyrachlor-methyl, aminopyralid, amitrole, ammoniumsulfamate, anilofos, asulam, atrazine, azafenidin, azimsulfuron, beflubutamid, benazolin, benazolin-ethyl, benfluralin, benfuresate, bensulfuron, bensulfuron-methyl, bensulide, bentazone, benzobicyclon, benzofenap, bicyclopyron, bifenox, bilanafos, bilanafos-sodium, bispyribac, bispyribac-sodium, bromacil, bromobutide, bromofenoxim, bromoxynil, bromoxynilbutyrate, -potassium, -heptanoate, and -octanoate, busoxinone, butachlor, butafenacil, butamifos, butenachlor, butralin, butroxydim, butylate, cafenstrole, carbetamide, carfentrazone, carfentrazone-ethyl, chloramben, chlorbromuron, chlorfenac, chlorfenac-sodium, chlorfenprop, chlorflurenol, chlorflurenolmethyl, chloridazon, chlorimuron, chlorimuron-ethyl, chlorophthalim, chlorotoluron, chlorthal-dimethyl, chlorsulfuron, cinidon, cinidon-ethyl, cinmethylin, cinosulfuron, clacyfos, clethodim, clodinafop, clodinafop-propargyl, clomazone, clomeprop, clopyralid, cloransulam, cloransulam-methyl, cumyluron, cyanamide, cyanazine, cycloate, cyclopyrimorate, cyclosulfamuron, cycloxydim, cyhalofop, cyhalofopbutyl, cyprazine, 2,4-D, 2,4-D-butotyl, -butyl, -dimethylammonium, -diolamin, -ethyl, -2-ethylhexyl, -isobutyl, -isooctyl, -isopropylammonium, -potassium, -triisopropanolammonium, and - trolamine, 2,4-DB, 2,4-DB-butyl, -dimethylammonium, -isooctyl, -potassium, and -sodium, daimuron (dymron), dalapon, dazomet, n-decanol, desmedipham, detosyl-pyrazolate (DTP), dicamba, dichlobenil, 2-(2,4-dichlorobenzyl)-4,4-dimethyl-1,2-oxazolidin-3-one, 2-(2,5-dichlorobenzyl)-4,4-dimethyl-1,2-oxazolidin-3-one, dichlorprop, dichlorprop-P, diclofop, diclofop-methyl, diclofop-P-methyl, diclosulam, difenzoquat, diflufenican, diflufenzopyr, diflufenzopyr-sodium, dimefuron, dimepiperate, dimethachlor, dimethametryn, dimethenamid, dimethenamid-P, dimetrasulfuron, dinitramine, dinoterb, diphenamid, diquat, diquat-dibromid, dithiopyr, diuron, DNOC, endothal, EPTC, esprocarb, ethalfluralin, ethametsulfuron, ethametsulfuron-methyl, ethiozin, ethofumesate, ethoxyfen, ethoxyfen-ethyl, ethoxysulfuron, etobenzanid, F-9600, F-5231, i.e. N-{2-chloro-4-fluoro-5-[4-(3-fluoropropyl)-5-oxo-4,5-dihydro-1H-tetrazol-1-yl]phenyl}ethanesulfonamide, F-7967, i. e. 3-[7-chloro-5-fluoro-2-(trifluoromethyl)-1H-benzimidazol-4-yl]-1-methyl-6-(trifluoromethyl)pyrimidine-2,4(1H,3H)-dione, fenoxaprop, fenoxaprop-P, fenoxaprop-ethyl, fenoxaprop-P-ethyl, fenoxasulfone, fenquinotrione, fentrazamide, flamprop, flamprop-M-isopropyl, flamprop-M-methyl, flazasulfuron, florasulam, fluazifop, fluazifop-P, fluazifop-butyl, fluazifop-P-butyl, flucarbazone, flucarbazone-sodium, flucetosulfuron, fluchloralin, flufenacet, flufenpyr, flufenpyr-ethyl, flumetsulam, flumiclorac, flumiclorac-pentyl, flumioxazin, fluometuron, flurenol, flurenol-butyl, -dimethylammonium and -methyl, fluoroglycofen, fluoroglycofenethyl, flupropanate, flupyrsulfuron, flupyrsulfuron-methyl-sodium, fluridone, flurochloridone, fluroxypyr, fluroxypyr-meptyl, flurtamone, fluthiacet, fluthiacet-methyl, fomesafen, fomesafen-sodium, foramsulfuron, fosamine, glufosinate, glufosinate-ammonium, glufosinate-P-sodium, glufosinate-P-ammonium, glufosinate-P-sodium, glyphosate, glyphosate-ammonium, -isopropylammonium, -diammonium, -dimethylammonium, -potassium, -sodium, and -trimesium, H-9201, i.e. O-(2,4-dimethyl-6-nitrophenyl) O-ethyl isopropylphosphoramidothioate, halauxifen, halauxifen-methyl, halosafen, halosulfuron, halosulfuron-methyl, haloxyfop, haloxyfop-P, haloxyfop-ethoxyethyl, haloxyfop-P-ethoxyethyl, haloxyfop-methyl, haloxyfop-P-methyl, hexazinone, HW-02, i.e. 1-(dimethoxyphosphoryl) ethyl-(2,4-dichlorophenoxy)acetate, imazamethabenz, imazamethabenz-methyl, imazamox, imazamox-ammonium, imazapic, imazapic-ammonium, imazapyr, imazapyr-isopropylammonium, imazaquin, imazaquin-ammonium, imazethapyr, imazethapyr-immonium, imazosulfuron, indanofan, indaziflam, iodosulfuron, iodosulfuron-methyl-sodium, ioxynil, ioxyniloctanoate, -potassium and -sodium, ipfencarbazone, isoproturon, isouron, isoxaben, isoxaflutole, karbutilate, KUH-043, i.e. 3-({[5-(difluoromethyl)-1-methyl-3-(trifluoromethyl)-1H-pyrazol-4-yl]methyl}sulfonyl)-5,5-dimethyl-4,5-dihydro-1,2-oxazole, ketospiradox, lactofen, lenacil, linuron, MCPA, MCPA-butotyl, -dimethylammonium, -2-ethylhexyl, -isopropylammonium, -potassium, and -sodium, MCPB, MCPB-methyl, -ethy,l and -sodium, mecoprop, mecoprop-sodium, and -butotyl, mecoprop-P, mecoprop-P-butotyl, -dimethylammonium, -2-ethylhexyl, and -potassium, mefenacet, mefluidide, mesosulfuron, mesosulfuron-methyl, mesotrione, methabenzthiazuron, metam, metamifop, metamitron, metazachlor, metazosulfuron, methabenzthiazuron, methiopyrsulfuron, methiozolin, methyl isothiocyanate, metobromuron, metolachlor, S-metolachlor, metosulam, metoxuron, metribuzin, metsulfuron, metsulfuron-methyl, molinat, monolinuron, monosulfuron, monosulfuron-ester, MT-5950, i.e. N-(3-chloro-4-isopropylphenyl)-2-methylpentan amide, NGGC-011, napropamide, NC-310, i.e. [5-(benzyloxy)-1-methyl-1H-pyrazol-4-yl](2,4-dichlorophenyl)methanone, neburon, nicosulfuron, nonanoic acid (pelargonic acid), norflurazon, oleic acid (fatty acids), orbencarb, orthosulfamuron, oryzalin, oxadiargyl, oxadiazon, oxasulfuron, oxaziclomefon, oxyfluorfen, paraquat, paraquat dichloride, pebulate, pendimethalin, penoxsulam, pentachlorphenol, pentoxazone, pethoxamid, petroleum oils, phenmedipham, picloram, picolinafen, pinoxaden, piperophos, pretilachlor, primisulfuron, primisulfuron-methyl, prodiamine, profoxydim, prometon,

prometryn, propachlor, propanil, propaquizafop, propazine, propham, propisochlor, propoxycarbazone, propoxycarbazonesodium, propyrisulfuron, propyzamide, prosulfocarb, prosulfuron, pyraclonil, pyraflufen, pyraflufenethyl, pyrasulfotole, pyrazolynate (pyrazolate), pyrazosulfuron, pyrazosulfuron-ethyl, pyrazoxyfen, pyribambenz, pyribambenz-isopropyl, pyribambenz-propyl, pyribenzoxim, pyributicarb, pyridafol, pyridate, pyriftalid, pyriminobac, pyriminobac-methyl, pyrimisulfan, pyrithiobac, pyrithiobac-sodium, pyroxasulfone, pyroxsulam, quinclorac, quinmerac, quinoclamine, quizalofop, quizalofop-ethyl, quizalofop-P, quizalofop-P-ethyl, quizalofop-P-tefuryl, rimsulfuron, saflufenacil, sethoxydim, siduron, simazine, simetryn, SL-261, sulcotrion, sulfentrazone, sulfometuron, sulfometuron-methyl, sulfosulfuron, SYN-523, SYP-249, i.e. 1-ethoxy-3-methyl-1-oxobut-3-en-2-yl 5-[2-chloro-4-(trifluoromethyl)phenoxy]-2-nitrobenzoate, SYP-300, i.e. 1-[7-fluoro-3-oxo-4-(prop-2-yn-1-yl)-3,4-dihydro-2H-1,4-benzoxazin-6-yl]-3-propyl-2-thioxoimidazolidine-4,5-dione, 2,3,6-TBA, TCA (trichloroacetic acid), TCA-sodium, tebuthiuron, tefuryltrione, tembotrione, tepraloxydim, terbacil, terbucarb, terbumeton, terbuthylazin, terbutryn, thenylchlor, thiazopyr, thiencarbazone, thiencarbazonemethyl, thifensulfuron, thifensulfuron-methyl, thiobencarb, tiafenacil, tolpyralate, topramezone, tralkoxydim, triafamone, tri-allate, triasulfuron, triaziflam, tribenuron, tribenuron-methyl, triclopyr, trietazine, trifloxysulfuron, trifloxysulfuron-sodium, trifludimoxazin, trifluralin, triflusulfuron, triflusulfuron-methyl, tritosulfuron, urea sulfate, vernolate, XDE-848, ZJ-0862, i.e. 3,4-dichloro-N-{2-[(4,6-dimethoxypyrimidin-2-yl)oxy]benzyl}aniline, and the following compounds:

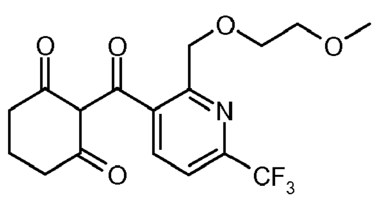

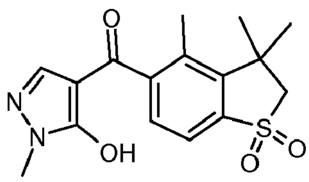

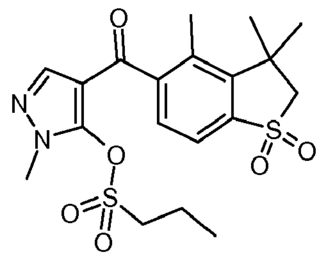

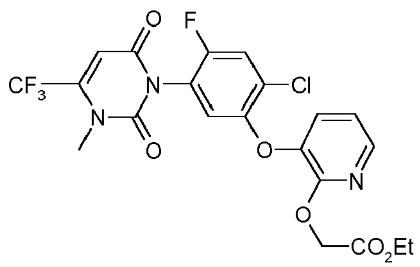

Examples for plant growth regulators which could be mixed with the compound composition of the invention or the compounds of formula (I) are:

Acibenzolar, acibenzolar-S-methyl, 5-aminolevulinic acid, ancymidol, 6-benzylaminopurine, Brassinolid, catechine, chlormequat chloride, cloprop, cyclanilide, 3-(cycloprop-1-enyl) propionic acid, daminozide, dazomet, n-decanol, dikegulac, dikegulac-sodium, endothal, endothaldipotassium, -disodium, and -mono(N,N-dimethylalkylammonium), ethephon, flumetralin, flurenol, flurenol-butyl, flurprimidol, forchlorfenuron, gibberellic acid, inabenfide, indol-3-acetic acid (IAA), 4-indol-3-ylbutyric acid, isoprothiolane, probenazole, jasmonic acid, maleic hydrazide, mepiquat chloride, 1-methylcyclopropene, methyl jasmonate, 2-(1-naphthyl)acetamide, 1-naphthylacetic acid, 2- naphthyloxyacetic acid, nitrophenolate-mixture, paclobutrazol, N-(2-phenylethyl)-beta-alanine, N-phenyl-phthalamic acid, prohexadione, prohexadione-calcium, prohydrojasmone, salicylic acid, strigolactone, tecnazene, thidiazuron, triacontanol, trinexapac, trinexapac-ethyl, tsitodef, uniconazole, uniconazole-P.

*Methods and uses*

The invention also relates to a method for controlling unwanted microorganisms, characterized in that a compound combination or a formulation according to the invention is applied to the microorganisms and/or in their habitat.

The invention further relates to seed which has been coated with a compound combination or a formulation according to the invention.

The invention finally provides a method for protecting seed against unwanted microorganisms by using seed treated with a compound combination or a formulation according to the invention.

The compound combination and formulation according to the invention have potent microbicidal activity and can be used for control of unwanted microorganisms, such as fungi and bacteria, in crop protection and in the protection

of materials.

The compound combination and formulation according to the invention have very good fungicidal properties and can be used in crop protection, for example for control of Plasmodiophoromycetes, Oomycetes, Chytridiomycetes, Zygomycetes, Ascomycetes, Basidiomycetes and Deuteromycetes.

Bactericides can be used in crop protection, for example, for control of Pseudomonadaceae, Rhizobiaceae, Enterobacteriaceae, Corynebacteriaceae and Streptomycetaceae.

The compound combination and formulation according to the invention can be used for curative or protective control of phytopathogenic fungi. The invention therefore also relates to curative and protective methods for controlling phytopathogenic fungi by the use of the inventive combination or formulation, which are applied to the seed, the plant or plant parts, the fruit or the soil in which the plants grow.

*Plants*

All plants and plant parts can be treated in accordance with the invention. Plants are understood here to mean all plants and plant populations, such as desired and undesired wild plants or crop plants (including naturally occurring crop plants). Crop plants may be plants which can be obtained by conventional breeding and optimization methods or by biotechnological and genetic engineering methods or combinations of these methods, including the transgenic plants and including the plant cultivars which are protectable and non-protectable by plant breeders' rights. Plant parts are understood to mean all parts and organs of plants above and below the ground, such as shoot, leaf, flower and root, examples of which include leaves, needles, stalks, stems, flowers, fruit bodies, fruits and seeds, and also roots, tubers and rhizomes. The plant parts also include harvested material and vegetative and generative propagation material, for example cuttings, tubers, rhizomes, slips and seeds.

Plants which can be treated in accordance with the invention include the following: cotton, flax, grapevine, fruit, vegetables, such as *Rosaceae sp.* (for example pome fruits such as apples and pears, but also stone fruits such as apricots, cherries, almonds and peaches, and soft fruits such as strawberries), *Ribesioidae sp., Juglandaceae sp., Betulaceae sp., Anacardiaceae sp., Fagaceae sp., Moraceae sp., Oleaceae sp., Actinidaceae sp., Lauraceae sp., Musaceae sp.* (for example banana trees and plantations), *Rubiaceae sp.* (for example coffee), *Theaceae sp., Sterculiceae sp., Rutaceae sp.* (for example lemons, oranges and grapefruit); *Solanaceae sp.* (for example tomatoes), *Liliaceae sp., Asteraceae sp.* (for example lettuce), *Umbelliferae sp., Cruciferae sp., Chenopodiaceae sp., Cucurbitaceae sp.* (for example cucumber), *Alliaceae sp.* (for example leek, onion), *Papilionaceae sp.* (for example peas); major crop plants, such as *Gramineae sp.* (for example maize, turf, cereals such as wheat, rye, rice, barley, oats, millet and triticale), *Asteraceae sp.* (for example sunflower), *Brassicaceae sp.* (for example white cabbage, red cabbage, broccoli, cauliflower, Brussels sprouts, pak choi, kohlrabi, radishes, and oilseed rape, mustard, horseradish and cress), *Fabacae sp.* (for example bean, peanuts), *Papilionaceae sp.* (for example soya bean), *Solanaceae sp.* (for example potatoes), *Chenopodiaceae sp.* (for example sugar beet, fodder beet, swiss chard, beetroot); useful plants and ornamental plants for gardens and wooded areas; and genetically modified varieties of each of these plants.

*Pathogens*

Non-limiting examples of pathogens of fungal diseases which can be treated in accordance with the invention include:

diseases caused by powdery mildew pathogens, for example Blumeria species, for example Blumeria graminis; Podosphaera species, for example Podosphaera leucotricha; Sphaerotheca species, for example Sphaerotheca fuliginea; Uncinula species, for example Uncinula necator;

diseases caused by rust disease pathogens, for example Gymnosporangium species, for example Gymnosporangium sabinae; Hemileia species, for example Hemileia vastatrix; Phakopsora species, for example Phakopsora pachyrhizi or Phakopsora meibomiae; Puccinia species, for example Puccinia recondita, Puccinia graminis oder Puccinia striiformis; Uromyces species, for example Uromyces appendiculatus;

diseases caused by pathogens from the group of the Oomycetes, for example Albugo species, for example Albugo candida; Bremia species, for example Bremia lactucae; Peronospora species, for example Peronospora pisi or P. brassicae; Phytophthora species, for example Phytophthora infestans; Plasmopara species, for example Plasmopara viticola; Pseudoperonospora species, for example Pseudoperonospora humuli or Pseudoperonospora cubensis; Pythium species, for example Pythium ultimum;

leaf blotch diseases and leaf wilt diseases caused, for example, by Alternaria species, for example Alternaria solani;

Cercospora species, for example Cercospora beticola; Cladiosporium species, for example Cladiosporium cucumerinum; Cochliobolus species, for example Cochliobolus sativus (conidial form: Drechslera, syn: Helminthosporium) or Cochliobolus miyabeanus; Colletotrichum species, for example Colletotrichum lindemuthanium; Cycloconium species, for example Cycloconium oleaginum; Diaporthe species, for example Diaporthe citri; Elsinoe species, for example Elsinoe fawcettii; Gloeosporium species, for example Gloeosporium laeticolor; Glomerella species, for example Glomerella cingulata; Guignardia species, for example Guignardia bidwelli; Leptosphaeria species, for example Leptosphaeria maculans; Magnaporthe species, for example Magnaporthe grisea; Microdochium species, for example Microdochium nivale; Mycosphaerella species, for example Mycosphaerella graminicola, Mycosphaerella arachidicola or Mycosphaerella fijiensis; Phaeosphaeria species, for example Phaeosphaeria nodorum; Pyrenophora species, for example Pyrenophora teres or Pyrenophora tritici repentis; Ramularia species, for example Ramularia collo-cygni or Ramularia areola; Rhynchosporium species, for example Rhynchosporium secalis; Septoria species, for example Septoria apii or Septoria lycopersici; Stagonospora species, for example Stagonospora nodorum; Typhula species, for example Typhula incarnata; Venturia species, for example Venturia inaequalis;

root and stem diseases caused, for example, by Corticium species, for example Corticium graminearum; Fusarium species, for example Fusarium oxysporum; Gaeumannomyces species, for example Gaeumannomyces graminis; Plasmodiophora species, for example Plasmodiophora brassicae; Rhizoctonia species, for example Rhizoctonia solani; Sarocladium species, for example Sarocladium oryzae; Sclerotium species, for example Sclerotium oryzae; Tapesia species, for example Tapesia acuformis; Thielaviopsis species, for example Thielaviopsis basicola;

ear and panicle diseases (including corn cobs) caused, for example, by Alternaria species, for example Alternaria spp.; Aspergillus species, for example Aspergillus flavus; Cladosporium species, for example Cladosporium cladosporioides; Claviceps species, for example Claviceps purpurea; Fusarium species, for example Fusarium culmorum; Gibberella species, for example Gibberella zeae; Monographella species, for example Monographella nivalis; Stagnospora species, for example Stagnospora nodorum;

diseases caused by smut fungi, for example Sphacelotheca species, for example Sphacelotheca reiliana; Tilletia species, for example Tilletia caries or Tilletia controversa; Urocystis species, for example Urocystis occulta; Ustilago species, for example Ustilago nuda;

fruit rot caused, for example, by Aspergillus species, for example Aspergillus flavus; Botrytis species, for example Botrytis cinerea; Penicillium species, for example Penicillium expansum or Penicillium purpurogenum; Rhizopus species, for example Rhizopus stolonifer; Sclerotinia species, for example Sclerotinia sclerotiorum; Verticilium species, for example Verticilium alboatrum;

seed- and soil-borne rot and wilt diseases, and also diseases of seedlings, caused, for example, by Alternaria species, for example Alternaria brassicicola; Aphanomyces species, for example Aphanomyces euteiches; Ascochyta species, for example Ascochyta lentis; Aspergillus species, for example Aspergillus flavus; Cladosporium species, for example Cladosporium herbarum; Cochliobolus species, for example Cochliobolus sativus (conidial form: Drechslera, Bipolaris Syn: Helminthosporium); Colletotrichum species, for example Colletotrichum coccodes; Fusarium species, for example Fusarium culmorum; Fusarium virguliforme, Fuasrium oxysporum, Gibberella species, for example Gibberella zeae; Macrophomina species, for example Macrophomina phaseolina; Microdochium species, for example Microdochium nivale; Monographella species, for example Monographella nivalis; Penicillium species, for example Penicillium expansum; Phoma species, for example Phoma lingam; Phomopsis species, for example Phomopsis sojae; Phytophthora species, for example Phytophthora cactorum; Pyrenophora species, for example Pyrenophora graminea; Pyricularia species, for example Pyricularia oryzae; Pythium species, for example Pythium ultimum; Rhizoctonia species, for example Rhizoctonia solani; Rhizopus species, for example Rhizopus oryzae; Sclerotium species, for example Sclerotium rolfsii; Septoria species, for example Septoria nodorum; Typhula species, for example Typhula incarnata; Verticillium species, for example Verticillium dahliae;

cancers, galls and witches' broom caused, for example, by Nectria species, for example Nectria galligena;

wilt diseases caused, for example, by Monilinia species, for example Monilinia laxa;

deformations of leaves, flowers and fruits caused, for example, by Exobasidium species, for example Exobasidium vexans; Taphrina species, for example Taphrina deformans;

degenerative diseases in woody plants, caused, for example, by Esca species, for example Phaeomoniella chlamydospora, Phaeoacremonium aleophilum or Fomitiporia mediterranea; Ganoderma species, for example Ganoderma boninense;

diseases of flowers and seeds caused, for example, by Botrytis species, for example Botrytis cinerea;

diseases of plant tubers caused, for example, by Rhizoctonia species, for example Rhizoctonia solani; Helminthosporium species, for example Helminthosporium solani;

diseases caused by bacterial pathogens, for example Xanthomonas species, for example Xanthomonas campestris pv. oryzae; Pseudomonas species, for example Pseudomonas syringae pv. lachrymans; Erwinia species, for example Erwinia amylovora.

Preference is given to controlling the following diseases of soya beans:
Fungal diseases on leaves, stems, pods and seeds caused, for example, by Alternaria leaf spot (Alternaria spec. atrans tenuissima), Anthracnose (Colletotrichum gloeosporoides dematium var. truncatum), brown spot (Septoria glycines), cercospora leaf spot and blight (Cercospora kikuchii), choanephora leaf blight (Choanephora infundibulifera trispora (Syn.)), dactuliophora leaf spot (Dactuliophora glycines), downy mildew (Peronospora manshurica), drechslera blight (Drechslera glycini), frogeye leaf spot (Cercospora sojina), leptosphaerulina leaf spot (Leptosphaerulina trifolii), phyllostica leaf spot (Phyllosticta sojaecola), pod and stem blight (Phomopsis sojae), powdery mildew (Microsphaera diffusa), pyrenochaeta leaf spot (Pyrenochaeta glycines), rhizoctonia aerial, foliage, and web blight (Rhizoctonia solani), rust (Phakopsora pachyrhizi, Phakopsora meibomiae), scab (Sphaceloma glycines), stemphylium leaf blight (Stemphylium botryosum), target spot (Corynespora cassiicola).

Fungal diseases on roots and the stem base caused, for example, by black root rot (Calonectria crotalariae), charcoal rot (Macrophomina phaseolina), fusarium blight or wilt, root rot, and pod and collar rot (Fusarium oxysporum, Fusarium orthoceras, Fusarium semitectum, Fusarium equiseti), mycoleptodiscus root rot (Mycoleptodiscus terrestris), neocosmospora (Neocosmospora vasinfecta), pod and stem blight (Diaporthe phaseolorum), stem canker (Diaporthe phaseolorum var. caulivora), phytophthora rot (Phytophthora megasperma), brown stem rot (Phialophora gregata), pythium rot (Pythium aphanidermatum, Pythium irregulare, Pythium debaryanum, Pythium myriotylum, Pythium ultimum), rhizoctonia root rot, stem decay, and damping-off (Rhizoctonia solani), sclerotinia stem decay (Sclerotinia sclerotiorum), sclerotinia southern blight (Sclerotinia rolfsii), thielaviopsis root rot (Thielaviopsis basicola).

Preference is further given to controlling leaf blotch diseases and leaf wilt diseases as well as root and stem diseases of fruits and vegetables.

*Plant Growth Regulation*

In some cases, the compound combination and formulation according to the invention can, at particular concentrations or application rates, also be used as growth regulators or agents to improve plant properties, or as microbicides, for example as fungicides, antimycotics, bactericides, viricides (including formulations against viroids) or as formulations against MLO (Mycoplasma-like organisms) and RLO (Rickettsia-like organisms).

The compound combination and a formulation according to the invention intervene in physiological processes of plants and can therefore also be used as plant growth regulators. Plant growth regulators may exert various effects on plants. The effect of the substances depends essentially on the time of application in relation to the developmental stage of the plant, and also on the amounts of active ingredient applied to the plants or their environment and on the type of application. In each case, growth regulators should have a particular desired effect on the crop plants.

Growth regulating effects, comprise earlier germination, better emergence, more developed root system and/or improved root growth, increased ability of tillering, more productive tillers, earlier flowering, increased plant height and/or biomass, shorting of stems, improvements in shoot growth, number of kernels/ear, number of ears/$m^2$, number of stolons and/or number of flowers, enhanced harvest index, bigger leaves, less dead basal leaves, improved phyllotaxy, earlier maturation / earlier fruit finish, homogenous riping, increased duration of grain filling, better fruit finish, bigger fruit/vegetable size, sprouting resistance and reduced lodging.

Increased or improved yield is referring to total biomass per hectare, yield per hectare, kernel/fruit weight, seed size and/or hectolitre weight as well as to improved product quality, comprising:

improved processability relating to size distribution (kernel, fruit, etc.), homogenous riping, grain moisture, better milling, better vinification, better brewing, increased juice yield, harvestability, digestibility, sedimentation value, falling number, pod stability, storage stability, improved fiber length/strength/uniformity, increase of milk and/or meet quality of silage fed animals, adaption to cooking and frying;

further comprising improved marketability relating to improved fruit/grain quality, size distribution (kernel, fruit, etc.), increased storage / shelf-life, firmness / softness, taste (aroma, texture, etc.), grade (size, shape, number of berries, etc.), number of berries/fruits per bunch, crispness, freshness, coverage with wax, frequency of physiological disorders, colour, etc.;

further comprising increased desired ingredients such as e.g. protein content, fatty acids, oil content, oil quality, aminoacid composition, sugar content, acid content (pH), sugar/acid ratio (Brix), polyphenols, starch content, nutritional quality, gluten content/index, energy content, taste, etc.;

and further comprising decreased undesired ingredients such as e.g. less mycotoxines, less aflatoxines, geosmin level, phenolic aromas, lacchase, polyphenol oxidases and peroxidases, nitrate content etc.

Plant growth-regulating compounds can be used, for example, to slow down the vegetative growth of the plants. Such growth depression is of economic interest, for example, in the case of grasses, since it is thus possible to reduce the frequency of grass cutting in ornamental gardens, parks and sport facilities, on roadsides, at airports or in fruit crops. Also of significance is the inhibition of the growth of herbaceous and woody plants on roadsides and in the vicinity of pipelines or overhead cables, or quite generally in areas where vigorous plant growth is unwanted.

Also important is the use of growth regulators for inhibition of the longitudinal growth of cereal. This reduces or completely eliminates the risk of lodging of the plants prior to harvest. In addition, growth regulators in the case of cereals can strengthen the culm, which also counteracts lodging. The employment of growth regulators for shortening and strengthening culms allows the deployment of higher fertilizer volumes to increase the yield, without any risk of lodging of the cereal crop.

In many crop plants, vegetative growth depression allows denser planting, and it is thus possible to achieve higher yields based on the soil surface. Another advantage of the smaller plants obtained in this way is that the crop is easier to cultivate and harvest.

Reduction of the vegetative plant growth may also lead to increased or improved yields because the nutrients and assimilates are of more benefit to flower and fruit formation than to the vegetative parts of the plants.

Alternatively, growth regulators can also be used to promote vegetative growth. This is of great benefit when harvesting the vegetative plant parts. However, promoting vegetative growth may also promote generative growth in that more assimilates are formed, resulting in more or larger fruits.

Furthermore, beneficial effects on growth or yield can be achieved through improved nutrient use efficiency, especially nitrogen (N)-use efficiency, phosphours (P)-use efficiency, water use efficiency, improved transpiration, respiration and/or CO2 assimilation rate, better nodulation, improved Cametabolism etc.

Likewise, growth regulators can be used to alter the composition of the plants, which in turn may result in an improvement in quality of the harvested products. Under the influence of growth regulators, parthenocarpic fruits may be formed. In addition, it is possible to influence the sex of the flowers. It is also possible to produce sterile pollen, which is of great importance in the breeding and production of hybrid seed.

Use of growth regulators can control the branching of the plants. On the one hand, by breaking apical dominance, it is possible to promote the development of side shoots, which may be highly desirable particularly in the cultivation of ornamental plants, also in combination with an inhibition of growth. On the other hand, however, it is also possible to inhibit the growth of the side shoots. This effect is of particular interest, for example, in the cultivation of tobacco or in the cultivation of tomatoes.

Under the influence of growth regulators, the amount of leaves on the plants can be controlled such that defoliation of the plants is achieved at a desired time. Such defoliation plays a major role in the mechanical harvesting of cotton, but is also of interest for facilitating harvesting in other crops, for example in viticulture. Defoliation of the plants can also be undertaken to lower the transpiration of the plants before they are transplanted.

Furthermore, growth regulators can modulate plant senescence, which may result in prolonged green leaf area duration, a longer grain filling phase, improved yield quality, etc.

Growth regulators can likewise be used to regulate fruit dehiscence. On the one hand, it is possible to prevent premature fruit dehiscence. On the other hand, it is also possible to promote fruit dehiscence or even flower abortion to achieve a desired mass ("thinning"). In addition it is possible to use growth regulators at the time of harvest to reduce the forces required to detach the fruits, in order to allow mechanical harvesting or to facilitate manual harvesting.

Growth regulators can also be used to achieve faster or else delayed ripening of the harvested material before or after harvest. This is particularly advantageous as it allows optimal adjustment to the requirements of the market. Moreover, growth regulators in some cases can improve the fruit colour. In addition, growth regulators can also be used to synchronize maturation within a certain period of time. This establishes the prerequisites for complete mechanical or manual harvesting in a single operation, for example in the case of tobacco, tomatoes or coffee.

By using growth regulators, it is additionally possible to influence the resting of seed or buds of the plants, such

that plants such as pineapple or ornamental plants in nurseries, for example, germinate, sprout or flower at a time when they are normally not inclined to do so. In areas where there is a risk of frost, it may be desirable to delay budding or germination of seeds with the aid of growth regulators, in order to avoid damage resulting from late frosts.

Finally, growth regulators can induce resistance of the plants to frost, drought or high salinity of the soil. This allows the cultivation of plants in regions which are normally unsuitable for this purpose.

*Resistance Induction / Plant Health and other effects*

The compound combination and formulation according to the invention may also exhibit a potent strengthening effect in plants. Accordingly, they can be used for mobilizing the defences of the plant against attack by undesirable microorganisms.

Plant-strengthening (resistance-inducing) substances in the present context are substances capable of stimulating the defence system of plants in such a way that the treated plants, when subsequently inoculated with undesirable microorganisms, develop a high degree of resistance to these microorganisms.

Further, in context with the present invention plant physiology effects comprise the following:

Abiotic stress tolerance, comprising tolerance to high or low temperatures, drought tolerance and recovery after drought stress, water use efficiency (correlating to reduced water consumption), flood tolerance, ozone stress and UV tolerance, tolerance towards chemicals like heavy metals, salts, pesticides etc.

Biotic stress tolerance, comprising increased fungal resistance and increased resistance against nematodes, viruses and bacteria. In context with the present invention, biotic stress tolerance preferably comprises increased fungal resistance and increased resistance against nematodes.

Increased plant vigor, comprising plant health / plant quality and seed vigor, reduced stand failure, improved appearance, increased recovery after periods of stress, improved pigmentation (e.g. chlorophyll content, stay-green effects, etc.) and improved photosynthetic efficiency.

*Mycotoxins*

In addition, the compound combination and formulation according to the invention can reduce the mycotoxin content in the harvested material and the foods and feeds prepared therefrom. Mycotoxins include particularly, but not exclusively, the following: deoxynivalenol (DON), nivalenol, 15-Ac-DON, 3-Ac-DON, T2- and HT2-toxin, fumonisins, zearalenon, moniliformin, fusarin, diaceotoxyscirpenol (DAS), beauvericin, enniatin, fusaroproliferin, fusarenol, ochratoxins, patulin, ergot alkaloids and aflatoxins which can be produced, for example, by the following fungi: *Fusarium* spec., such as *F. acuminatum, F. asiaticum, F. avenaceum, F. crookwellense, F. culmorum, F. graminearum* (*Gibberella zeae*), *F. equiseti, F. fujikoroi, F. musarum, F. oxysporum, F. proliferatum, F. poae, F. pseudograminearum, F. sambucinum, F. scirpi, F. semitectum, F. solani, F. sporotrichoides, F. langsethiae, F. subglutinans, F. tricinctum, F. verticillioides* etc., and also by *Aspergillus* spec., such as *A. flavus, A. parasiticus, A. nomius, A. ochraceus, A. clavatus, A. terreus, A. versicolor, Penicillium* spec., such as *P. verrucosum, P. viridicatum, P. citrinum, P. expansum, P. claviforme, P. roqueforti, Claviceps* spec., such as *C. purpurea, C. fusiformis, C. paspali, C. africana, Stachybotrys* spec. and others.

*Material Protection*

The compound combination and formulation according to the invention can also be used in the protection of materials, for protection of industrial materials against attack and destruction by phytopathogenic fungi.

In addition, the compound combination and formulation according to the invention can be used as antifouling formulations, alone or in combinations with other active ingredients.

Industrial materials in the present context are understood to mean inanimate materials which have been prepared for use in industry. For example, industrial materials which are to be protected by inventive formulations from microbial alteration or destruction may be adhesives, glues, paper, wallpaper and board/cardboard, textiles, carpets, leather, wood, fibers and tissues, paints and plastic articles, cooling lubricants and other materials which can be infected with or destroyed by microorganisms. Parts of production plants and buildings, for example cooling-water circuits, cooling and heating systems and ventilation and air-conditioning units, which may be impaired by the proliferation of microorganisms may also be mentioned within the scope of the materials to be protected. Industrial materials within the scope of the present invention preferably include adhesives, sizes, paper and card, leather, wood, paints, cooling lubricants and heat transfer fluids, more preferably wood.

The compound combination and formulation according to the invention may prevent adverse effects, such as rotting, decay, discoloration, decoloration or formation of mould.

In the case of treatment of wood the compound combination and formulation according to the invention may also be used against fungal diseases liable to grow on or inside timber. The term "timber" means all types of species of

wood, and all types of working of this wood intended for construction, for example solid wood, high-density wood, laminated wood, and plywood. The method for treating timber according to the invention mainly consists in contacting a formulation according to the invention; this includes for example direct application, spraying, dipping, injection or any other suitable means.

In addition, the compound combination and formulation according to the invention can be used to protect objects which come into contact with saltwater or brackish water, especially hulls, screens, nets, buildings, moorings and signalling systems, from fouling.

The compound combination and formulation according to the invention can also be employed for protecting storage goods. Storage goods are understood to mean natural substances of vegetable or animal origin or processed products thereof which are of natural origin, and for which long-term protection is desired. Storage goods of vegetable origin, for example plants or plant parts, such as stems, leaves, tubers, seeds, fruits, grains, can be protected freshly harvested or after processing by (pre)drying, moistening, comminuting, grinding, pressing or roasting. Storage goods also include timber, both unprocessed, such as construction timber, electricity poles and barriers, or in the form of finished products, such as furniture. Storage goods of animal origin are, for example, hides, leather, furs and hairs. The inventive formulation may prevent adverse effects, such as rotting, decay, discoloration, decoloration or formation of mould.

Microorganisms capable of degrading or altering the industrial materials include, for example, bacteria, fungi, yeasts, algae and slime organisms. The compounds of the formula (I) preferably act against fungi, especially moulds, wood-discoloring and wood-destroying fungi (*Ascomycetes, Basidiomycetes, Deuteromycetes* and *Zygomycetes*), and against slime organisms and algae. Examples include microorganisms of the following genera: *Alternaria,* such as *Alternaria tenuis; Aspergillus,* such as *Aspergillus niger; Chaetomium,* such as *Chaetomium globosum; Coniophora,* such as *Coniophora puetana; Lentinus,* such as *Lentinus tigrinus; Penicillium,* such as *Penicillium glaucum; Polyporus,* such as *Polyporus versicolor*; *Aureobasidium,* such as *Aureobasidium pullulans; Sclerophoma,* such as *Sclerophoma pityophila*; *Trichoderma,* such as *Trichoderma viride; Ophiostoma spp., Ceratocystis spp., Humicola spp., Petriella spp., Trichurus spp., Coriolus spp., Gloeophyllum spp., Pleurotus spp., Poria spp., Serpula spp.* and *Tyromyces spp., Cladosporium spp., Paecilomyces spp. Mucor spp., Escherichia,* such as *Escherichia coli; Pseudomonas,* such as *Pseudomonas aeruginosa*; *Staphylococcus,* such as *Staphylococcus aureus, Candida spp.* and *Saccharomyces spp.,* such as *Saccharomyces cerevisae.*

*Formulations*

The present invention further relates to a formulation for controlling harmful microorganisms, preferably phytopathogenic harmful fungi, in crop protection and in the protection of materials, characterized by a content of an active compound combination according to the invention, in addition to at least one extender and/or surfactant.

According to the invention, a carrier is a natural or synthetic, organic or inorganic substance with which the active ingredients are mixed or combined for better applicability, in particular for application to plants or plant parts or seed. The carrier, which may be solid or liquid, is generally inert and should be suitable for use in agriculture.

Useful solid carriers include: for example ammonium salts and natural rock flours, such as kaolins, clays, talc, chalk, quartz, attapulgite, montmorillonite or diatomaceous earth, and synthetic rock flours, such as finely divided silica, alumina and silicates; useful solid carriers for granules include: for example, crushed and fractionated natural rocks such as calcite, marble, pumice, sepiolite and dolomite, and also synthetic granules of inorganic and organic flours, and granules of organic material such as paper, sawdust, coconut shells, maize cobs and tobacco stalks; useful emulsifiers and/or foam-formers include: for example nonionic and anionic emulsifiers, such as polyoxyethylene fatty acid esters, polyoxyethylene fatty alcohol ethers, for example alkylaryl polyglycol ethers, alkylsulphonates, alkyl sulphates, arylsulphonates and also protein hydrolysates; suitable dispersants are nonionic and/or ionic substances, for example from the classes of the alcohol-POE and/or -POP ethers, acid and/or POP POE esters, alkylaryl and/or POP POE ethers, fat and/or POP POE adducts, POE- and/or POP-polyol derivatives, POE- and/or POP-sorbitan or - sugar adducts, alkyl or aryl sulphates, alkyl- or arylsulphonates and alkyl or aryl phosphates or the corresponding PO-ether adducts. Additionally suitable are oligo- or polymers, for example those derived from vinylic monomers, from acrylic acid, from EO and/or PO alone or in combination with, for example, (poly)alcohols or (poly)amines. It is also possible to use lignin and its sulphonic acid derivatives, unmodified and modified celluloses, aromatic and/or aliphatic sulphonic acids and also their adducts with formaldehyde.

The active ingredients can be converted to the customary formulations, such as solutions, emulsions, wettable powders, water- and oil-based suspensions, powders, dusts, pastes, soluble powders, soluble granules, granules for broadcasting, suspoemulsion concentrates, natural products impregnated with active ingredient, synthetic substances impregnated with active ingredient, fertilizers and also microencapsulations in polymeric substances.

The active ingredients can be applied as such, in the form of their formulations or the use forms prepared therefrom, such as ready-to-use solutions, emulsions, water- or oil-based suspensions, powders, wettable powders, pastes, soluble powders, dusts, soluble granules, granules for broadcasting, suspoemulsion concentrates, natural products impregnated with active ingredient, synthetic substances impregnated with active ingredient, fertilizers and also microencapsulations in polymeric substances. Application is accomplished in a customary manner, for example by watering, spraying, atomizing, broadcasting, dusting, foaming, spreading-on and the like. It is also possible to deploy the active ingredients by the ultra-low volume method or to inject the active ingredient preparation/the active ingredient itself into the soil. It is also possible to treat the seed of the plants.

The formulations mentioned can be prepared in a manner known per se, for example by mixing the active ingredients with at least one customary extender, solvent or diluent, emulsifier, dispersant and/or binder or fixing agent, wetting agent, a water repellent, if appropriate siccatives and UV stabilizers and if appropriate dyes and pigments, antifoams, preservatives, secondary thickeners, stickers, gibberellins and also other processing auxiliaries.

The present invention includes not only formulations which are already ready for use and can be deployed with a suitable apparatus to the plant or the seed, but also commercial concentrates which have to be diluted with water prior to use.

The formulations according to the invention may be present as such or in their (commercial) formulations and in the use forms prepared from these formulations as a mixture with other (known) active ingredients, such as insecticides, attractants, sterilants, bactericides, acaricides, nematicides, growth regulators, herbicides, fertilizers, safeners and/or semiochemicals.

The auxiliaries used may be those substances which are suitable for imparting particular properties to the formulation itself or and/or to preparations derived therefrom (for example spray liquors, seed dressings), such as certain technical properties and/or also particular biological properties. Typical auxiliaries include: extenders, solvents and carriers.

Suitable extenders are, for example, water, polar and nonpolar organic chemical liquids, for example from the classes of the aromatic and nonaromatic hydrocarbons (such as paraffins, alkylbenzenes, alkylnaphthalenes, chlorobenzenes), the alcohols and polyols (which may optionally also be substituted, etherified and/or esterified), the ketones (such as acetone, cyclohexanone), esters (including fats and oils) and (poly)ethers, the unsubstituted and substituted amines, amides, lactams (such as N-alkylpyrrolidones) and lactones, the sulphones and sulphoxides (such as dimethyl sulphoxide).

Liquefied gaseous extenders or carriers are understood to mean liquids which are gaseous at standard temperature and under standard pressure, for example aerosol propellants such as halohydrocarbons, or else butane, propane, nitrogen and carbon dioxide.

In the formulations it is possible to use tackifiers such as carboxymethylcellulose, natural and synthetic polymers in the form of powders, granules or latices, such as gum arabic, polyvinyl alcohol and polyvinyl acetate, or else natural phospholipids such as cephalins and lecithins and synthetic phospholipids. Further additives may be mineral and vegetable oils.

If the extender used is water, it is also possible to use, for example, organic solvents as auxiliary solvents. Useful liquid solvents are essentially: aromatics such as xylene, toluene or alkylnaphthalenes, chlorinated aromatics or chlorinated aliphatic hydrocarbons such as chlorobenzenes, chloroethylenes or methylene chloride, aliphatic hydrocarbons such as cyclohexane or paraffins, for example petroleum fractions, alcohols such as butanol or glycol and their ethers and esters, ketones such as acetone, methyl ethyl ketone, methyl isobutyl ketone or cyclohexanone, strongly polar solvents such as dimethylformamide and dimethyl sulphoxide, or else water.

The formulations according to the invention may additionally comprise further components, for example surfactants. Suitable surfactants are emulsifiers and/or foam formers, dispersants or wetting agents having ionic or nonionic properties, or mixtures of these surfactants. Examples thereof are salts of polyacrylic acid, salts of lignosulphonic acid, salts of phenolsulphonic acid or naphthalenesulphonic acid, polycondensates of ethylene oxide with fatty alcohols or with fatty acids or with fatty amines, substituted phenols (preferably alkylphenols or arylphenols), salts of sulphosuccinic esters, taurine derivatives (preferably alkyl taurates), phosphoric esters of polyethoxylated alcohols or phenols, fatty esters of polyols, and derivatives of the compounds containing sulphates, sulphonates and phosphates, for example alkylaryl polyglycol ethers, alkylsulphonates, alkyl sulphates, arylsulphonates, protein hydrolysates, lignosulphite waste liquors and methylcellulose. The presence of a surfactant is necessary if one of the active ingredients and/or one of the inert carriers is insoluble in water and when application is effected in water. The proportion of surfactants is between 5 and 40 per cent by weight of the inventive formulation.

It is possible to use dyes such as inorganic pigments, for example iron oxide, titanium oxide and Prussian Blue, and organic dyes such as alizarin dyes, azo dyes and metal phthalocyanine dyes, and trace nutrients such as salts of iron, manganese, boron, copper, cobalt, molybdenum and zinc.

Further additives may be perfumes, mineral or vegetable, optionally modified oils, waxes and nutrients (including trace nutrients), such as salts of iron, manganese, boron, copper, cobalt, molybdenum and zinc.

Additional components may be stabilizers, such as cold stabilizers, preservatives, antioxidants, light stabilizers, or other agents which improve chemical and/or physical stability.

If appropriate, other additional components may also be present, for example protective colloids, binders,

adhesives, thickeners, thixotropic substances, penetrants, stabilizers, sequestering agents, complex formers. In general, the active ingredients can be combined with any solid or liquid additive commonly used for formulation purposes.

The formulations contain generally between 0.05 and 99% by weight, 0.01 and 98% by weight, preferably between 0.1 and 95% by weight, more preferably between 0.5 and 90% of active ingredient, most preferably between 10 and 70 per cent by weight.

The formulations described above can be used for controlling unwanted microorganisms, in which the formulations comprising compounds of the formula (I) are applied to the microorganisms and/or in their habitat.

*Mixtures*

Compound combinations according to the invention can be used as such or in formulations thereof and can be mixed with known bactericides, acaricides, nematicides or insecticides, in order thus to broaden, for example, the activity spectrum or to prevent development of resistance.

Useful mixing partners include, for example, known insecticides, acaricides, nematicides or else bactericides (see also Pesticide Manual, 14th ed.).

A mixture with other known active ingredients, such as herbicides, or with fertilizers and growth regulators, safeners and/or semiochemicals, is also possible.

*Seed Treatment*

The invention furthermore includes a method for treating seed.

A further aspect of the present invention relates in particular to seeds (dormant, primed, pregerminated or even with emerged roots and leaves) coated with a compound combination or formulation according to the invention. The inventive seeds are used in methods for protection of seeds and emerged plants from the seeds from phytopathogenic harmful fungi.

The compound combination and formulation according to the invention are also suitable for the treatment of seeds and young seedlings. A large part of the damage to crop plants caused by harmful organisms is triggered by the infection of the seeds before sowing or after germination of the plant. This phase is particularly critical since the roots and shoots of the growing plant are particularly sensitive, and even small damage may result in the death of the plant. Accordingly, there is great interest in protecting the seed and the germinating plant by using appropriate formulation.

It is also desirable to optimize the amount of the active ingredient used so as to provide the best possible protection for the seeds, the germinating plants and emerged seedlings from attack by phytopathogenic fungi, but without damaging the plants themselves by the active ingredient used. In particular, methods for the treatment of seed should also take into consideration the intrinsic phenotypes of transgenic plants in order to achieve optimum protection of the seed and the germinating plant with a minimum of crop protection formulations being employed.

The present invention therefore also relates to a method for protecting seeds, germinating plants and emerged seedlings against attack by animal pests and/or phytopathogenic harmful microorganisms by treating the seeds with an inventive combination or formulation. The invention also relates to the use of the combinations or formulation according to the invention for treating seeds for protecting the seeds, the germinating plants and emerged seedlings against animal pests and/or phytopathogenic microorganisms. The invention further relates to seeds which have been coated with an inventive combination or formulation for protection from animal pests and/or phytopathogenic microorganisms.

One of the advantages of the present invention is that the treatment of the seeds with these formulations not only protects the seed itself, but also the resulting plants after emergence, from animal pests and/or phytopathogenic harmful microorganisms. In this way, the immediate treatment of the crop at the time of sowing or shortly thereafter protect plants as well as seed treatment in prior to sowing. It is likewise considered to be advantageous that the inventive active ingredients combination or formulation can be used especially also for transgenic seed, in which case the plant which grows from this seed is capable of expressing a protein which acts against pests, herbicidal damage or abiotic stress. The treatment of such seeds with the inventive active ingredients or formulation, for example an insecticidal protein, can result in control of certain pests.

The compound combination and formulation according to the invention are suitable for protection of seed of any plant variety which is used in agriculture, in the greenhouse, in forests or in horticulture. More particularly, the seed is that of cereals (such as wheat, barley, rye, millet and oats), oilseed rape, maize, cotton, soybeen, rice, potatoes, sunflower, beans, coffee, beet (e.g. sugar beet and fodder beet), peanut, vegetables (such as tomato, cucumber, onions and lettuce), lawns and ornamental plants. Of particular significance is the treatment of the seed of wheat, soybean, oilseed rape, maize and rice.

As also described below, the treatment of transgenic seed with the inventive active ingredients or formulations is of particular significance. This refers to the seed of plants containing at least one heterologous gene which allows the expression of a polypeptide or protein, e.g. having insecticidal properties. These heterologous genes in transgenic seeds

may originate, for example, from microorganisms of the species Bacillus, Rhizobium, Pseudomonas, Serratia, Trichoderma, Clavibacter, Glomus or Gliocladium. These heterologous genes preferably originate from Bacillus sp., in which case the gene product is effective against the European corn borer and/or the Western corn rootworm. Particularly preferably, the heterologous genes originate from Bacillus thuringiensis.

Preferably, the seed is treated in a state in which it is sufficiently stable for no damage to occur in the course of treatment. In general, seeds can be treated at any time between harvest and some time after sowing. It is customary to use seed which has been separated from the plant and freed from cobs, shells, stalks, coats, hairs or the flesh of the fruits. For example, it is possible to use seed which has been harvested, cleaned and dried down to a moisture content of less than 15% by weight. Alternatively, it is also possible to use seed which, after drying, for example, has been treated with water and then dried again, or seeds just after priming, or seeds stored in primed conditions or pre-germinated seeds, or seeds sown on nursery trays, tapes or paper.

When treating the seeds, it generally has to be ensured that the amount of the inventive combination or formulation applied to the seed and/or the amount of further additives is selected such that the germination of the seed is not impaired, or that the resulting plant is not damaged. This must be ensured particularly in the case of active ingredients which can exhibit phytotoxic effects at certain application rates.

The compound combination or formulation according to the invention can be applied directly, i.e. without containing any other components and without having been diluted. In general, it is preferable to apply the formulation to the seed. The compound combination according to the invention can be converted to the customary seed dressing formulation relevant to on-seed applications, such as solutions, emulsions, suspensions, powders, foams, slurries or combined with other coating formulations for seed, such as film forming materials, pelleting materials, fine iron or other metal powders, granules, coating material for inactivated seeds, and also ULV formulations.

These seed dressing formulations are prepared in a known manner, by mixing the active ingredients or active ingredient combinations with customary additives, for example customary extenders and solvents or diluents, dyes, wetting agents, dispersants, emulsifiers, antifoams, preservatives, secondary thickeners, adhesives, gibberellins, and also water.

Useful dyes which may be present in the seed dressing formulations usable in accordance with the invention are all dyes which are customary for such purposes. It is possible to use either pigments, which are sparingly soluble in water, or dyes, which are soluble in water. Examples include the dyes known by the names Rhodamine B, C.I. Pigment Red 112 and C.I. Solvent Red 1.

Useful wetting agents which may be present in the seed dressing formulations usable in accordance with the invention are all substances which promote wetting and which are conventionally used for the formulation of active agrochemical ingredients. Usable with preference are alkylnaphthalenesulphonates, such as diisopropyl- or diisobutylnaphthalenesulphonates.

Useful dispersants and/or emulsifiers which may be present in the seed dressing formulations usable in accordance with the invention are all nonionic, anionic and cationic dispersants conventionally used for the formulation of active agrochemical ingredients. Usable with preference are nonionic or anionic dispersants or mixtures of nonionic or anionic dispersants. Useful nonionic dispersants include especially ethylene oxide/propylene oxide block polymers, alkylphenol polyglycol ethers and tristryrylphenol polyglycol ether, and the phosphated or sulphated derivatives thereof. Suitable anionic dispersants are especially lignosulphonates, polyacrylic acid salts and arylsulphonate/formaldehyde condensates.

Antifoams which may be present in the seed dressing formulations usable in accordance with the invention are all foam-inhibiting substances conventionally used for the formulation of active agrochemical ingredients. Silicone antifoams and magnesium stearate can be used with preference.

Preservatives which may be present in the seed dressing formulations usable in accordance with the invention are all substances usable for such purposes in agrochemical formulations. Examples include dichlorophene and benzyl alcohol hemiformal.

Secondary thickeners which may be present in the seed dressing formulations usable in accordance with the invention are all substances usable for such purposes in agrochemical formulations. Preferred examples include cellulose derivatives, acrylic acid derivatives, xanthan, modified clays and finely divided silica.

Adhesives which may be present in the seed dressing formulations usable in accordance with the invention are all customary binders usable in seed dressing products. Preferred examples include polyvinylpyrrolidone, polyvinyl acetate, polyvinyl alcohol and tylose.

The seed dressing formulations can be used to treat a wide variety of different kinds of seed either directly or after prior dilution with water. For instance, the concentrates or the preparations obtainable therefrom by dilution with water can be used to dress the seed of cereals, such as wheat, barley, rye, oats, and triticale, and also seeds of maize, soybean, rice, oilseed rape, peas, beans, cotton, sunflowers, and beets, or else a wide variety of different vegetable seeds. The formulations or the dilute preparations thereof, can also be used for seeds of transgenic plants. In this case, additional synergistic effects may also occur in interaction with the substances formed by expression.

For treatment of seeds with the seed dressing formulation or the preparations prepared therefrom by adding water, all mixing units usable customarily for on-seed applications are useful. Specifically, the procedure in on-seed applications is to place the seeds into a mixer, to add the particular desired amount of the seed dressing formulations, either as such or after prior dilution with water, and to mix everything until all applied formulations are distributed homogeneously on the seeds. If appropriate, this is followed by a drying operation.

The application rate of the seed dressing formulations usable in accordance with the invention can be varied within a relatively wide range. It is guided by the particular content of the active ingredients in the formulations and by the seeds. The application rate of each single active ingredient is generally between 0.001 and 15 g per kilogram of seed, preferably between 0.01 and 5 g per kilogram of seed.

*Antimycotic Effects*

In addition, the compound combination and formulation according to the invention also have very good antimycotic effects. They have a very broad antimycotic activity spectrum, especially against dermatophytes and yeasts, moulds and diphasic fungi (for example against Candida species, such as Candida albicans, Candida glabrata), and Epidermophyton floccosum, Aspergillus species, such as Aspergillus niger and Aspergillus fumigatus, Trichophyton species, such as Trichophyton mentagrophytes, Microsporon species such as Microsporon canis and audouinii. The enumeration of these fungi by no means constitutes a restriction of the mycotic spectrum covered, and is merely of illustrative character.

The compound combination and formulation according to the invention can be used also to control important fungal pathogens in fish and crustacea farming, e.g. saprolegnia diclina in trouts, saprolegnia parasitica in crayfish.

The compound combination and formulation according to the invention can therefore be used both in medical and in non-medical applications.

The compound combination and formulation according to the invention can be used as such or the use forms prepared therefrom, such as ready-to-use solutions, suspensions, wettable powders, pastes, soluble powders, dusts and granules. Application is accomplished in a customary manner, for example by watering, spraying, atomizing, broadcasting, dusting, foaming, spreading-on and the like. It is also possible to deploy them by the ultra-low volume method or to inject them into the soil. It is also possible to treat the seed of the plants.

*GMO*

As already mentioned above, it is possible to treat all plants and their parts in accordance with the invention. In a preferred embodiment, wild plant species and plant cultivars, or those obtained by conventional biological breeding methods, such as crossing or protoplast fusion, and also parts thereof, are treated. In a further preferred embodiment, transgenic plants and plant cultivars obtained by genetic engineering methods, if appropriate in combination with conventional methods (Genetically Modified Organisms), and parts thereof are treated. The terms "parts" or "parts of plants" or "plant parts" have been explained above. More preferably, plants of the plant cultivars which are commercially available or are in use are treated in accordance with the invention. Plant cultivars are understood to mean plants which have new properties ("traits") and have been obtained by conventional breeding, by mutagenesis or by recombinant DNA techniques. They can be cultivars, varieties, bio- or genotypes.

The method of treatment according to the invention can be used in the treatment of genetically modified organisms (GMOs), e.g. plants or seeds. Genetically modified plants (or transgenic plants) are plants of which a heterologous gene has been stably integrated into genome. The expression "heterologous gene" essentially means a gene which is provided or assembled outside the plant and when introduced in the nuclear, chloroplastic or mitochondrial genome gives the transformed plant new or improved agronomic or other properties by expressing a protein or polypeptide of interest or by downregulating or silencing other gene(s) which are present in the plant (using for example, antisense technology, cosuppression technology, RNA interference - RNAi - technology or microRNA - miRNA - technology). A heterologous gene that is located in the genome is also called a transgene. A transgene that is defined by its particular location in the plant genome is called a transformation or transgenic event.

Plants and plant cultivars which are preferably to be treated according to the invention include all plants which have genetic material which impart particularly advantageous, useful traits to these plants (whether obtained by breeding and/or biotechnological means).

Plants and plant cultivars which are also preferably to be treated according to the invention are resistant against one or more biotic stresses, i.e. said plants show a better defense against animal and microbial pests, such as against nematodes, insects, mites, phytopathogenic fungi, bacteria, viruses and/or viroids.

Plants and plant cultivars which may also be treated according to the invention are those plants which are resistant to one or more abiotic stresses. Abiotic stress conditions may include, for example, drought, cold temperature exposure, heat exposure, osmotic stress, flooding, increased soil salinity, increased mineral exposure, ozone exposure, high light exposure, limited availability of nitrogen nutrients, limited availability of phosphorus nutrients, shade avoidance.

Plants and plant cultivars which may also be treated according to the invention, are those plants characterized by enhanced yield characteristics. Increased yield in said plants can be the result of, for example, improved plant physiology, growth and development, such as water use efficiency, water retention efficiency, improved nitrogen use, enhanced carbon assimilation, improved photosynthesis, increased germination efficiency and accelerated maturation. Yield can furthermore be affected by improved plant architecture (under stress and non-stress conditions), including but not limited to, early flowering, flowering control for hybrid seed production, seedling vigor, plant size, internode number and distance, root growth, seed size, fruit size, pod size, pod or ear number, seed number per pod or ear, seed mass, enhanced seed filling, reduced seed dispersal, reduced pod dehiscence and lodging resistance. Further yield traits include seed formulation, such as carbohydrate content and formulation for example cotton or starch, protein content, oil content and formulation, nutritional value, reduction in anti-nutritional compounds, improved processability and better storage stability.

Plants that may be treated according to the invention are hybrid plants that already express the characteristic of heterosis or hybrid vigor which results in generally higher yield, vigor, health and resistance towards biotic and abiotic stresses).

Plants or plant cultivars (obtained by plant biotechnology methods such as genetic engineering) which may be treated according to the invention are herbicide-tolerant plants, i.e. plants made tolerant to one or more given herbicides. Such plants can be obtained either by genetic transformation, or by selection of plants containing a mutation imparting such herbicide tolerance.

Plants or plant cultivars (obtained by plant biotechnology methods such as genetic engineering) which may also be treated according to the invention are insect-resistant transgenic plants, i.e. plants made resistant to attack by certain target insects. Such plants can be obtained by genetic transformation, or by selection of plants containing a mutation imparting such insect resistance.

Plants or plant cultivars (obtained by plant biotechnology methods such as genetic engineering) which may also be treated according to the invention are tolerant to abiotic stresses. Such plants can be obtained by genetic transformation, or by selection of plants containing a mutation imparting such stress resistance.

Plants or plant cultivars (obtained by plant biotechnology methods such as genetic engineering) which may also be treated according to the invention show altered quantity, quality and/or storage-stability of the harvested product and/or altered properties of specific ingredients of the harvested product.

Plants or plant cultivars (that can be obtained by plant biotechnology methods such as genetic engineering) which may also be treated according to the invention are plants, such as cotton plants, with altered fiber characteristics. Such plants can be obtained by genetic transformation, or by selection of plants contain a mutation imparting such altered fiber characteristics.

Plants or plant cultivars (that can be obtained by plant biotechnology methods such as genetic engineering) which may also be treated according to the invention are plants, such as oilseed rape or related Brassica plants, with altered oil profile characteristics. Such plants can be obtained by genetic transformation, or by selection of plants contain a mutation imparting such altered oil profile characteristics.

Plants or plant cultivars (that can be obtained by plant biotechnology methods such as genetic engineering) which may also be treated according to the invention are plants, such as oilseed rape or related Brassica plants, with altered seed shattering characteristics. Such plants can be obtained by genetic transformation, or by selection of plants contain a mutation imparting such altered seed shattering characteristics and include plants such as oilseed rape plants with delayed or reduced seed shattering.

Plants or plant cultivars (that can be obtained by plant biotechnology methods such as genetic engineering) which may also be treated according to the invention are plants, such as Tobacco plants, with altered post-translational protein modification patterns.

*Application Rates*

When using the compound combination or formulation according to the invention as fungicides, the application rates can be varied within a relatively wide range, depending on the kind of application. The application rate is
in the case of treatment of plant parts, for example leaves: from 0.1 to 10 000 g/ha, preferably from 10 to 1000 g/ha, more preferably from 50 to 300 g/ha (in the case of application by watering or dripping, it is even possible to reduce the application rate, especially when inert substrates such as rockwool or perlite are used);
in the case of seed treatment: from 0.1 to 200 g per 100 kg of seed, preferably from 1 to 150 g per 100 kg of seed, more preferably from 2.5 to 25 g per 100 kg of seed, even more preferably from 2.5 to 12.5 g per 100 kg of seed;
in the case of soil treatment: from 0.1 to 10 000 g/ha, preferably from 1 to 5000 g/ha,
wherein the given amounts refer to the total amount of active ingredient in the respective combination or formulation.

These application rates are merely by way of example and are not limiting for the purposes of the invention.

The invention is illustrated by the examples below. However, the invention is not limited to the examples.

Examples

The compounds of formula (I) are know (i.e. WO 2013/071169 A1, WO2017/091602, WO2017/091617 and WO2017/091627). Further WO 2018/161008 A1 discloses processes to make such compounds.

Scheme 1 below illustrates a synthetic route for the compound of formula I.04. Further details with regard to this synthetic route are provided in WO 2018/161008 A1.

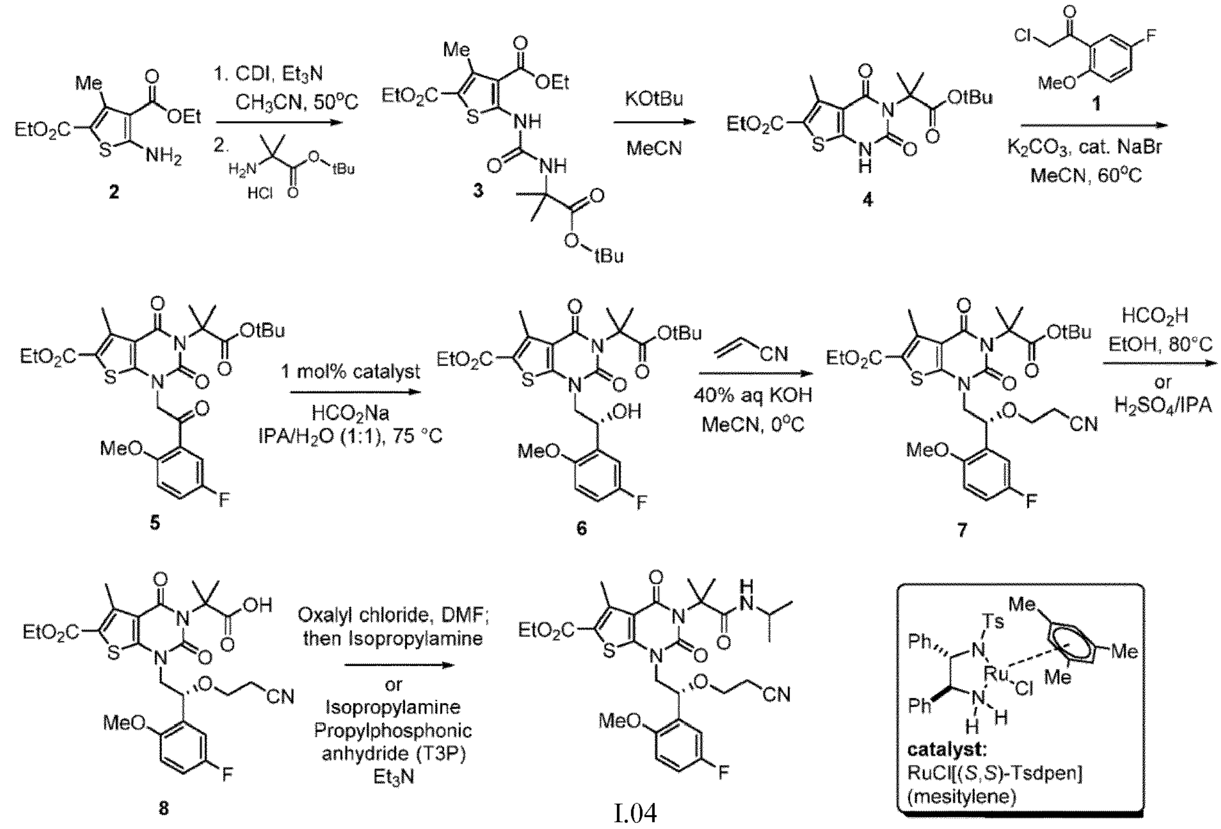


2, 3, 4, 1, 5, 6, 7, 8, I.04

**Scheme 1: Synthetic route to I.04**

Scheme 2 below illustrates a synthetic route for the compound of formula I.04. Further details with regard to this synthetic route are also provided in WO 2018/161008 A1.

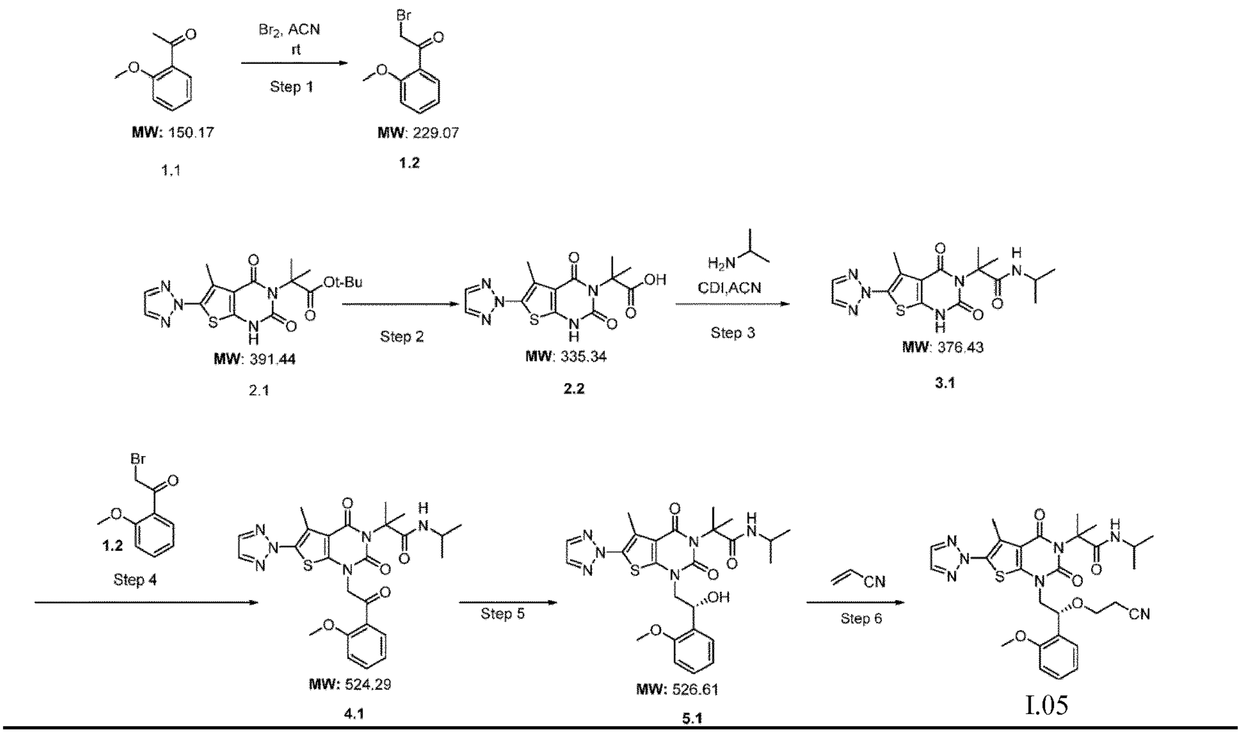


**Scheme 2: Synthetic route to I.05**

## Biological examples

The active compound combinations according to the invention show an advanced fungicidal activity. While the individual active compounds exhibit weaknesses with regard to the fungicidal activity, the combinations have an activity which exceeds a simple addition of activities.

A synergistic effect of fungicides is always present when the fungicidal activity of the active compound combinations exceeds the total of the activities of the active compounds when applied individually. The expected activity for a given combination of two active compounds can be calculated as follows (cf. Colby, S.R., "Calculating Synergistic and Antagonistic Responses of Herbicide Combinations", Weeds 1967, 15, 20-22):

If

X is the efficacy when active compound A is applied at an application rate of m ppm (or g/ha),

Y is the efficacy when active compound B is applied at an application rate of n ppm (or g/ha),

E is the efficacy when the active compounds A and B are applied at application rates of m and n ppm (or g/ha), respectively, and

then

$$E = X + Y - \frac{X \cdot Y}{100}$$

The degree of efficacy, expressed in % is denoted. 0 % means an efficacy which corresponds to that of the control while an efficacy of 100 % means that no disease is observed.

If the actual fungicidal activity exceeds the calculated value, then the activity of the combination is superadditive, i.e. a synergistic effect exists. In this case, the efficacy which was actually observed must be greater than the value for the expected efficacy (E) calculated from the abovementioned formula.

A further way of demonstrating a synergistic effect is the method of Tammes (cf. "Isoboles, a graphic representation of synergism in pesticides" in Neth. J. Plant Path., 1964, 70, 73-80).

## Claims

1. Active compound combination comprising

(A) at least one compound of formula (I)

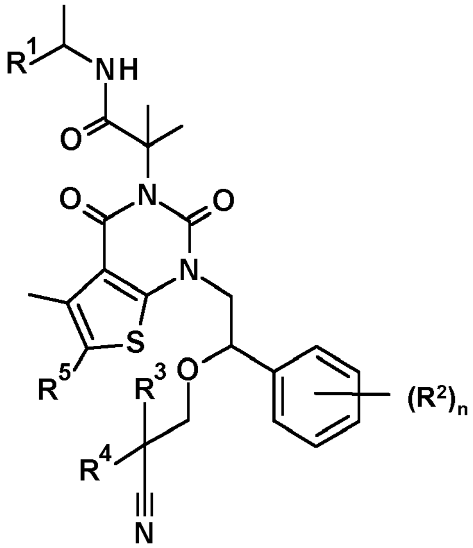

(I),

wherein

$R^1$ represents hydrogen or $-CH_3$;
$R^2$ represents independently of one another $-OCH_3$ or F,
$R^3$, $R^4$ represents independently of one another hydrogen or $-CH_3$;
$R^5$ represents either

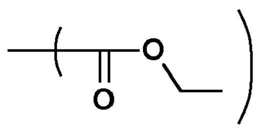

or

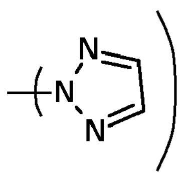

n is an integer and is 0, 1 or 2;

or a salt or N-oxide thereof,
and
(B) at least one further active compound selected from the following groups

(1) inhibitors of the ergosterol synthesis,
(2) inhibitors of the respiratory chain at complex I or II,

(3) inhibitors of the respiratory chain at complex III,
(4) inhibitors of the mitosis and cell division,
(5) compounds capable of having a multisite action,
(6) compounds capable of inducing a host defense,
(7) inhibitors of the amino acid and/or protein biosynthesis,
(8) inhibitors of the ATP production,
(9) inhibitors of the cell wall synthesis,
(10) inhibitors of the lipid and membrane synthesis,
(11) inhibitors of the melanine biosynthesis,
(12) inhibitors of the nucleic acid synthesis,
(13) inhibitors of the signal transduction,
(14) compounds capable of acting as uncoupler,
(15) other fungicides.

**2.** Active compound combination according to claim 1 comprising at least (A) one compound of formula (I.01)

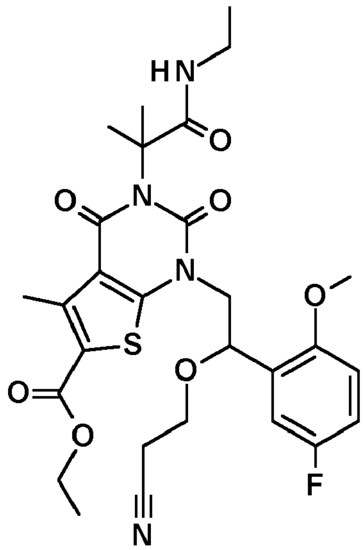

(I.01)

or a salt or N-oxide thereof.

**3.** Active compound combination according to claim 1 comprising at least (B) one compound of formula (I.02)

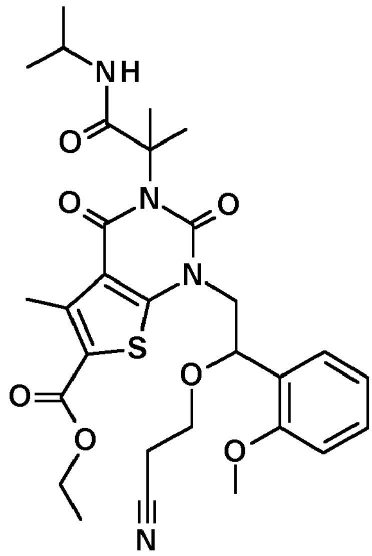

(I.02)

or a salt or N-oxide thereof.

4. Active compound combination according to claim 1 comprising at least (C) one compound of formula (I.03)

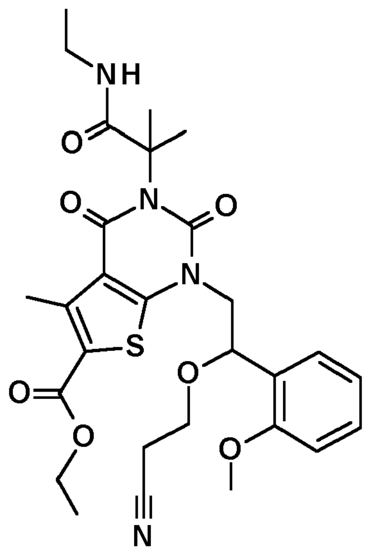

(I.03)

or a salt or N-oxide thereof.

5. Active compound combination according to claim 1 comprising at least (D) one compound of formula (I.04)

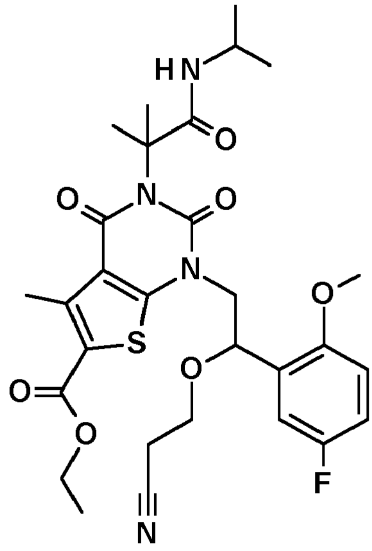

(I.04)

or a salt or N-oxide thereof.

6. Active compound combination according to claim 1 comprising at least (E) one compound of formula (I.05)

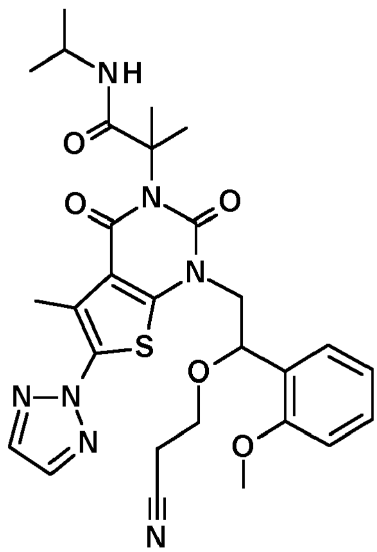

(I.05)

or a salt or N-oxide thereof.

7. Active compound combination according to claim 1 comprising at least (F) one compound of formula (I.06)

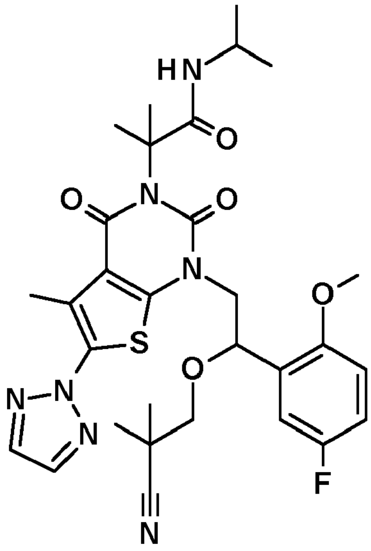

(I.06)

or a salt or N-oxide thereof.

**8.** Active compound combination according to at least one of claims 1 to 7, wherein the further active compound is selected from the group consisting of (1.001) cyproconazole, (1.002) difenoconazole, (1.003) epoxiconazole, (1.004) fenhexamid, (1.005) fenpropidin, (1.006) fenpropimorph, (1.007) fenpyrazamine, (1.008) fluquinconazole, (1.009) flutriafol, (1.010) imazalil, (1.011) imazalil sulfate, (1.012) ipconazole, (1.013) metconazole, (1.014) myclobutanil, (1.015) paclobutrazol, (1.016) prochloraz, (1.017) propiconazole, (1.018) prothioconazole, (1.019) Pyrisoxazole, (1.020) spiroxamine, (1.021) tebuconazole, (1.022) tetraconazole, (1.023) triadimenol, (1.024) tridemorph, (1.025) triticonazole, (1.026) (1R,2S,5S)-5-(4-chlorobenzyl)-2-(chloromethyl)-2-methyl-1-(1H-1,2,4-triazol-1-ylmethyl)cyclopentanol, (1.027) (1S,2R,5R)-5-(4-chlorobenzyl)-2-(chloromethyl)-2-methyl-1-(1H-1,2,4-triazol-1-ylmethyl)cyclopentanol, (1.028) (2R)-2-(1-chlorocyclopropyl)-4-[(1R)-2,2-dichlorocyclopropyl]-1-(1H-1,2,4-triazol-1-yl)butan-2-ol, (1.029) (2R)-2-(1-chlorocyclopropyl)-4-[(1S)-2,2-dichlorocyclopropyl]-1-(1H-1,2,4-triazol-1-yl)butan-2-ol, (1.030) (2R)-2-[4-(4-chlorophenoxy)-2-(trifluoromethyl)phenyl]-1-(1H-1,2,4-triazol-1-yl)propan-2-ol, (1.031) (2S)-2-(1-chlorocyclopropyl)-4-[(1R)-2,2-dichlorocyclopropyl]-1-(1H-1,2,4-triazol-1-yl)butan-2-ol, (1.032) (2S)-2-(1-chlorocyclopropyl)-4-[(1S)-2,2-dichlorocyclopropyl]-1-(1H-1,2,4-triazol-1-yl)butan-2-ol, (1.033) (2S)-2-[4-(4-chlorophenoxy)-2-(trifluoromethyl)phenyl]-1-(1H-1,2,4-triazol-1-yl)propan-2-ol, (1.034) (R)-[3-(4-chloro-2-fluorophenyl)-5-(2,4-difluorophenyl)-1,2-oxazol-4-yl](pyridin-3-yl)methanol, (1.035) (S)-[3-(4-chloro-2-fluorophenyl)-5-(2,4-difluorophenyl)-1,2-oxazol-4-yl](pyridin-3-yl)methanol, (1.036) [3-(4-chloro-2-fluorophenyl)-5-(2,4-difluorophenyl)-1,2-oxazol-4-yl](pyridin-3-yl)methanol, (1.037) 1-({(2R,4S)-2-[2-chloro-4-(4-chlorophenoxy)phenyl]-4-methyl-1,3-dioxolan-2-yl}methyl)-1H-1,2,4-triazole, (1.038) 1-({(2S,4S)-2-[2-chloro-4-(4-chlorophenoxy)phenyl]-4-methyl-1,3-dioxolan-2-yl}methyl)-1H-1,2,4-triazole, (1.039) 1-{[3-(2-chlorophenyl)-2-(2,4-difluorophenyl)oxiran-2-yl]methyl}-1H-1,2,4-triazol-5-yl thiocyanate, (1.040) 1-{[rel(2R,3R)-3-(2-chlorophenyl)-2-(2,4-difluorophenyl)oxiran-2-yl]methyl} -1H-1,2,4-triazol-5-yl thiocyanate, (1.041) 1-{[rel(2R,3S)-3-(2-chlorophenyl)-2-(2,4-difluorophenyl)oxiran-2-yl]methyl}-1H-1,2,4-triazol-5-yl thiocyanate, (1.042) 2-[(2R,4R,5R)-1-(2,4-dichlorophenyl)-5-hydroxy-2,6,6-trimethylheptan-4-yl]-2,4-dihydro-3H-1,2,4-triazole-3-thione, (1.043) 2-[(2R,4R,5S)-1-(2,4-dichlorophenyl)-5-hydroxy-2,6,6-trimethylheptan-4-yl]-2,4-dihydro-3H-1,2,4-triazole-3-thione, (1.044) 2-[(2R,4S,5R)-1-(2,4-dichlorophenyl)-5-hydroxy-2,6,6-trimethylheptan-4-yl]-2,4-dihydro-3H-1,2,4-triazole-3-thione, (1.045) 2-[(2R,4S,5S)-1-(2,4-dichlorophenyl)-5-hydroxy-2,6,6-trimethylheptan-4-yl]-2,4-dihydro-3H-1,2,4-triazole-3-thione, (1.046) 2-[(2S,4R,5R)-1-(2,4-dichlorophenyl)-5-hydroxy-2,6,6-trimethylheptan-4-yl]-2,4-dihydro-3H-1,2,4-triazole-3-thione, (1.047) 2-[(2S,4R,5S)-1-(2,4-dichlorophenyl)-5-hydroxy-2,6,6-trimethylheptan-4-yl]-2,4-dihydro-3H-1,2,4-triazole-3-thione, (1.048) 2-[(2S,4S,5R)-1-(2,4-dichlorophenyl)-5-hydroxy-2,6,6-trimethylheptan-4-yl]-2,4-dihydro-3H-1,2,4-triazole-3-thione, (1.049) 2-[(2S,4S,5S)-1-(2,4-dichlorophenyl)-5-hydroxy-2,6,6-trimethylheptan-4-yl]-2,4-dihydro-3H-1,2,4-triazole-3-thione, (1.050) 2-[1-(2,4-dichlorophenyl)-5-hydroxy-2,6,6-trimethylheptan-4-yl]-2,4-dihydro-3H-1,2,4-triazole-3-thione, (1.051) 2-[2-chloro-4-(2,4-dichlorophenoxy)phenyl]-1-(1H-1,2,4-triazol-1-yl)propan-2-ol, (1.052) 2-[2-chloro-4-(4-chlorophenoxy)phenyl]-1-(1H-1,2,4-triazol-1-yl)butan-2-ol, (1.053) 2-[4-(4-chlorophenoxy)-2-(trifluoromethyl)phenyl]-1-(1H-1,2,4-triazol-1-yl)butan-2-ol, (1.054) 2-[4-(4-chlorophenoxy)-2-(trifluoromethyl)phenyl]-1-(1H-1,2,4-triazol-1-yl)pentan-2-ol, (1.055) 2-[4-(4-chlorophenoxy)-2-(trifluoromethyl)phenyl]-1-(1H-1,2,4-triazol-1-yl)propan-2-ol, (1.056) 2-{[3-(2-chlorophenyl)-2-(2,4-difluorophenyl)oxiran-2-yl]methyl}-2,4-dihydro-3H-1,2,4-triazole-3-thione, (1.057) 2- {[rel(2R,3R)-3-(2-chlorophenyl)-2-(2,4-difluorophenyl)oxiran-2-yl]methyl}-2,4-dihydro-3H-1,2,4-triazole-3-thione, (1.058) 2-{[rel(2R,3S)-3-(2-chlorophenyl)-2-(2,4-difluorophenyl)oxiran-2-yl]methyl}-2,4-dihydro-3H-1,2,4-triazole-3-thione, (1.059) 5-(4-chlorobenzyl)-2-(chloromethyl)-2-methyl-1-(1H-1,2,4-triazol-1-ylmethyl)cyclopentanol, (1.060) 5-(allylsulfanyl)-1-{[3-(2-chlorophenyl)-2-(2,4-diauorophenyl)oxiran-2-yl]methyl}-1H-1,2,4-triazole, (1.061) 5-(allylsulfanyl)-1-{[rel(2R,3R)-3-(2-chlorophenyl)-2-(2,4-difluorophenyl)oxiran-2-yl]methyl}-1H-1,2,4-triazole, (1.062) 5-(allylsulfanyl)-1-{[rel(2R,3S)-3-(2-chlorophenyl)-2-(2,4-difluorophenyl)oxiran-2-yl]methyl}-1H-1,2,4-triazole, (1.063) N'-(2,5-dimethyl-4-{[3-(1,1,2,2-tetrafluoroethoxy)phenyl]sulfanyl}phenyl)-N-ethyl-N-methylimidoformamide, (1.064) N'-(2,5-dimethyl-4-{[3-(2,2,2-trifluoroethoxy)phenyl]sulfanyl}phenyl)-N-ethyl-N-methylimidoformamide, (1.065) N'-(2,5-dimethyl-4-{[3-(2,2,3,3-tetrafluoropropoxy)phenyl]sulfanyl}phenyl)-N-ethyl-N-methylimidoformamide, (1.066) N'-(2,5-dimethyl-4-{[3-(pentafluoroethoxy)phenyl]sulfanyl}phenyl)-N-ethyl-N-methylimidoformamide, (1.067) N'-(2,5-dimethyl-4-{3-[(1,1,2,2-tetrafluoroethyl)sulfanyl]phenoxy}phenyl)-N-ethyl-N-methylimidoformamide, (1.068) N'-(2,5-dimethyl-4- {3-[(2,2,2-trifluoroethyl)sulfanyl]phenoxy}phenyl)-N-ethyl-N-methylimidoformamide, (1.069) N'-(2,5-dimethyl-4-{3-[(2,2,3,3-tetrafluoropropyl)sulfanyl]phenoxy}phenyl)-N-ethyl-N-methylimidoformamide, (1.070) N'-(2,5-dimethyl-4- {3-[(pentafluoroethyl)sulfanyl]phenoxy}phenyl)-N-ethyl-N-methylimidoformamide, (1.071) N'-(2,5-dimethyl-4-phenoxyphenyl)-N-ethyl-N-methylimidoformamide, (1.072) N'-(4- {[3-(difluoromethoxy)phenyl]sulfanyl}-2,5-dimethylphenyl)-N-ethyl-N-methylimidoformamide, (1.073) N'-(4-{3-[(difluoromethyl)sulfanyl]phenoxy}-2,5-dimethylphenyl)-N-ethyl-N-methylimidoformamide, (1.074) N'-[5-bromo-6-(2,3-dihydro-1H-inden-2-yloxy)-2-methylpyridin-3-yl]-N-ethyl-N-methylimidoformamide, (1.075) N'-{4-[(4,5-dichloro-1,3-thiazol-2-yl)oxy]-2,5-dimethylphenyl}-N-ethyl-N-methylimidoformamide, (1.076) N'-{5-bromo-6-[(1R)-1-(3,5-difluorophenyl)ethoxy]-2-methylpyridin-3-yl} -N-ethyl-N-methylimidoformamide, (1.077) N'-{5-bromo-6-[(1S)-1-(3,5-difluorophenyl)ethoxy]-2-methylpyridm-3-yl}-N-ethyl-N-methylimidoformamide, (1.078) N'- {5-bromo-6-[(cis-4-isopropylcyclohexyl)oxy]-2-methylpyridin-3-yl}-N-ethyl-N-methylimidoformamide, (1.079) N'-{5-bromo-6-[(trans-4-isopropylcyclohexyl)oxy]-2-methylpyridin-3-yl}-N-ethyl-N-methylimidoformamide, (1.080) N'-{5-bromo-6-[1-(3,5-difluorophenyl)ethoxy]-2-methylpyridin-3-yl}-N-ethyl-N-methylimidoformamide, (1.081) Mefentrifluconazole, (1.082) Ipfentrifluconazole, (1.083) 2-[6-(4-bromophenoxy)-2-(trifluoromethyl)-3 -pyridyl] -1 -(1,2,4-triazol-1 -yl)propan-2-ol, (2.001) benzovindiflupyr, (2.002) bixafen, (2.003) boscalid, (2.004) carboxin, (2.005) fluopyram, (2.006) flutolanil, (2.007) fluxapyroxad, (2.008) furametpyr, (2.009) Isofetamid, (2.010) isopyrazam (anti-epimeric enantiomer 1R,4S,9S), (2.011) isopyrazam (anti-epimeric enantiomer 1S,4R,9R), (2.012) isopyrazam (anti-epimeric racemate 1RS,4SR,9SR), (2.013) isopyrazam (mixture of syn-epimeric racemate 1RS,4SR,9RS and anti-epimeric racemate 1RS,4SR,9SR), (2.014) isopyrazam (syn-epimeric enantiomer 1R,4S,9R), (2.015) isopyrazam (syn-epimeric enantiomer 1S,4R,9S), (2.016) isopyrazam (syn-epimeric racemate 1RS,4SR,9RS), (2.017) penflufen, (2.018) penthiopyrad, (2.019) pydiflumetofen, (2.020) Pyraziflumid, (2.021) sedaxane, (2.022) 1,3-dimethyl-N-(1,1,3-trimethyl-2,3-dihydro-1H-inden-4-yl)-1H-pyrazole-4-carboxamide, (2.023) 1,3-dimethyl-N-[(3R)-1,1,3-trimethyl-2,3-dihydro-1H-inden-4-yl]-1H-pyrazole-4-carboxamide, (2.024) 1,3-dimethyl-N-[(3S)-1,1,3-trimethyl-2,3-dihydro-1H-inden-4-yl]-1H-pyrazole-4-carboxamide, (2.025) 1-methyl-3-(trifluoromethyl)-N-[2'-(trifluoromethyl)biphenyl-2-yl]-1H-pyrazole-4-carboxamide, (2.026) 2-fluoro-6-(trifluoromethyl)-N-(1,1,3-trimethyl-2,3-dihydro-1H-inden-4-yl)benzamide, (2.027) 3-(difluoromethyl)-1-methyl-N-(1,1,3-trimethyl-2,3-dihydro-1H-inden-4-yl)-1H-pyrazole-4-carboxamide, (2.028) 3-(difluoromethyl)-1-methyl-N-[(3R)-1,1,3-trimethyl-2,3-dihydro-1H-inden-4-yl]-1H-pyrazole-4-carboxamide, (2.029) 3-(difluoromethyl)-1-methyl-N-[(3S)-1,1,3-trimethyl-2,3-dihydro-1H-inden-4-yl]-1H-pyrazole-4-carboxamide, (2.030) 3 -(difluoromethyl)-N-(7-fluoro-1,1,3-trimethyl-2,3-dihydro-1H-inden-4-yl)-1-methyl-1H-pyrazole-4-carboxamide, (2.031) 3-(difluoromethyl)-N-[(3R)-7-fluoro-1,1,3-trimethyl-2,3-dihydro-1H-inden-4-yl]-1-methyl-1H-pyrazole-4-carboxamide, (2.032) 3-(difluoromethyl)-N-[(3S)-7-fluoro-1,1,3-trimethyl-2,3-dihydro-1H-inden-4-yl]-1-methyl-1H-pyrazole-4-carboxamide, (2.033) 5,8-difluoro-N-[2-(2-fluoro-4-{[4-(trifluoromethyl)pyridin-2-yl]oxy}phenyl)ethyl]quinazolin-4-amine, (2.034) N-(2-cyclopentyl-5-fluorobenzyl)-N-cyclopropyl-3-(difluoromethyl)-5-fluoro-1-methyl-1H-pyrazole-4-carboxamide, (2.035) N-(2-tert-butyl-5-methylbenzyl)-N-cyclopropyl-3-(difluoromethyl)-5-fluoro-1-methyl-1H-pyrazole-4-carboxamide, (2.036) N-(2-tert-butylbenzyl)-N-cyclopropyl-3-(difluoromethyl)-5-fluoro-1-methyl-1H-pyrazole-4-carboxamide, (2.037) N-(5-chloro-2-ethylbenzyl)-N-cyclopropyl-3-(difluoromethyl)-5-fluoro-1-methyl-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide, (2.039) N-[(1R,4S)-9-(dichloromethylene)-1,2,3,4-tetrahydro-1,4-methanonaphthalen-5-yl]-3-(difluoromethyl)-1-methyl-1H-pyrazole-4-carboxamide, (2.040) N-[(1S,4R)-9-(dichloromethylene)-1,2,3,4-tetrahydro-1,4-methanonaphthalen-5-yl]-3-(difluoromethyl)-1-methyl-1H-pyrazole-4-carboxamide, (2.041) N-[1-(2,4-dichlorophenyl)-1-methoxypropan-2-yl]-3-(difluoromethyl)-1-methyl-1H-pyrazole-4-carboxamide, (2.042) N-[2-chloro-6-(trifluoromethyl)benzyl]-N-cyclopropyl-3-(difluoromethyl)-5-fluoro-1-methyl-1H-pyrazole-4-carboxamide, (2.043) N-[3-chloro-2-fluoro-6-(trifluoromethyl)benzyl]-N-cyclopropyl-3-(difluoromethyl)-5-fluoro-1-methyl-1H-pyrazole-4-carboxamide, (2.044) N-[5-chloro-2-(trifluoromethyl)benzyl]-N-cyclopropyl-3-(difluoromethyl)-5-fluoro-1-methyl-1H-pyrazole-4-carboxamide, (2.045) N-cyclopropyl-3-(difluoromethyl)-5-fluoro-1-methyl-N-[5-methyl-2-(trifluoromethyl)benzyl]-1H-pyrazole-4-carboxamide, (2.046) N-cyclopropyl-3-(difluoromethyl)-5-fluoro-N-(2-fluoro-6-isopropylbenzyl)-1-methyl-1H-pyrazole-4-carboxamide, (2.047) N-cyclopropyl-3-(difluoromethyl)-5-fluoro-N-(2-isopropyl-5-methylbenzyl)-1-methyl-1H-pyrazole-4-carboxamide, (2.048) N-cyclopropyl-3-(difluoromethyl)-5-fluoro-N-(2-isopropylbenzyl)-1-methyl-1H-pyrazole-4-carbothioamide, (2.049) N-cyclopropyl-3-(difluoromethyl)-5-fluoro-N-(2-isopropylbenzyl)-1 -methyl-1H-pyrazole-4-carboxamide, (2.050) N-cyclopropyl-3-(difluoromethyl)-5-fluoro-N-(5-fluoro-2-isopropylbenzyl)-1-methyl-1H-pyrazole-4-carboxamide, (2.051) N-cyclopropyl-3-(difluoromethyl)-N-(2-ethyl-4,5-dimethylbenzyl)-5-fluoro-1-methyl-1H-pyrazole-4-carboxamide, (2.052) N-cyclopropyl-3-(difluoromethyl)-N-(2-ethyl-5-fluorobenzyl)-5-fluoro-1-methyl-1H-pyrazole-4-carboxamide, (2.053) N-cyclopropyl-3-(difluoromethyl)-N-(2-ethyl-5-methylbenzyl)-5-fluoro-1-methyl-1H-pyrazole-4-carboxamide, (2.054) N-cyclopropyl-N-(2-cyclopropyl-5-fluorobenzyl)-3-(difluoromethyl)-5-fluoro-1-methyl-1H-pyrazole-4-carboxamide, (2.055) N-cyclopropyl-N-(2-cyclopropyl-5-methylbenzyl)-3-(difluoromethyl)-5-fluoro-1-methyl-1H-pyrazole-4-carboxamide, (2.056) N-cyclopropyl-N-(2-cyclopropylbenzyl)-3-(difluoromethyl)-5-fluoro-1-methyl-1H-pyrazole-4-carboxamide, (3.001) ametoctradin, (3.002) amisulbrom, (3.003) azoxystrobin, (3.004) coumethoxystrobin, (3.005) coumoxystrobin, (3.006) cyazofamid, (3.007) dimoxystrobin, (3.008) enoxastrobin, (3.009) famoxadone, (3.010) fenamidone, (3.011) flufenoxystrobin, (3.012) fluoxastrobin, (3.013) kresoxim-methyl, (3.014) metominostrobin, (3.015) orysastrobin, (3.016) picoxystrobin, (3.017) pyraclostrobin, (3.018) pyrametostrobin, (3.019) pyraoxystrobin, (3.020) trifloxystrobin, (3.021) (2E)-2-{2-[({[(1E)-1-(3-{[(E)-1-fluoro-2-phenylvinyl]oxy}phenyl)ethylidene]amino}oxy)methyl]phenyl}-2-(methoxyimino)-N-methylacetamide, (3.022) (2E,3Z)-5-{[1-(4-chlorophenyl)-1H-pyrazol-3-yl]oxy} -2-(methoxyimino)-N,3-dimethylpent-3-enamide, (3.023) (2R)-2- {2-[(2,5-dimethylphenoxy)methyl]phenyl}-2-methoxy-N-methylacetamide, (3.024) (2S)-2-{2-[(2,5-dimethylphenoxy)methyl]phenyl}-2-methoxy-N-methylacetamide, (3.025) (3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-yl 2-methylpropanoate, (3.026) 2-{2-[(2,5-dimethylphenoxy)methyl]phenyl}-2-methoxy-N-methylacetamide, (3.027) N-(3-ethyl-3,5,5-trimethylcyclohexyl)-3-formamido-2-hydroxybenzamide, (3.028) (2E,3Z)-5-{[1-(4-chloro-2-fluorophenyl)-1H-pyrazol-3-yl]oxy}-2-(methoxyimino)-N,3-dimethylpent-3-enamide, (3.029) methyl {5-[3-(2,4-dimethylphenyl)-1H-pyrazol-1-yl]-2-methylbenzyl}carbamate, (3.030) (1S)-2,2-bis(4-fluorophenyl)-1-methylethyl N-{[3-(acetyloxy)-4-methoxy-2-pyridyl]carbonyl}-L-alaninate, (4.001) carbendazim, (4.002) diethofencarb, (4.003) ethaboxam, (4.004) fluopicolide, (4.005) pencycuron, (4.006) thiabendazole, (4.007) thiophanate-methyl, (4.008) zoxamide, (4.009) 3-chloro-4-(2,6-difluorophenyl)-6-methyl-5-phenylpyridazine, (4.010) 3-chloro-5-(4-chlorophenyl)-4-(2,6-difluorophenyl)-6-methylpyridazine, (4.011) 3-chloro-5-(6-chloropyridin-3-yl)-6-methyl-4-(2,4,6-trifluorophenyl)pyridazine, (4.012) 4-(2-bromo-4-fluorophenyl)-N-(2,6-difluorophenyl)-1,3-dimethyl-1H-pyrazol-5-amine, (4.013) 4-(2-bromo-4-fluorophenyl)-N-(2-bromo-6-fluorophenyl)-1,3-dimethyl-1H-pyrazol-5-amine, (4.014) 4-(2-bromo-4-fluorophenyl)-N-(2-bromophenyl)-1,3-dimethyl-1H-pyrazol-5-amine, (4.015) 4-(2-bromo-4-fluorophenyl)-N-(2-chloro-6-fluorophenyl)-1,3-dimethyl-1H-pyrazol-5-amine, (4.016) 4-(2-bromo-4-fluorophenyl)-N-(2-chlorophenyl)-1,3-dimethyl-1H-pyrazol-5-amine, (4.017) 4-(2-bromo-4-fluorophenyl)-N-(2-fluorophenyl)-1,3-dimethyl-1H-pyrazol-5-amine, (4.018) 4-(2-chloro-4-fluorophenyl)-N-(2,6-difluorophenyl)-1,3-dimethyl-1H-pyrazol-5-amine, (4.019) 4-(2-chloro-4-fluorophenyl)-N-(2-chloro-6-fluorophenyl)-1,3-dimethyl-1H-pyrazol-5-amine, (4.020) 4-(2-chloro-4-fluorophenyl)-N-(2-chlorophenyl)-1,3-dimethyl-1H-pyrazol-5-amine, (4.021) 4-(2-chloro-4-fluorophenyl)-N-(2-fluorophenyl)-1,3-dimethyl-1H-pyrazol-5-amine, (4.022) 4-(4-chlorophenyl)-5-(2,6-difluorophenyl)-3,6-dimethylpyridazine, (4.023) N-(2-bromo-6-fluorophenyl)-4-(2-chloro-4-fluorophenyl)-1,3-dimethyl-1H-pyrazol-5-amine, (4.024) N-(2-bromophenyl)-4-(2-chloro-4-fluorophenyl)-1,3-dimethyl-1H-pyrazol-5-amine, (4.025) N-(4-chloro-2,6-difluorophenyl)-4-(2-chloro-4-fluorophenyl)-1,3-dimethyl-1H-pyrazol-5-amine. (5.001) bordeaux mixture, (5.002) captafol, (5.003) captan, (5.004) chlorothalonil, (5.005) copper hydroxide, (5.006) copper naphthenate, (5.007) copper oxide, (5.008) copper oxychloride, (5.009) copper(2+) sulfate, (5.010) dithianon, (5.011) dodine, (5.012) folpet, (5.013) mancozeb, (5.014) maneb, (5.015) metiram, (5.016) metiram zinc, (5.017) oxine-copper, (5.018) propineb, (5.019) sulfur and sulfur preparations including calcium polysulfide, (5.020) thiram, (5.021) zineb, (5.022) ziram, (5.023) 6-ethyl-5,7-dioxo-6,7-dihydro-5H-pyrrolo[3',4':5,6][1,4]dithiino[2,3-c][1,2]thiazole-3-carbonitrile, (6.001) acibenzolar-S-methyl, (6.002) isotianil, (6.003) probenazole, (6.004) tiadinil, (7.001) cyprodinil, (7.002) kasugamycin, (7.003) kasugamycin hydrochloride hydrate, (7.004) oxytetracycline, (7.005) pyrimethanil, (7.006) 3-(5-fluoro-3,3,4,4-tetramethyl-3,4-dihydroisoquinolin-1-yl)quinolone, (8.001) silthiofam, (9.001) benthiavalicarb, (9.002) dimethomorph, (9.003) flumorph, (9.004) iprovalicarb, (9.005) mandipropamid, (9.006) pyrimorph, (9.007) valifenalate, (9.008) (2E)-3-(4-tert-butylphenyl)-3-(2-chloropyridin-4-yl)-1-(morpholin-4-yl)prop-2-en-1-one, (9.009) (2Z)-3-(4-tert-butylphenyl)-3-(2-chloropyridin-4-yl)-1-(morpholin-4-yl)prop-2-en-1-one, (10.001) propamocarb, (10.002) propamocarb hydrochloride, (10.003) tolclofos-methyl, (11.001) tricyclazole, (11.002) 2,2,2-trifluoroethyl {3-methyl-1-[(4-methylbenzoyl)amino]butan-2-yl}carbamate, (12.001) benalaxyl, (12.002) benalaxyl-M (kiralaxyl), (12.003) metalaxyl, (12.004) metalaxyl-M (mefenoxam), (13.001) fludioxonil, (13.002) iprodione, (13.003) procymidone, (13.004) proquinazid, (13.005) quinoxyfen, (13.006) vinclozolin, (14.001) fluazinam, (14.002) meptyldinocap, (15.001) Abscisic acid, (15.002) benthiazole, (15.003) bethoxazin, (15.004) capsimycin, (15.005) carvone, (15.006) chinomethionat, (15.007) cufraneb, (15.008) cyflufenamid, (15.009) cymoxanil, (15.010) cyprosulfamide, (15.011) flutianil, (15.012) fosetyl-aluminium, (15.013) fosetyl-calcium, (15.014) fosetyl-sodium, (15.015) methyl isothiocyanate, (15.016) metrafenone, (15.017) mildiomycin, (15.018) natamycin, (15.019) nickel dimethyldithiocarbamate, (15.020) nitrothal-isopropyl, (15.021) oxamocarb, (15.022) Oxathiapiprolin, (15.023) oxyfenthiin, (15.024) pentachlorophenol and salts, (15.025) phosphorous acid and its salts, (15.026) propamocarb-fosetylate, (15.027) pyriofenone (chlazafenone), (15.028) tebufloquin, (15.029) tecloftalam, (15.030) tolnifanide, (15.031) 1-(4-{4-[(5R)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.032) 1-(4-{4-[(5S)-5-(2,6-difluorophenyl)-4,5-dihydro-1,2-oxazol-3-yl]-1,3-thiazol-2-yl}piperidin-1-yl)-2-[5-methyl-3-(trifluoromethyl)-1H-pyrazol-1-yl]ethanone, (15.033) 2-(6-benzylpyridin-2-yl)quinazoline, (15.034) 2,6-dimethyl-1H,5H-[1,4]dithiino[2,3-c:5,6-c']dipyrrole-1,3,5,7(2H,6H)-tetrone, (15.035) 2-[3,5-bis(difluoromethyl)-1H-pyrazol-1-yl]-1-[4-(4-{5-[2-(prop-2-yn-1-yloxy)phenyl]-4,5-dihydro-1,2-oxazol-3-yl}-1,3-thiazol-2-yl)piperidin-1-yl]ethanone, (15.036) 2-[3,5-bis(difluoromethyl)-1H-pyrazol-1-yl]-1-[4-(4-{5-[2-chloro-6-(prop-2-yn-1-yloxy)phenyl]-4,5-dihydro-1,2-oxazol-3-yl}-1,3-thiazol-2-yl)piperidin-1-yl]ethanone, (15.037) 2-[3,5-bis(difluoromethyl)-1H-pyrazol-1-yl]-1-[4-(4-{5-[2-fluoro-6-(prop-2-yn-1-yloxy)phenyl]-4,5-dihydro-1,2-oxazol-3-yl}-1,3-thiazol-2-yl)piperidin-1-yl]ethanone, (15.038) 2-[6-(3-fluoro-4-methoxyphenyl)-5-methylpyridin-2-yl]quinazoline, (15.039) 2-{(5R)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.040) 2-{(5S)-3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.041) 2-{2-[(7,8-difluoro-2-methylquinolin-3-yl)oxy]-6-fluorophenyl}propan-2-ol, (15.042) 2- {2-fluoro-6-[(8-fluoro-2-methylquinolin-3-yl)oxy]phenyl}propan-2-ol, (15.043) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}-3-chlorophenyl methanesulfonate, (15.044) 2-{3-[2-(1-{[3,5-bis(difluoromethyl)-1H-pyrazol-1-yl]acetyl}piperidin-4-yl)-1,3-thiazol-4-yl]-4,5-dihydro-1,2-oxazol-5-yl}phenyl methanesulfonate, (15.045) 2-phenylphenol and salts, (15.046) 3-(4,4,5-trifluoro-3,3-dimethyl-3,4-dihydroisoquinolin-1-yl)quinoline, (15.047) 3-(4,4-difluoro-3,3-dimethyl-3,4-dihydroisoquinolin-1-yl)quinoline, (15.048) 4-amino-5-fluoropyrimidin-2-ol (tautomeric form: 4-amino-5-fluoropyrimidin-2(1H)-one), (15.049) 4-oxo-4-[(2-phenylethyl)amino]butanoic acid, (15.050) 5-amino-1,3,4-thiadiazole-2-thiol, (15.051) 5-chloro-N'-phenyl-N'-(prop-2-yn-1-yl)thiophene-2-sulfonohydrazide, (15.052) 5-fluoro-2-[(4-fluorobenzyl)oxy]pyrimidin-4-amine, (15.053) 5-fluoro-2-[(4-methylbenzyl)oxy]pyrimidin-4-amine, (15.054) 9-fluoro-2,2-dimethyl-5-(quinolin-3-yl)-2,3-dihydro-1,4-benzoxazepine, (15.055) but-3-yn-1-yl {6-[({[(Z)-(1-methyl-1H-tetrazol-5-yl)(phenyl)methylene]amino}oxy)methyl]pyridin-2-yl}carbamate, (15.056) ethyl (2Z)-3-amino-2-cyano-3-phenylacrylate, (15.057) phenazine-1-carboxylic acid, (15.058) propyl 3,4,5-trihydroxybenzoate, (15.059) quinolin-8-ol, (15.060) quinolin-8-ol sulfate (2:1), (15.061) tert-butyl {6-[({[(1-methyl-1H-tetrazol-5-yl)(phenyl)methylene]amino}oxy)methyl]pyridin-2-yl}carbamate, and (15.062) 5-fluoro-4-imino-3-methyl-1-[(4-methylphenyl)sulfonyl]-3,4-dihydropyrimidin-2(1H)-one, (15.063) Metyltetraprole, (15.064) Aminopyrifen, and (15. 065) Pyrapropoyne, (15.066) (N'-[2-chloro-4-(2-fluorophenoxy)-5-methylphenyl]-N-ethyl-N-methylimidoformamide), (15.067) (N'-(2-chloro-5-methyl-4-phenoxyphenyl)-N-ethyl-N-methylimidoformamide), (15.068) (2- {2-[(7,8-difluoro-2-methylquinolin-3-yl)oxy]-6-fluorophenyl}propan-2-ol), (15.069) (5-bromo-1-(5,6-dimethylpyridin-3-yl)-3,3-dimethyl-3,4-dihydroisoquinoline), (15.070) (3-(4,4-difluoro-5,5-dimethyl-4,5-dihydrothieno[2,3-c]pyridin-7-yl)quinoline), (15.071) (1-(4,5-dimethyl-1H-benzimidazol-1-yl)-4,4-difluoro-3,3-dimethyl-3,4-dihydroisoquinoline), (15.072) 8-fluoro-3-(5-fluoro-3,3-dimethyl-3,4-dihydroisoquinolin-1-yl)quinolone, (15.073) 8-fluoro-3-(5-fluoro-3,3,4,4-tetramethyl-3,4-dihydroisoquinolin-1-yl)quinolone, (15.074) 3-(4,4-difluoro-3,3-dimethyl-3,4-dihydroisoquinolin-1-yl)-8-fluoroquinoline, (15.075) (N-methyl-N-phenyl-4-[5-(trifluoromethyl)-1,2,4-oxadiazol-3-yl]benzamide), (15.076) (methyl {4-[5-(trifluoromethyl)-1,2,4-oxadiazol-3-yl]phenyl}carbamate), (15.077) (N-{4-[5-(trifluoromethyl)-1,2,4-oxadiazol-3-yl]benzyl}cyclopropanecarboxamide), (15.078) N-methyl-4-(5-(trifluoromethyl)-1,2,4-oxadiazol-3-yl]benzamide, (15.079) N-[(E)-methoxyiminomethyl]-4-[5-(trifluoromethyl)-1,2,4-oxadiazol-3-yl]benzamide, (15.080) N-[(Z)-methoxyiminomethyl]-4-[5-(trifluoromethyl)-1,2,4-oxadiazol-3-yl]benzamide, (15. 081) N-[4-[5-(trifluoromethyl)-1,2,4-oxadiazol-3-yl]phenyl]cyclopropanecarboxamide, (15.082) N-(2-fluorophenyl)-4-[5-(trifluoromethyl)-1,2,4-oxadiazol-3-yl]benzamide, (15.083) 2,2-difluoro-N-methyl-2-[4-[5-(trifluoromethyl)-1,2,4-oxadiazol-3-yl]phenyl]acetamide, (15.084) N-allyl-N-[[4-[5-(trifluoromethyl)-1,2,4-oxadiazol-3-yl)phenyl]methyl]acetamide, (15.085) N-[(E)-N-methoxy-C-methyl-carbonimidoyl]-4-(5-(trifluoromethyl)-1,2,4-oxadiazol-3-yl]benzamide, (15.086) N-[(Z)-N-methoxy-C-methyl-carbonimidoyl]-4-[5-(trifluoromethyl)-1,2,4-oxadiazol-3-yl]benzamide, (15.087) N-allyl-N-[[4-[5-(trifluoromethyl)-1,2,4-oxadiazol-3-yl]phenyl]methyl]propanamide, (15.088) 4,4-dimethyl-1-[[4-[5-(trifluoromethyl)-1,2,4-oxadiazol-3-yl]phenyl]methyl]pyrro lidin-2-one, (15.089) N-methyl-4-[5-(trifluoromethyl)-1,2,4-oxadiazol-3-yl]benzenecarbothioamide, (15.090) 5-methyl-1-[[4-[5-(trifluoromethyl)-1,2,4-oxadiazol-3-yl]phenyl]methyl]pyrrolidin-2-one, (15.091) N-((2,3-difluoro-4-[5-(trifluoromethyl)-1,2,4-oxadiazol-3-yl]phenyl]methyl]-3,3,3-trifluoro-propanamide, (15.092) 1-methoxy-1-methyl-3-[[4-[5-(trifluorometyhl}-1,2,4-oxadiazol-3-yl]phenyl]methyl]urea, (15.093) 1,1-diethyl-3-[[4-[5-(trifluoromethyl}-1,2,4-oxadiazol-3-yl]phenyl]methyl]urea, (15.094) N-[[4-[5-(trifluoromethyl)-1,2,4-oxadiazol-3-yl]phen-yl)methyl)propanamide, (15.095) N-methoxy-N-[[4-[5-(trifluoromethyl)-1,2,4-oxadiazol-3-yl]phenyl]methyl]cyclopropanecarboxamide, (15.096) 1-methoxy-3-methyl-1-[[4-[5-(trifluoromethyl)-1,2,4-oxadiazol-3-yl]phenyl]methyl]urea,

(15.097) N-methoxy-N-[[4-[5-(trifluoromethyl)-1,2,4-oxadiazol-3-yl]phenyl]methly)cyclopropanecarboxamide; (15.098) N,2-dimethoxy-N-[[4-[5-(trifluoromethyl}-1,2,4-oxadiazol-3-yl]phenyl]methyl]propanamide, (15.099) N-ethyl-2-methyl-N-[[4-[5-(trifluoromethyl)-1,2,4-oxadiazol-3-yl)phenyl]melhyl]propanamide, (15.100) 1-methoxy-3-methyl-1-[[4-[5-(trifluoromethyl)-1,2,4-oxadiazol-3-yl]phenyl]mehtyl]urea, (15.101) 1,3-dimethoxy-1-[[4- [5-(trifluoromehtyl)-1,2,4-oxadiazol-3-yl]phenyl]methyl]urea, (15.102) 3-ethyl-1-metho xy-1-[[4-[5-(trifluoromethyl)-1,2,4-oxadiazol-3- yl]phenyl]methyl]ur ea, (15.103) 1-[[4-[5-(t rifluoromethyl) -1,2, 4-oxad iazol-3-yl]phenyl]methyl]piperidin-2-one, (15.104) 4,4-dimethyl-2-[[4-(5-(trifluoromethyl)-1,2,4-oxadiazol-3-yl]phenyl]methyl]isooxazolidin-3-one, (15.105) 5,5-dimelhyl-2-[[4-[5-(trifluoromethyl)-1,2,4-oxadiazo 1-3-yl]phenyl]meth yl]isoxazolidin-3-one, (15.106), 3,3-dimethyl-1-[[4-[5-(trifluoromethyl)-1,2,4-oxadiazol-3-yl]phenyl]methyl[piperidin-2-one, (15.107) 1-[[3-fluoro-4-(5-(trifluoromelhyl)-1,2,4-oxadiazol-3-yl]phenyl]methyl]azepan-2-one, (15.108) 4,4-dimethyl-2-[[4-(5-(trifluoromethyl)-1,2,4-oxadiazol-3-yl]phenyl]methyl]isoxazolidin-3-one and (15.109) 5,5-dimethyl-2-[[4-[5-(trifluoromethyl) -1,2,4- oxadiazol-3-yl]phenyl]methyl]isoxazolidin-3-one.

**9.** Active compound combination according to at least one of claims 1 to 8, wherein the further active compound is selected from the group consisting of (1.012) ipconazole, (1.018) prothioconazole, (1.020) spiroxamine, (1.021) tebuconazole, (1.083) 2-[6-(4-bromophenoxy)-2-(trifluoromethyl)-3-pyridyl]-1-(1,2,4-triazol-1-yl)propan-2-ol, (2.002) bixafen, (2.005) fluopyram, (2.017) penflufen, (2.027) 3-(difluoromethyl)-1-methyl-N-(1,1,3-trimethyl-2,3-dihydro-1H-inden-4-yl)-1H-pyrazole-4-carboxamide, (2.038) N-(5-chloro-2-isopropylbenzyl)-N-cyclopropyl-3-(difluoromethyl)-5-fluoro-1-methyl-1H-pyrazole-4-carboxamide, (3.012) fluoxastrobin, (3.020) trifloxystrobin, (3.025) (3S,6S,7R,8R)-8-benzyl-3-[({3-[(isobutyryloxy)methoxy]-4-methoxypyridin-2-yl}carbonyl)-amino]-6-methyl-4,9-dioxo-1,5-dioxonan-7-yl 2-methylpropanoate, (3.030) (1S)-2,2-bis(4-fluorophenyl)-1-methylethyl N-{[3-(acetyloxy)-4-methoxy-2-pyridyl]carbonyl}-L-alaninate, (4.005) pencycuron, (5.004) chlorothalonil, (5.012) folpet, (5.013) mancozeb, (5.018) propineb, (12.003) metalaxyl, (12.004) metalaxyl-M (mefenoxam), (13.001) fludioxonil, (13.004) proquinazid, (15.008) cyflufenamid, and (15.012) fosetyl-aluminium.

**10.** Active compound combination according to at least one of claims 1 to 9, which comprises at least at least two further active compounds.

**11.** Formulation for controlling harmful microorganisms, preferably phytopathogenic harmful fungi, in crop protection and in the protection of materials, **characterized by** a content of an active compound combination according to at least one of claims 1 to 10, in addition to at least one extender and/or surfactant.

**12.** Method for controlling harmful microorganisms, preferably phytopathogenic harmful fungi, in crop protection and in the protection of materials, **characterized in that** an active compound combination according to at least one of claims 1 to 10 or a formulation according to claim 11 is applied to the harmful microorganisms and/or their habitat.

**13.** Use of an active compound combination according to at least one of claims 1 to 10 or a formulation according to claim 11 for control of harmful microorganisms, preferably phytopathogenic harmful fungi, in crop protection and in the protection of materials.

**14.** Use of an active compound combination according to at least one of claims 1 to 10 or a formulation according to claim 11 for treatment of a transgenic plant.

**15.** Use of an active compound combination according to at least one of claims 1 to 10 or a formulation according to claim 11 for treatment of seed, preferably seed of a transgenic plant.

**16.** Seed coated with an active compound combination according to at least one of claims 1 to 10 or a formulation according to claim 11.

Europäisches Patentamt
European Patent Office
Office européen des brevets

# EUROPEAN SEARCH REPORT

Application Number
EP 18 21 5136

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2018/161008 A1 (GILEAD APOLLO LLC [US]; MONSANTO TECHNOLOGY LLC [US]) 7 September 2018 (2018-09-07)<br>* the claims; the examples and paragraphs 1-5 * | 1-16<br>1-16 | INV.<br>A01N25/00<br>A01N43/90<br>C07D495/04<br>A01P3/00 |
| X<br>Y | US 2017/166585 A1 (BENNETT JENNIFER LYNN [US] ET AL) 15 June 2017 (2017-06-15)<br>* the claims; the examples; the embodiments; paragraphs 84-89, 201-263 * | 1-16<br>1-16 | |
| X<br>Y | US 2017/166584 A1 (GHOSH SHOMIR [US] ET AL) 15 June 2017 (2017-06-15)<br>* the claims; paragraphs 6-8, 249-392, and the examples * | 1-16<br>1-16 | |
| X<br>Y | US 2017/166583 A1 (GHOSH SHOMIR [US] ET AL) 15 June 2017 (2017-06-15)<br>* the claims; the examples; paragraphs 6-8, 302-398, 403, 409 and 415 * | 1-16<br>1-16 | TECHNICAL FIELDS SEARCHED (IPC) |
| X<br>Y | US 2013/123231 A1 (HARRIMAN GERALDINE C [US] ET AL) 16 May 2013 (2013-05-16)<br>* the claims; the examples; paragraphs 253-335 * | 1-16<br>1-16 | A01N<br>C07D |

~~The present search report has been drawn up for all claims~~

2

| Place of search | Date of completion of the search | Examiner |
|---|---|---|
| Munich | 23 January 2019 | Lorenzo Varela, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 18 21 5136

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [X] None of the further search fees have been paid within the fixed time limit. The present European search report has been drawn up for those parts of the European patent application which relate to the invention first mentioned in the claims, namely claims:

  1-16(partially)

- [ ] The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION
# SHEET B

Application Number
EP 18 21 5136

The Search Division considers that the present European patent application does not comply with the requirements of unity of invention and relates to several inventions or groups of inventions, namely:

1. claims: 1-16(partially)

   1) active compound combination comprising (A) at least one compound of formula (I) and (B) at least one further fungicide selected from the group (1) inhibitors of the ergosterol synthesis; 2) method for controlling harmful microorganisms with the mentioned combination; 3) use of the mentioned combination for control of harmful microorganisms, for treatment of a transgenic plant or a seed, and 4) seed coated with the mentioned composition.

---

2. claims: 1-16(partially)

   1) active compound combination comprising (A) at least one compound of formula (I) and (B) at least one further fungicide selected from the group (2) inhibitors of the respiratory chain at complex I or II; 2) method for controlling harmful microorganisms with the mentioned combination; 3) use of the mentioned combination for control of harmful microorganisms, for treatment of a transgenic plant or a seed, and 4) seed coated with the mentioned composition.

---

3. claims: 1-16(partially)

   1) active compound combination comprising (A) at least one compound of formula (I) and (B) at least one further fungicide selected from the group (3) inhibitors of the respiratory chain at complex III; 2) method for controlling harmful microorganisms with the mentioned combination; 3) use of the mentioned combination for control of harmful microorganisms, for treatment of a transgenic plant or a seed, and 4) seed coated with the mentioned composition.

---

4. claims: 1-16(partially)

   1) active compound combination comprising (A) at least one compound of formula (I) and (B) at least one further fungicide selected from the group (4) inhibitors of the mitosis and cell division; 2) method for controlling harmful microorganisms with the mentioned combination; 3) use of the mentioned combination for control of harmful microorganisms, for treatment of a transgenic plant or a seed, and 4) seed coated with the mentioned composition.

---

5. claims: 1-16(partially)

Europäisches Patentamt
European Patent Office
Office européen des brevets

## LACK OF UNITY OF INVENTION SHEET B

Application Number

EP 18 21 5136

The Search Division considers that the present European patent application does not comply with the requirements of unity of invention and relates to several inventions or groups of inventions, namely:

1) active compound combination comprising (A) at least one compound of formula (I) and (B) at least one further fungicide selected from the group (5) compounds capable of having a multisite action; 2) method for controlling harmful microorganisms with the mentioned combination; 3) use of the mentioned combination for control of harmful microorganisms, for treatment of a transgenic plant or a seed, and 4) seed coated with the mentioned composition.

---

6. claims: 1-8, 10-16(all partially)

1) active compound combination comprising (A) at least one compound of formula (I) and (B) at least one fungicide selected from the group (6) compounds capable of inducing a host defense; 2) method for controlling harmful microorganisms with the mentioned combination; 3) use of the mentioned combination for control of harmful microorganisms, for treatment of a transgenic plant or a seed, and 4) seed coated with the mentioned composition.

---

7. claims: 1-8, 10-16(all partially)

1) active compound combination comprising (A) at least one compound of formula (I) and (B) at least one further active compound selected from the following group (7) inhibitors of the amino acid and/or protein biosynthesis; 2) method for controlling harmful microorganisms with the mentioned combination; 3) use of the mentioned combination for control of harmful microorganisms, for treatment of a transgenic plant or a seed, and 4) seed coated with the mentioned composition.

---

8. claims: 1-8, 10-16(all partially)

1) active compound combination comprising (A) at least one compound of formula (I) and (B) at least one further fungicide selected from the following group (8) inhibitors of the ATP production; 2) method for controlling harmful microorganisms with the mentioned combination; 3) use of the mentioned combination for control of harmful microorganisms, for treatment of a transgenic plant or a seed, and 4) seed coated with the mentioned composition.

---

9. claims: 1-8, 10-16(all partially)

1) active compound combination comprising (A) at least one compound of formula (I) and (B) at least one further

Europäisches Patentamt
European Patent Office
Office européen des brevets

# LACK OF UNITY OF INVENTION SHEET B

Application Number
EP 18 21 5136

The Search Division considers that the present European patent application does not comply with the requirements of unity of invention and relates to several inventions or groups of inventions, namely:

fungicide selected from the following group (9) inhibitors of the cell wall synthesis; 2) method for controlling harmful microorganisms with the mentioned combination; 3) use of the mentioned combination for control of harmful microorganisms, for treatment of a transgenic plant or a seed, and 4) seed coated with the mentioned composition.

---

10. claims: 1-8, 10-16(all partially)

1) active compound combination comprising (A) at least one compound of formula (I) and (B) at least one further fungicide selected from the following group (10) inhibitors of the lipid and membrane synthesis; 2) method for controlling harmful microorganisms with the mentioned combination; 3) use of the mentioned combination for control of harmful microorganisms, for treatment of a transgenic plant or a seed, and 4) seed coated with the mentioned composition.

---

11. claims: 1-8, 10-16(all partially)

1) active compound combination comprising (A) at least one compound of formula (I) and (B) at least one further fungicide selected from the following group (11) inhibitors of the melanine biosynthesis; 2) method for controlling harmful microorganisms with the mentioned combination; 3) use of the mentioned combination for control of harmful microorganisms, for treatment of a transgenic plant or a seed, and 4) seed coated with the mentioned composition.

---

12. claims: 1-16(partially)

1) active compound combination comprising (A) at least one compound of formula (I) and (B) at least one further fungicide selected from the following group (12) inhibitors of the nucleic acid synthesis; 2) method for controlling harmful microorganisms with the mentioned combination; 3) use of the mentioned combination for control of harmful microorganisms, for treatment of a transgenic plant or a seed, and 4) seed coated with the mentioned composition.

---

13. claims: 1-16(partially)

1) active compound combination comprising (A) at least one compound of formula (I) and (B) at least one further fungicide selected from the following group (13) inhibitors of the signal transduction; 2) method for controlling

Europäisches Patentamt
European Patent Office
Office européen des brevets

# LACK OF UNITY OF INVENTION SHEET B

Application Number
EP 18 21 5136

The Search Division considers that the present European patent application does not comply with the requirements of unity of invention and relates to several inventions or groups of inventions, namely:

harmful microorganisms with the mentioned combination; 3) use of the mentioned combination for control of harmful microorganisms, for treatment of a transgenic plant or a seed, and 4) seed coated with the mentioned composition.

---

14. claims: 1-8, 10-16(all partially)

1) active compound combination comprising (A) at least one compound of formula (I) and (B) at least one further fungicide selected from the following group (14) compounds capable of acting as uncoupler; 2) method for controlling harmful microorganisms with the mentioned combination; 3) use of the mentioned combination for control of harmful microorganisms, for treatment of a transgenic plant or a seed, and 4) seed coated with the mentioned composition.

---

15. claims: 1-16(partially)

1) active compound combination comprising (A) at least one compound of formula (I) and (B) at least one further fungicide which does not belong to the following groups (1) inhibitors of the ergosterol synthesis,(2) inhibitors of the respiratory chain at complex I or II,(3) inhibitors of the respiratory chain at complex III,(4) inhibitors of the mitosis and cell division,(5) compounds capable of having a multisite action,(6) compounds capable of inducing a host defense,(7) inhibitors of the amino acid and/or protein biosynthesis,(8) inhibitors of the ATP production,(9) inhibitors of the cell wall synthesis,(10) inhibitors of the lipid and membrane synthesis,(11) inhibitors of the melanine biosynthesis,(12) inhibitors of the nucleic acid synthesis,(13) inhibitors of the signal transduction, or (14) compounds capable of acting as uncoupler; 2) method for controlling harmful microorganisms with the mentioned combination; 3) use of the mentioned combination for control of harmful microorganisms, for treatment of a transgenic plant or a seed, and 4) seed coated with the mentioned composition.

---

## ANNEX TO THE EUROPEAN SEARCH REPORT ON EUROPEAN PATENT APPLICATION NO.

EP 18 21 5136

This annex lists the patent family members relating to the patent documents cited in the above-mentioned European search report. The members are as contained in the European Patent Office EDP file on
The European Patent Office is in no way liable for these particulars which are merely given for the purpose of information.

23-01-2019

| Patent document cited in search report | Publication date | Patent family member(s) | Publication date |
|---|---|---|---|
| WO 2018161008 A1 | 07-09-2018 | US 2019072728 A1 | 07-03-2019 |
| | | WO 2018161008 A1 | 07-09-2018 |
| US 2017166585 A1 | 15-06-2017 | AR 106822 A1 | 21-02-2018 |
| | | AU 2016359626 A1 | 17-05-2018 |
| | | BR 112018009212 A2 | 06-11-2018 |
| | | CA 3004747 A1 | 01-06-2017 |
| | | CL 2018001231 A1 | 16-11-2018 |
| | | CN 108347939 A | 31-07-2018 |
| | | CO 2018004891 A2 | 22-10-2018 |
| | | EA 201890910 A1 | 30-11-2018 |
| | | EP 3379933 A1 | 03-10-2018 |
| | | JP 2019503338 A | 07-02-2019 |
| | | KR 20180086191 A | 30-07-2018 |
| | | US 2017166585 A1 | 15-06-2017 |
| | | US 2019040078 A1 | 07-02-2019 |
| | | WO 2017091627 A1 | 01-06-2017 |
| US 2017166584 A1 | 15-06-2017 | AR 106806 A1 | 21-02-2018 |
| | | AU 2016361428 A1 | 24-05-2018 |
| | | BR 112018010453 A2 | 21-11-2018 |
| | | CA 3005900 A1 | 01-06-2017 |
| | | CL 2018001360 A1 | 24-08-2018 |
| | | CN 108349994 A | 31-07-2018 |
| | | CO 2018005369 A2 | 31-05-2018 |
| | | EA 201890926 A1 | 28-12-2018 |
| | | EP 3380479 A1 | 03-10-2018 |
| | | JP 2018536661 A | 13-12-2018 |
| | | KR 20180082557 A | 18-07-2018 |
| | | US 2017166584 A1 | 15-06-2017 |
| | | WO 2017091617 A1 | 01-06-2017 |
| US 2017166583 A1 | 15-06-2017 | AR 106799 A1 | 21-02-2018 |
| | | AU 2016361414 A1 | 24-05-2018 |
| | | BR 112018009911 A2 | 13-11-2018 |
| | | CA 3004798 A1 | 01-06-2017 |
| | | CL 2018001361 A1 | 24-08-2018 |
| | | CN 108290902 A | 17-07-2018 |
| | | CO 2018005381 A2 | 31-05-2018 |
| | | EA 201890949 A1 | 28-12-2018 |
| | | EP 3380478 A1 | 03-10-2018 |
| | | JP 2018536660 A | 13-12-2018 |
| | | KR 20180082558 A | 18-07-2018 |
| | | US 2017166583 A1 | 15-06-2017 |
| | | WO 2017091602 A1 | 01-06-2017 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 18 21 5136

This annex lists the patent family members relating to the patent documents cited in the above-mentioned European search report. The members are as contained in the European Patent Office EDP file on
The European Patent Office is in no way liable for these particulars which are merely given for the purpose of information.

23-01-2019

| Patent document cited in search report | Publication date | Patent family member(s) | Publication date |
|---|---|---|---|
| US 2013123231 A1 | 16-05-2013 | AU 2012335088 A1 | 29-05-2014 |
| | | AU 2017265088 A1 | 14-12-2017 |
| | | BR 112014011351 A2 | 06-06-2017 |
| | | CA 2855372 A1 | 16-05-2013 |
| | | CL 2014001241 A1 | 28-11-2014 |
| | | CN 104105485 A | 15-10-2014 |
| | | CN 106905346 A | 30-06-2017 |
| | | CY 1120224 T1 | 12-12-2018 |
| | | DK 2776038 T3 | 23-04-2018 |
| | | EA 201490826 A1 | 30-01-2015 |
| | | EP 2776038 A1 | 17-09-2014 |
| | | EP 3329919 A1 | 06-06-2018 |
| | | ES 2665580 T3 | 26-04-2018 |
| | | HK 1201207 A1 | 28-08-2015 |
| | | HR P20180534 T1 | 04-05-2018 |
| | | HU E037444 T2 | 28-09-2018 |
| | | JP 6114297 B2 | 12-04-2017 |
| | | JP 6276442 B2 | 07-02-2018 |
| | | JP 2014533281 A | 11-12-2014 |
| | | JP 2017114910 A | 29-06-2017 |
| | | JP 2018052989 A | 05-04-2018 |
| | | KR 20140106554 A | 03-09-2014 |
| | | KR 20190041027 A | 19-04-2019 |
| | | LT 2776038 T | 25-04-2018 |
| | | MX 344490 B | 16-12-2016 |
| | | NZ 624819 A | 28-10-2016 |
| | | NZ 723933 A | 23-03-2018 |
| | | PL 2776038 T3 | 29-06-2018 |
| | | PT 2776038 T | 18-04-2018 |
| | | SI 2776038 T1 | 29-06-2018 |
| | | TW 201350489 A | 16-12-2013 |
| | | TW 201720831 A | 16-06-2017 |
| | | TW 201831492 A | 01-09-2018 |
| | | UA 114901 C2 | 28-08-2017 |
| | | US 2013123231 A1 | 16-05-2013 |
| | | US 2015203510 A1 | 23-07-2015 |
| | | US 2016297834 A1 | 13-10-2016 |
| | | US 2019016732 A1 | 17-01-2019 |
| | | WO 2013071169 A1 | 16-05-2013 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2013071169 A1
- WO 2017091602 A
- WO 2017091617 A
- WO 2017091627 A
- US 6060051 A
- US 7094592 B
- WO 2016154297 A
- US 6245551 B
- TW 4764
- TW 5454
- TW 5096
- TW 5277
- EP 701275 A
- EP 718408 A
- IT 2008000196 W
- WO 2006043635 A
- WO 2003106457 A
- WO 2006003494 A
- WO 2010052161 A
- EP 2647626 A
- WO 2004099160 A
- JP 2010018586 A
- WO 2012029672 A
- WO 2013144213 A
- WO 2010051926 A
- CN 103232431
- WO 2013050317 A1
- WO 2013162715 A2
- WO 2013162716 A2
- US 20140213448 A1
- CN 101337937 A
- CN 103109816 A
- WO 2012034403 A1
- WO 2011085575 A1
- CN 101337940 A
- CN 101715774 A
- CN 103524422 A
- CN 102391261 A
- US 20140275503 A1
- WO 2007040280 A1
- WO 2007040282 A1
- WO 2015058021 A1
- WO 2015058028 A1
- CN 103265527 A
- WO 2013115391 A1
- WO 2010066780 A1
- WO 2011151146 A1
- WO 2014187846 A1
- DE 3639877 A1
- WO 2012029672 A1
- WO 2016005276 A1
- WO 2011105506 A1
- WO 2016133011 A1
- WO 2018161008 A1

### Non-patent literature cited in the description

- **ONGENA, M. et al.** Bacillus Lipopeptides: Versatile Weapons for Plant Disease Biocontrol. *Trends in Microbiology,* March 2008, vol. 16 (3), 115-125
- *CHEMICAL ABSTRACTS,* 885026-50-6
- *CHEMICAL ABSTRACTS,* 637360-23-7
- *CHEMICAL ABSTRACTS,* 872999-66-1
- *CHEMICAL ABSTRACTS,* 1225292-17-0
- *CHEMICAL ABSTRACTS,* 1440516-42-6
- *CHEMICAL ABSTRACTS,* 792914-58-0
- *CHEMICAL ABSTRACTS,* 1204776-60-2
- *CHEMICAL ABSTRACTS,* 1363400-41-2
- *CHEMICAL ABSTRACTS,* 1461743-15-6
- *CHEMICAL ABSTRACTS,* 1226889-14-0
- *CHEMICAL ABSTRACTS,* 1449220-44-3
- *CHEMICAL ABSTRACTS,* 1332628-83-7
- *CHEMICAL ABSTRACTS,* 1477923-37-7
- *CHEMICAL ABSTRACTS,* 1105672
- *CHEMICAL ABSTRACTS,* 1232543-85-9
- *CHEMICAL ABSTRACTS,* 1268277-22-0
- *CHEMICAL ABSTRACTS,* 1233882-22-8
- *CHEMICAL ABSTRACTS,* 1108184-52-6
- *CHEMICAL ABSTRACTS,* 1542271-46-4
- *CHEMICAL ABSTRACTS,* 1370358-69-2
- *CHEMICAL ABSTRACTS,* 1181213-14-8
- *CHEMICAL ABSTRACTS,* 1253850-56-4
- *CHEMICAL ABSTRACTS,* 933798-27-7
- *CHEMICAL ABSTRACTS,* 934001-66-8
- *CHEMICAL ABSTRACTS,* 1477919-27-9
- *CHEMICAL ABSTRACTS,* 1452877-50-7
- *CHEMICAL ABSTRACTS,* 1449021-97-9
- *CHEMICAL ABSTRACTS,* 1229023-34-0
- *CHEMICAL ABSTRACTS,* 1638765-58-8
- *CHEMICAL ABSTRACTS,* 1229023-00-0
- *CHEMICAL ABSTRACTS,* 1689566-03-7
- *CHEMICAL ABSTRACTS,* 1702305-40-5

- *CHEMICAL ABSTRACTS,* 1332838-17-1
- *CHEMICAL ABSTRACTS,* 129531-12-0
- *CHEMICAL ABSTRACTS,* 71526-07-3
- *CHEMICAL ABSTRACTS,* 52836-31-4
- Pesticide Manual
- **COLBY, S.R.** Calculating Synergistic and Antagonistic Responses of Herbicide Combinations. *Weeds,* 1967, vol. 15, 20-22
- Isoboles, a graphic representation of synergism in pesticides. *Neth. J. Plant Path.,* 1964, vol. 70, 73-80